(19) Europäisches Patentamt / European Patent Office / Office européen des brevets

(11) EP 4 644 395 A1

(12) # EUROPEAN PATENT APPLICATION
published in accordance with Art. 153(4) EPC

(43) Date of publication:
**05.11.2025 Bulletin 2025/45**

(21) Application number: **23910849.1**

(22) Date of filing: **28.12.2023**

(51) International Patent Classification (IPC):
***C07D 491/22*** *(2006.01)* ***A61K 31/4375*** *(2006.01)*
***A61K 47/68*** *(2017.01)* ***A61P 35/00*** *(2006.01)*

(52) Cooperative Patent Classification (CPC):
**A61K 31/4375; A61K 47/68; A61P 35/00; C07D 491/22**

(86) International application number:
**PCT/CN2023/142848**

(87) International publication number:
**WO 2024/140933 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**30.12.2022 CN 202211739606**
**07.04.2023 CN 202310367781**
**21.06.2023 CN 202310743291**
**28.07.2023 CN 202310941713**
**12.09.2023 CN 202311173712**
**25.12.2023 CN 202311802330**

(71) Applicant: **Changchun Genescience Pharmaceutical Co., Ltd.**
**Changchun, Jilin 130012 (CN)**

(72) Inventors:
- **YANG, Fanglong**
**Changchun, Jilin 130012 (CN)**
- **HUANG, Yue**
**Changchun, Jilin 130012 (CN)**
- **HE, Weiming**
**Changchun, Jilin 130012 (CN)**
- **DAI, Kaiqin**
**Changchun, Jilin 130012 (CN)**
- **WANG, Siqin**
**Changchun, Jilin 130012 (CN)**
- **JIN, Lei**
**Changchun, Jilin 130012 (CN)**

(74) Representative: **Gille Hrabal Partnerschaftsgesellschaft mbB**
**Patentanwälte**
**Brucknerstraße 20**
**40593 Düsseldorf (DE)**

(54) **CAMPTOTHECIN DERIVATIVE, PHARMACEUTICAL COMPOSITION, AND PREPARATION METHOD AND USE THEREFOR**

(57) The invention relates to a camptothecin derivative as shown in general formula I, a pharmaceutical composition, and a preparation method and use therefor. The camptothecin derivative has good tumor inhibitory activity, can be used for treating or preventing tumors, and can be used for preparing drugs for treating or preventing tumor diseases.

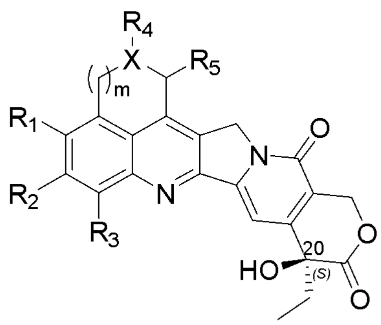

I

EP 4 644 395 A1

## Description

The present application claims priority to application No. 202211739606.8 filed on 30 December 2022, application No. 202310367781.7 filed on 7 April 2023, application No. 202310743291.2 filed on 21 June 2023, and application No. 202310941713.7 filed on 28 July 2023, application No. 202311173712.9 filed on 12 September 2023, and application No. 202311802330.8 filed on 25 December 2023, which are all titled "CAMPTOTHECIN DERIVATIVE, PHARMACEUTICAL COMPOSITION, PREPARATION METHOD THEREFOR, AND USE THEREOF".

## Technical Field

The present invention belongs to the field of pharmaceuticals, and specifically relates to a camptothecin derivative, a pharmaceutical composition, a preparation method therefor, and use thereof.

## Background

Camptothecin (CPT) is a natural product isolated from *Camptotheca acuminata*, a plant of nyssaceae. Camptothecin is a pentacyclic compound composed of quinoline ring AB, pyrrole ring C, pyridone ring D, and α-hydroxylactone ring E, in which the 20-C is S-configuration (see the structural formula below). Due to its excellent anti-cancer activity, it was put into clinical use in the early 1970s. Later, severe drug side effects such as diarrhea and hemorrhagic cystitis occurred clinically, and the clinical trial was terminated.

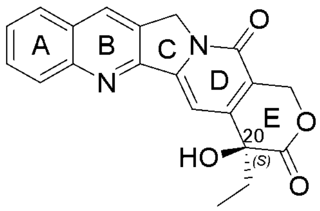

Camptothecin (CPT) structure

Research data shows that camptothecin can form a ternary complex with DNA topoisomerase I in cells, thereby inhibiting DNA despiralization, blocking DNA replication, and resulting in cell death (Cancer Res. 1989, 49, 6365). Camptothecin and a derivative thereof have very strong antitumor activity in an in vivo animal model such as lung cancer, breast cancer, colorectal cancer, and ovarian cancer (Nature Review Cancer. 2006, 6, 789). At present, a plurality of camptothecin drugs have been approved for marketing for tumor treatment (Med Res. Rev. 2015, 35, 753). Irinotecan is a medicament for treating colorectal cancer; topotecan is used for treating ovarian cancer; and belotecan is used for treating ovarian cancer and small cell lung cancer. The camptothecin derivative further comprises, e.g., exatecan, rubitecan, diflomotecan, lurtotecan, gimatecan, simimtecan, chimmitecan, or elomotecan. The camptothecin drugs or derivatives often have hematotoxicity caused by myelosuppression, such as leukopenia, thrombocytopenia, anemia, or neutropenia, as well as gastrointestinal side effects, such as nausea, emesis, or diarrhea. As found through clinical studies, measures for improving the safety and efficacy of camptothecin compounds comprise, e.g., increasing water solubility, improving pharmacokinetic properties thereof, enhancing activity, reducing dosage, or forming antibody-drug conjugates using conjugates thereof and antibodies. Therefore, research and development of camptothecin compounds and conjugates thereof with novel structures and capable of enhancing efficacy and improving safety issues still has very high clinical demand and application value.

## Summary of the Invention

The present invention provides a compound represented by formula I, a racemate, a stereoisomer, a tautomer, an isotopically labeled compound, a solvate, a polymorph, a pharmaceutically acceptable salt, or a prodrug compound thereof:

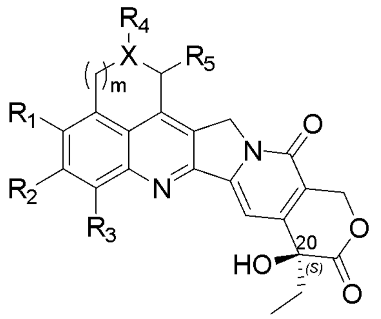

I

wherein $R_1$, $R_2$, and $R_3$ are identical or different, and are selected independently of each other from H, OH, CN, a halogen, a $C_{1-10}$ alkyl, a $C_{2-10}$ alkenyl, a $C_{2-10}$ alkynyl, a $C_{1-10}$ alkoxy, a $C_{1-10}$ haloalkyl, a $C_{1-10}$ haloalkoxy, a $C_{1-10}$ cyanoalkyl, a $C_{1-10}$ cyanoalkoxy, or a $C_{3-10}$ cycloalkyl;
$R_4$ is selected from H or

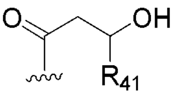 ;

$R_{41}$ is selected from H, a $C_{1-6}$ alkyl, a $C_{1-6}$ haloalkyl, a $C_{1-6}$ alkyl-NH-, a $(C_{1-6}$ alkyl$)_2$N-, a $C_{1-6}$ alkyl-NH-$C_{1-6}$ alkyl, a $(C_{1-6}$ alkyl$)_2$N-$C_{1-6}$ alkyl, a $C_{1-6}$ alkoxyalkyl, a $C_{2-6}$ alkenyl, a $C_{2-6}$ alkynyl, a $C_{3-6}$ cycloalkyl, a 3-6-membered heterocyclyl, a $C_{6-14}$ aryl, and a 5-14-membered heteroaryl;
$R_5$ is selected from H,

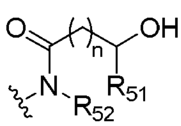 , 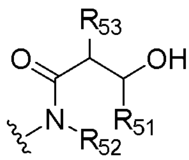 , or 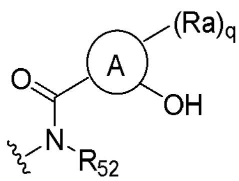 ;

$R_{51}$ and $R_{52}$ are identical or different, and are selected independently of each other from H, a $C_{1-6}$ alkyl, a $C_{1-6}$ haloalkyl, a $C_{1-6}$ alkyl-NH-, a $(C_{1-6}$ alkyl$)_2$N-, a $C_{1-6}$ alkyl-NH-$C_{1-6}$ alkyl, a $(C_{1-6}$ alkyl$)_2$N-$C_{1-6}$ alkyl, a $C_{1-6}$ alkoxyalkyl, a $C_{2-6}$ alkenyl, a $C_{2-6}$ alkynyl, a $C_{3-6}$ cycloalkyl, a 3-6-membered heterocyclyl, a $C_{6-14}$ aryl, and a 5-14-membered heteroaryl; $R_{53}$ is selected from a $C_{1-6}$ alkyl, a $C_{1-6}$ haloalkyl, a $C_{1-6}$ alkyl-NH-, a $(C_{1-6}$ alkyl$)_2$N-, a $C_{1-6}$ alkyl-NH-$C_{1-6}$ alkyl, a $(C_{1-6}$ alkyl$)_2$N-$C_{1-6}$ alkyl, a $C_{1-6}$ alkoxyalkyl, a $C_{2-6}$ alkenyl, a $C_{2-6}$ alkynyl, a $C_{3-6}$ cycloalkyl, a 3-6-membered heterocyclyl, a $C_{6-14}$ aryl, and a 5-14-membered heteroaryl; ring A is selected from a $C_{3-8}$ cycloalkyl or a C3-8 heterocyclyl, Ra is selected from H, hydroxyl, CN, a halogen, a $C_{1-6}$ alkyl, or a $C_{1-6}$ haloalkyl; n is selected from 0, 1, or 2; and q is selected from 0, 1, or 2;
X is selected from CH or N; and
m is an integer selected from 0-6.

According to some embodiments, the $R_1$ is selected from H, OH, CN, a halogen, a $C_{1-6}$ alkyl, a $C_{2-6}$ alkenyl, a $C_{2-6}$ alkynyl, a $C_{3-6}$ cycloalkyl, or a $C_{1-6}$ haloalkoxy;

according to some embodiments, the $R_1$ is selected from H, OH, CN, a halogen, a $C_{1-6}$ alkyl, a $C_{2-6}$ alkenyl, a $C_{2-6}$ alkynyl, or a $C_{1-6}$ haloalkoxy;
according to some embodiments, the $R_1$ is selected from H, OH, Br, methyl, difluoromethoxy, 2,2,2-trifluoroethoxy, ethenyl, cyclopropyl, or ethynyl; and
according to some embodiments, the $R_1$ is selected from H, OH, Br, methyl, difluoromethoxy, 2,2,2-trifluoroethoxy, ethenyl, or ethynyl.

According to some embodiments, the $R_2$ is selected from H, a halogen, CN, or a $C_{1-6}$ alkyl; and according to some embodiments, the $R_2$ is selected from H or F.

According to some embodiments, the $R_3$ is selected from H or a $C_{1-6}$ alkyl; and

according to some embodiments, the $R_3$ is H.

according to some embodiments, the $R_4$ is selected from H or

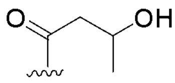

;

for example,

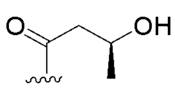

or

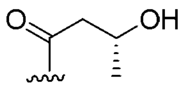

;

and

according to some embodiments, the $R_4$ is selected from H or

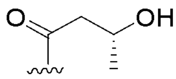

.

According to some embodiments, X-$R_4$ is

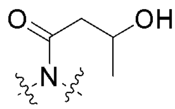

,

and is preferably

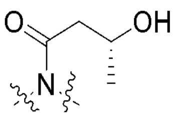

.

According to some embodiments, the X-$R_4$ is -$CH_2$-.

According to some embodiments, the $R_5$ is selected from H,

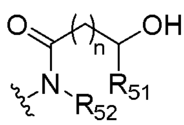

,

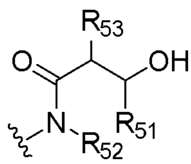

,

or

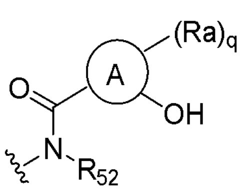

;

wherein the $R_{51}$ is selected from H, methyl, ethyl, isopropyl, or cyclopropyl; the $R_{52}$ is selected from H or methyl; the $R_{53}$ is selected from methyl; the ring A is selected from a $C_{3-6}$ cycloalkyl; the Ra is selected from H, hydroxyl, CN, a halogen, a $C_{1-6}$

alkyl, or a $C_{1-6}$ haloalkyl; the n is selected from 0 or 1; and the q is selected from 0 or 1; and according to some embodiments, the ring A is selected from a cyclobutane ring.

According to some embodiments, the $R_5$ is selected from H,

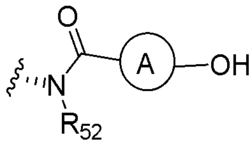,

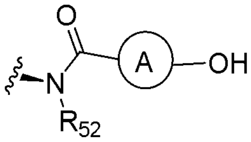, 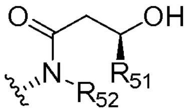, 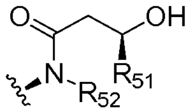, 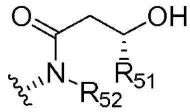, 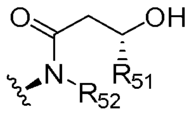,

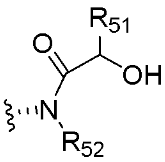, 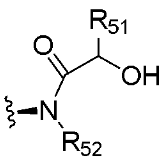, 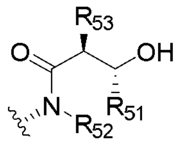, or 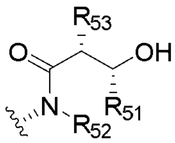;

according to some embodiments, the $R_{51}$ is selected from H, methyl, ethyl, isopropyl, or cyclopropyl; the $R_{52}$ is selected from H or methyl; the $R_{53}$ is selected from methyl; and the ring A is selected from a cyclobutane ring; and

according to some embodiments, the $R_5$ is selected from H,

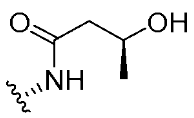, 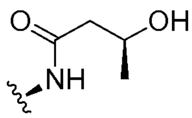,

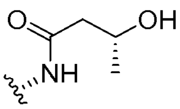, 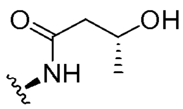, 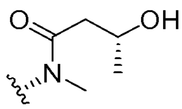, 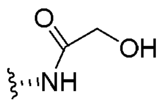, 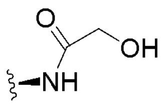, 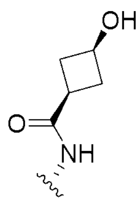,

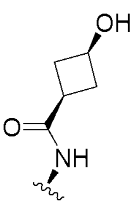, 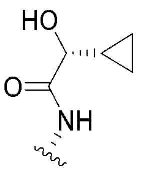, 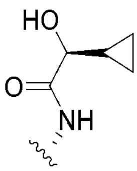, 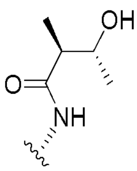, 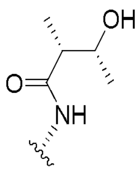, 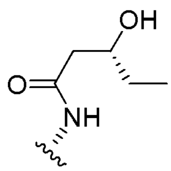, 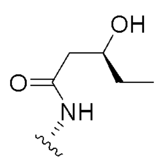,

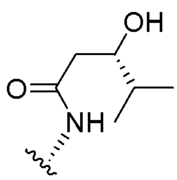, 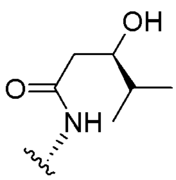, 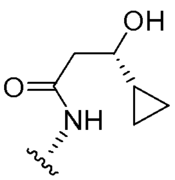, or 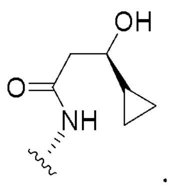.

According to some embodiments, the $R_5$ is selected from H,

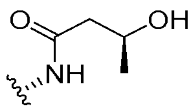, 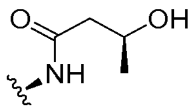,

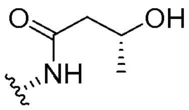, 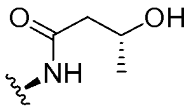, 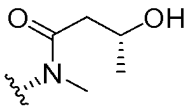, 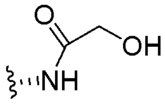, 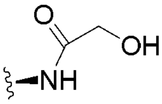, 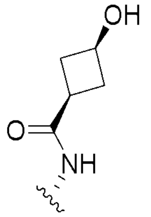,

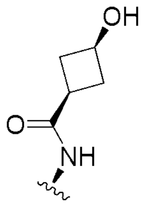, 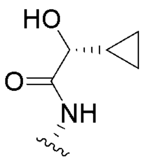, 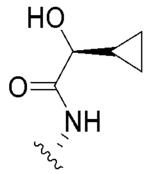, 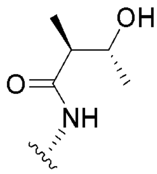, 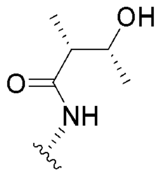, 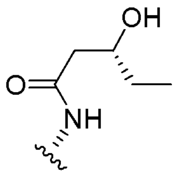, 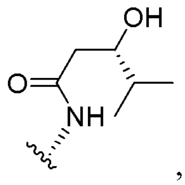, or

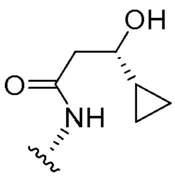.

According to some embodiments, the X is selected from CH or N; and when X is CH, the $R_4$ is H; or when X is N, the $R_5$ is H; and
according to some embodiments, the m is selected from 0, 1, or 2.

According to some embodiments, the compound represented by formula I has a structure as shown below:

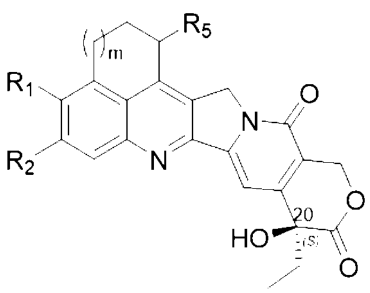

I-a

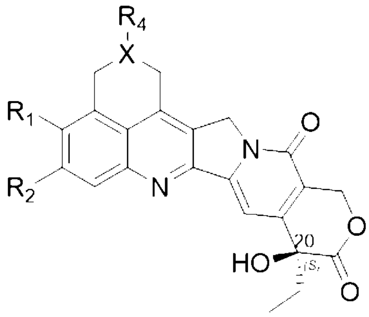

I-b

wherein the $R_1$, $R_2$, $R_4$, $R_5$, X, and m are as defined independently of each other herein.

According to some embodiments, the compound represented by formula I has a structure as shown below:

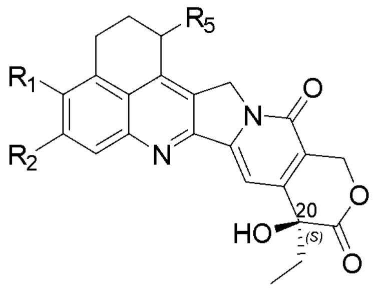

II

wherein the $R_1$, $R_2$, and $R_5$ are as defined independently of each other herein.

According to some embodiments, the compound represented by formula I has a structure as shown below:

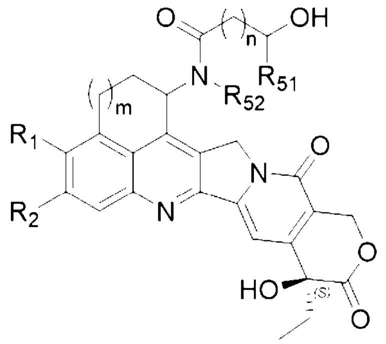

III-a

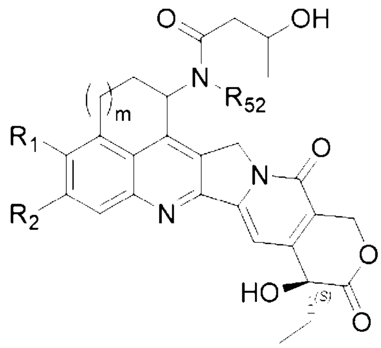

III-b

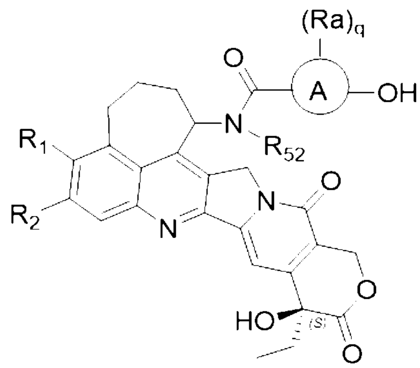

III-c

wherein the $R_1$, $R_2$, $R_{51}$, $R_{52}$, ring A, Ra, m, n, and q are as defined independently of each other herein.

According to some embodiments of the present invention, the compound of formula I has a structure as shown below:

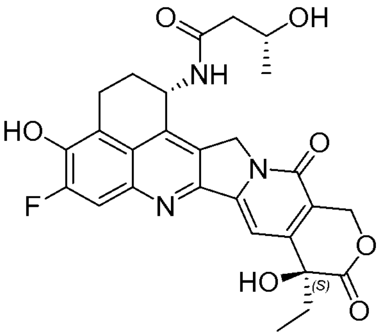,
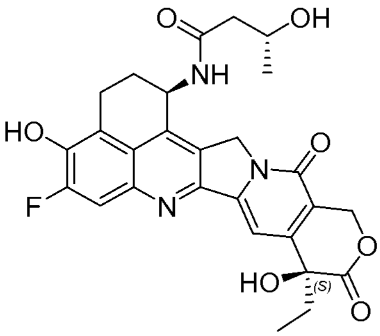,
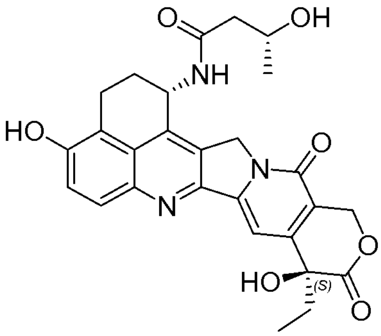,

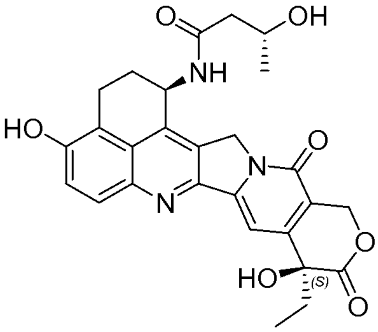,
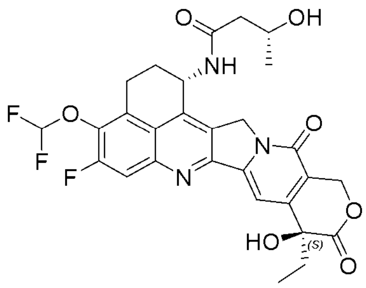,
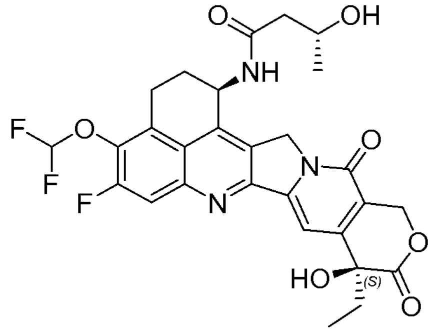,

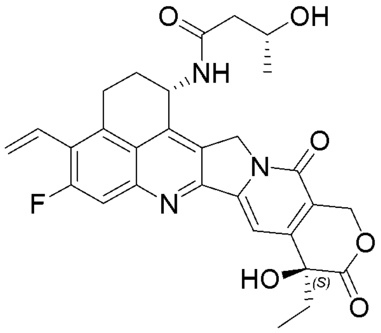,
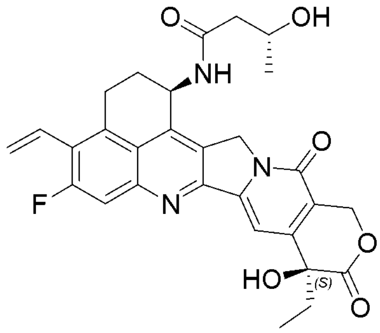,
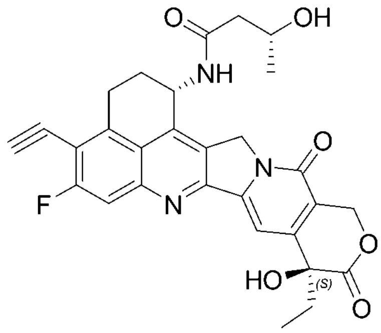,

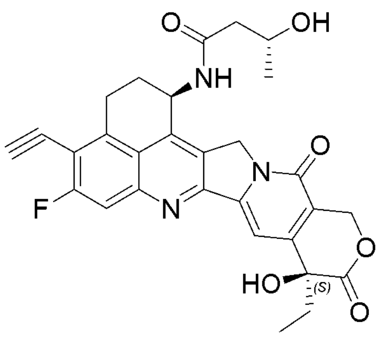,
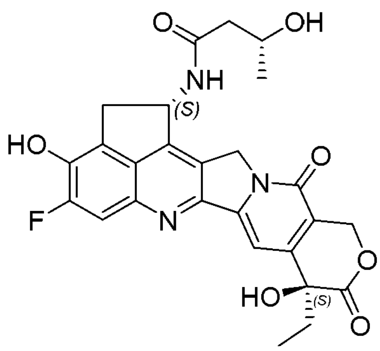,
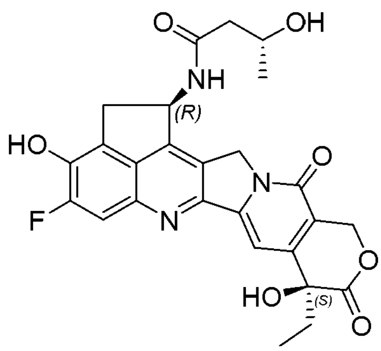,

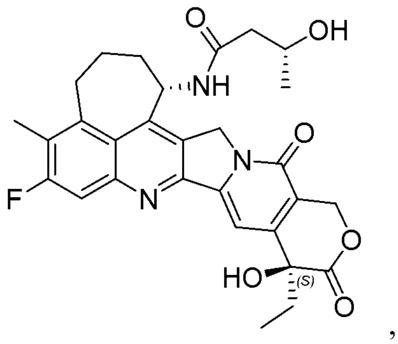,
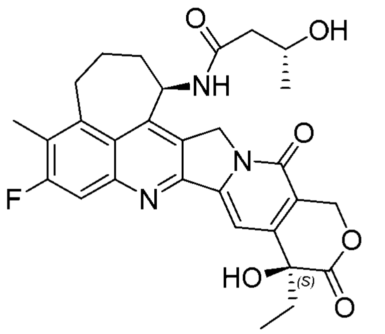,
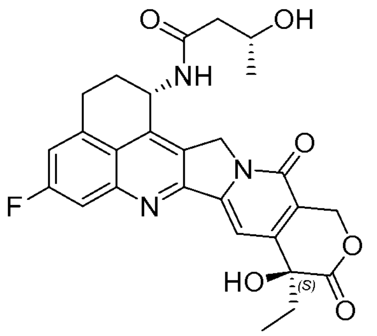,

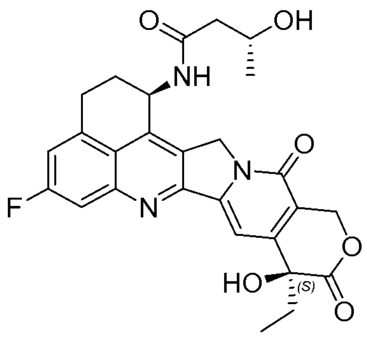,
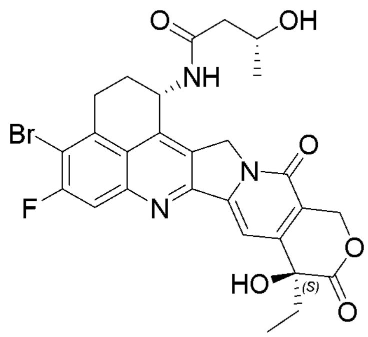,
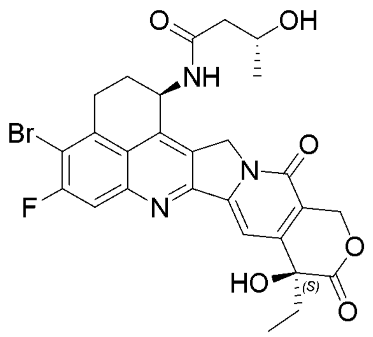,

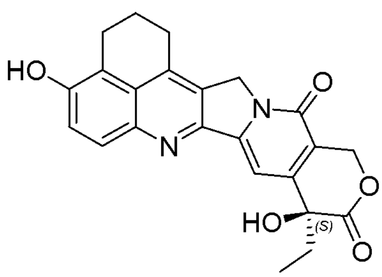,
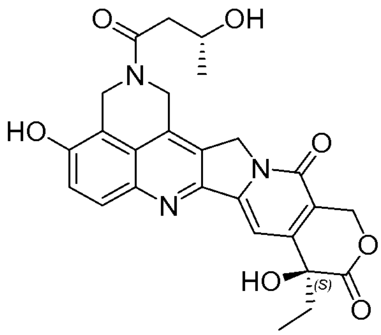,
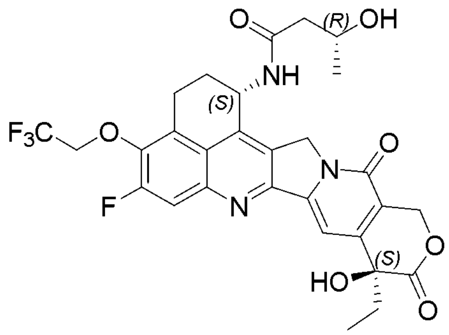

,
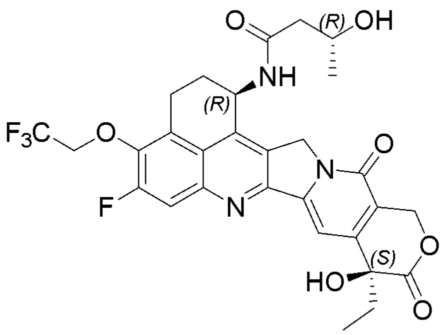,
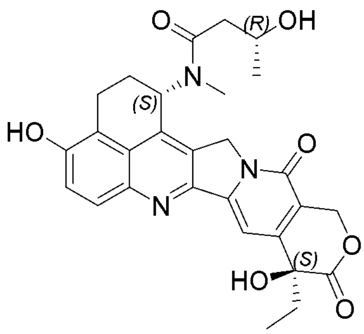,
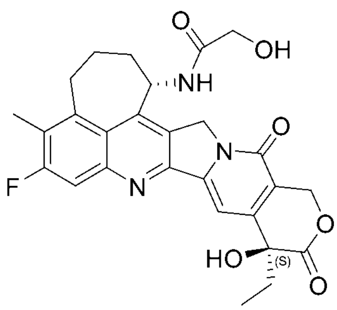,

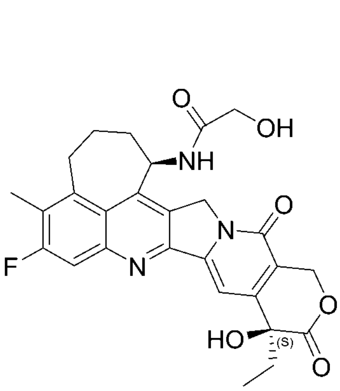,
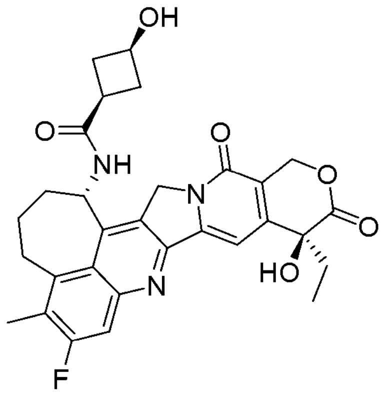,
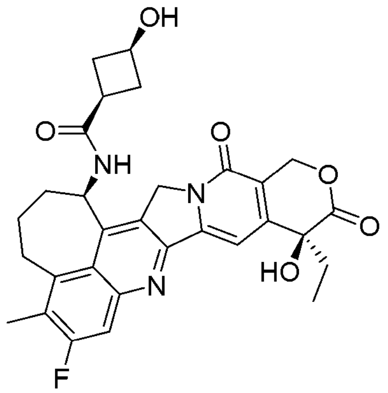,

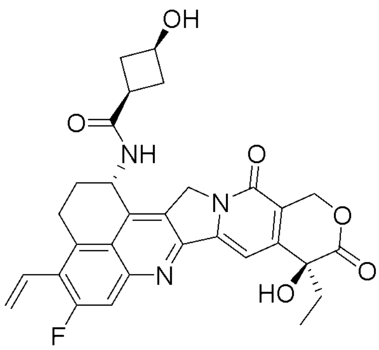,
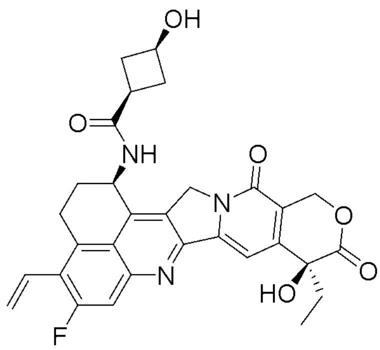,
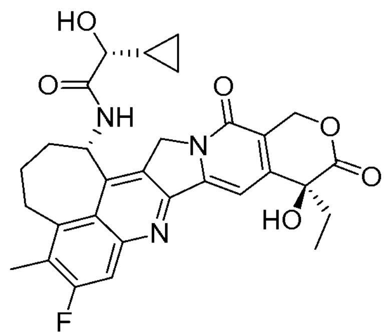,

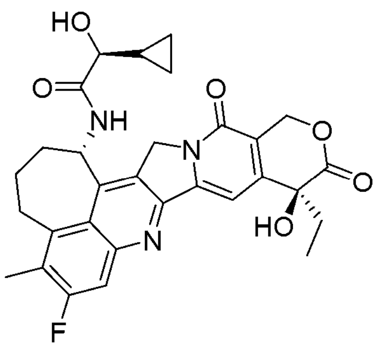,
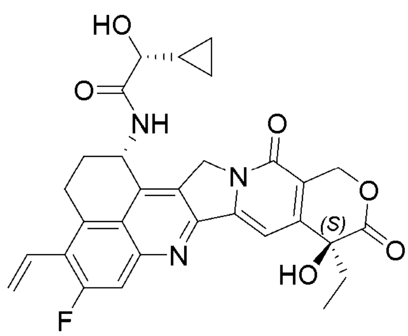,
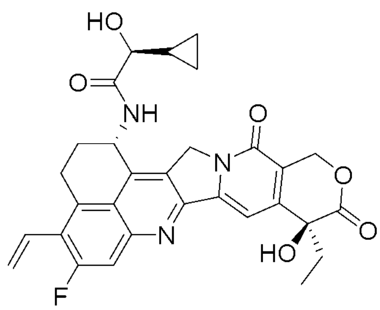,

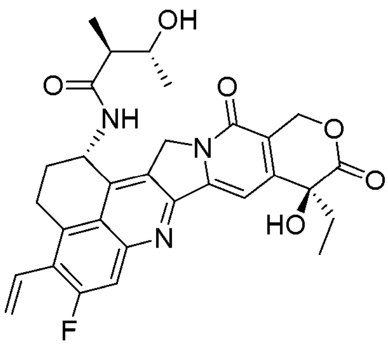,
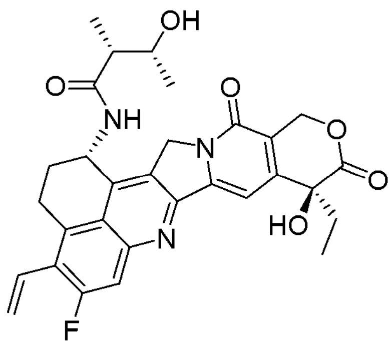,
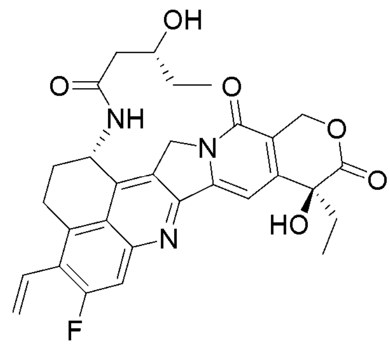,

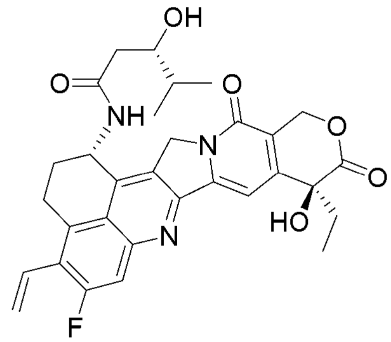,
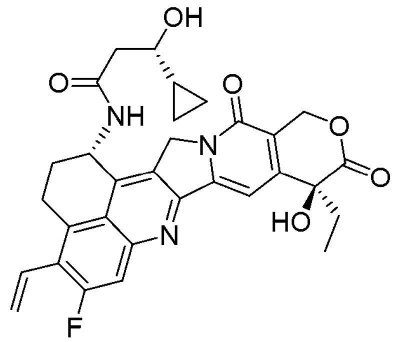,
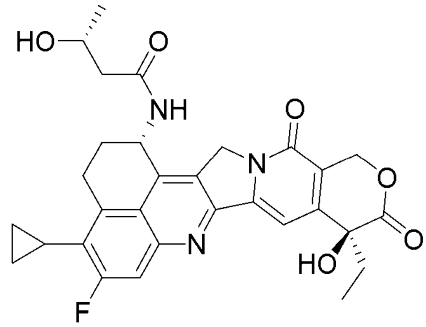,

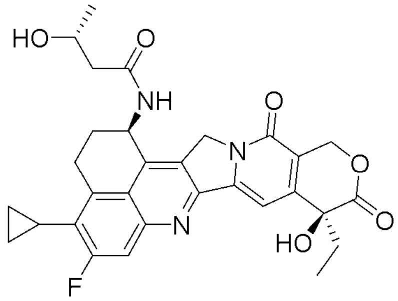,
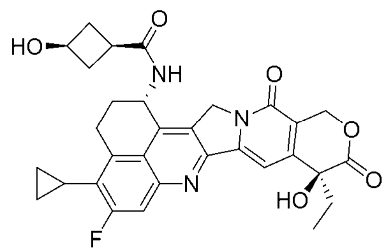,
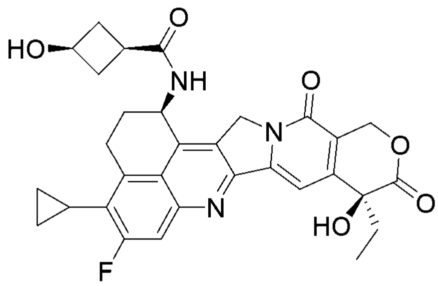

,
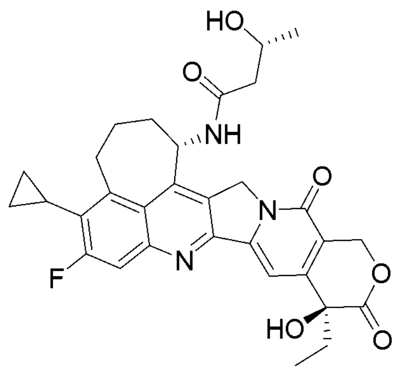,
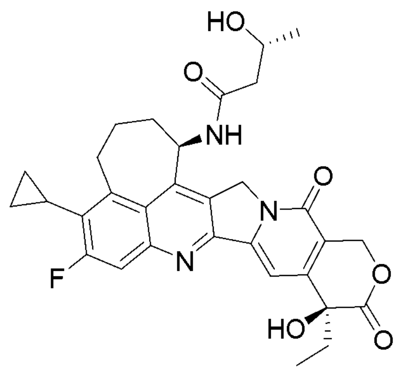,
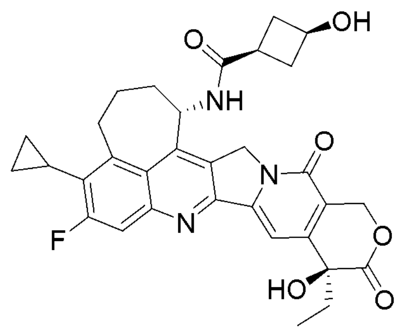,

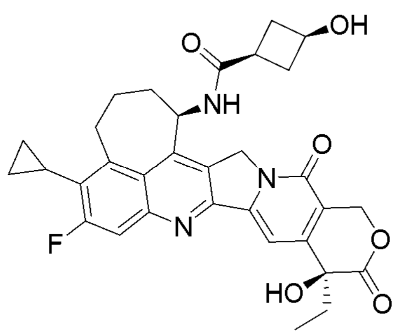,
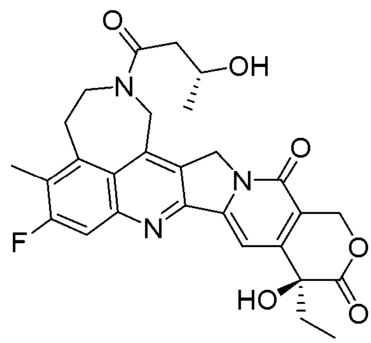,
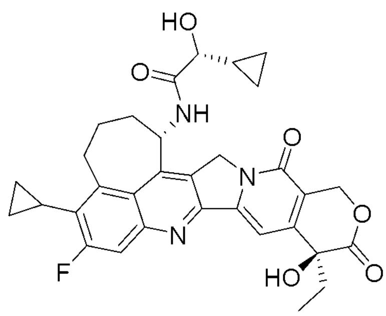,

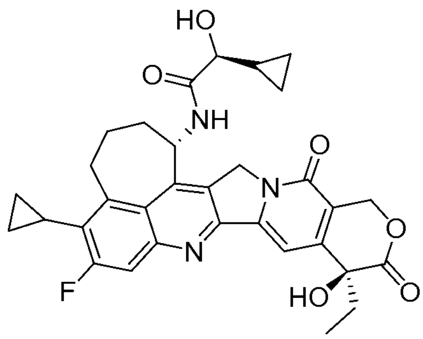 , 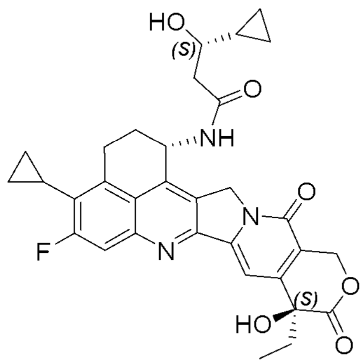 , 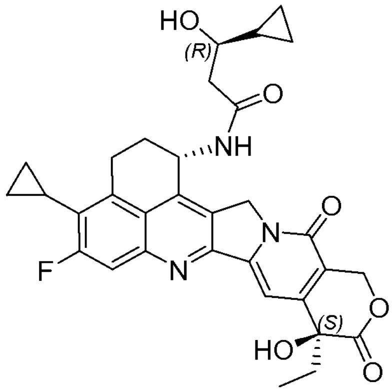 ,

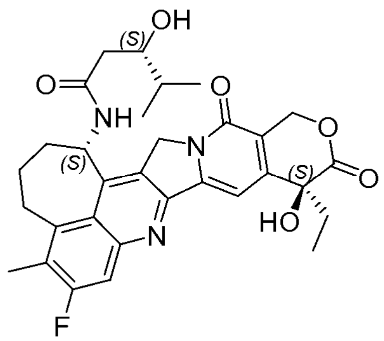 , 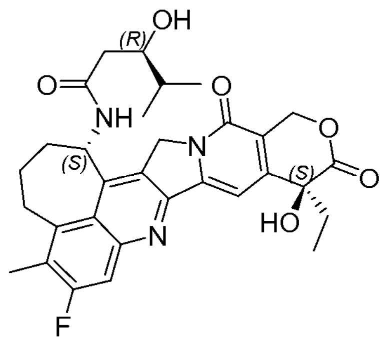 , 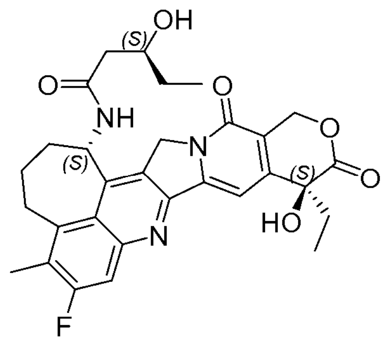 ,

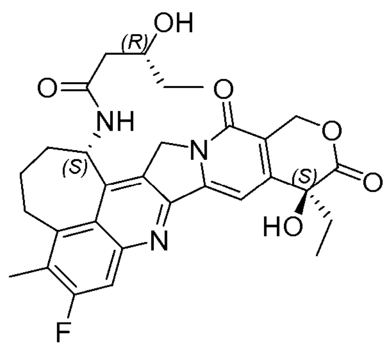 , 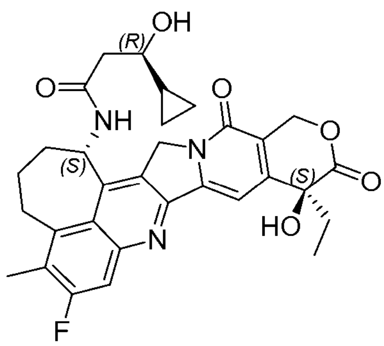 , 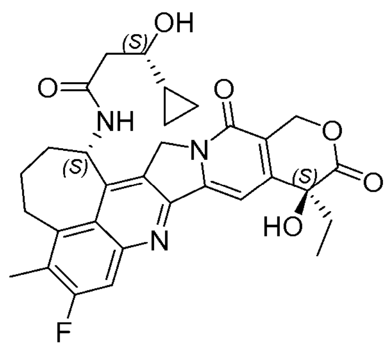 ,

or

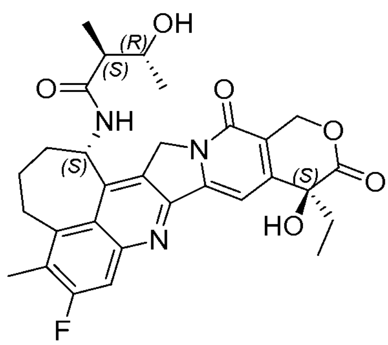 .

According to some embodiments, the present invention further provides a structural fragment D of the compound represented by formula I herein after dehydrogenation; and
according to some embodiments, the D has a structure as shown below:

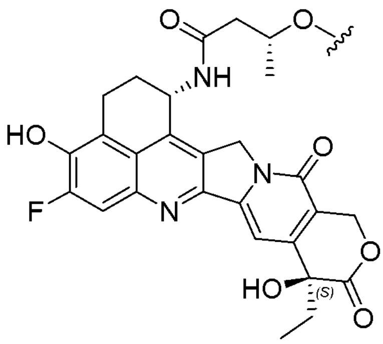
,
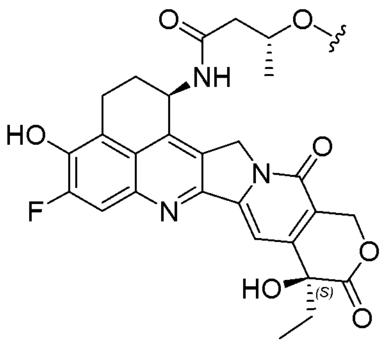
,
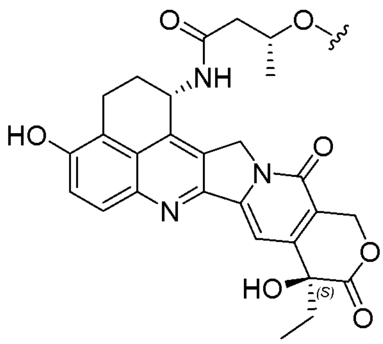
,
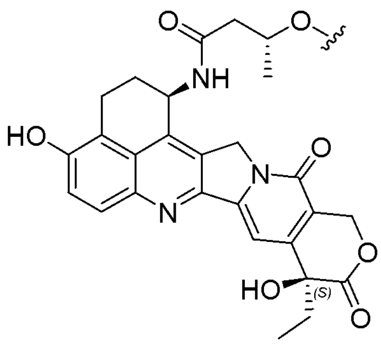
,
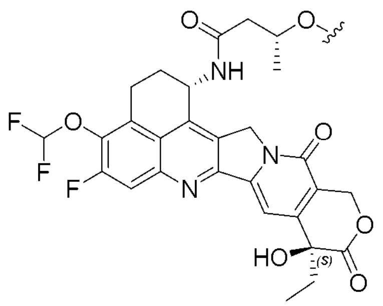
,
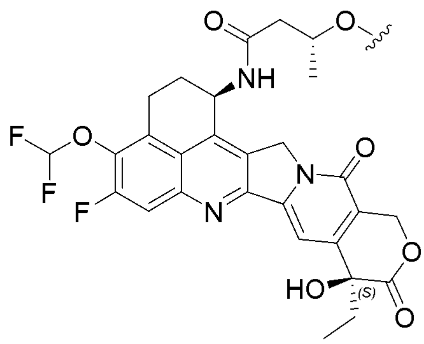
,
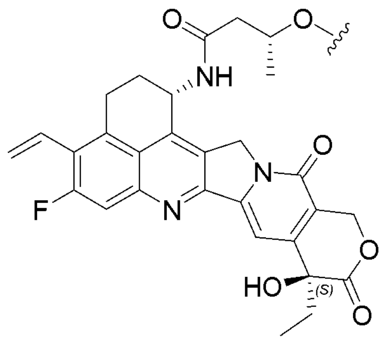
,
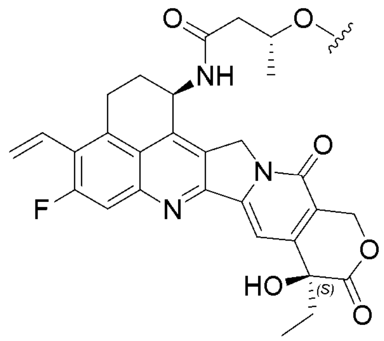
,
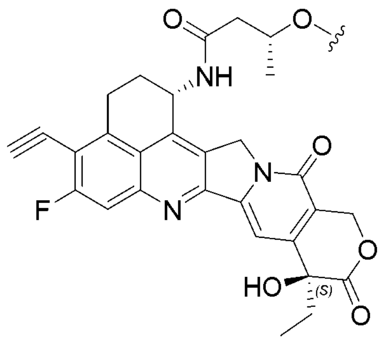
,
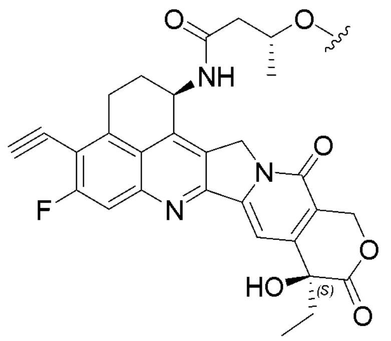
,
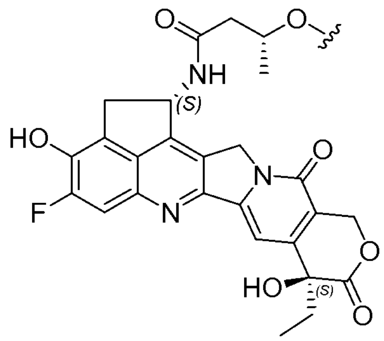
,
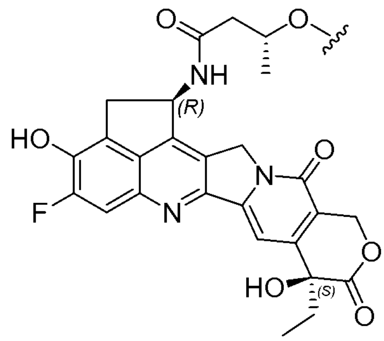
,

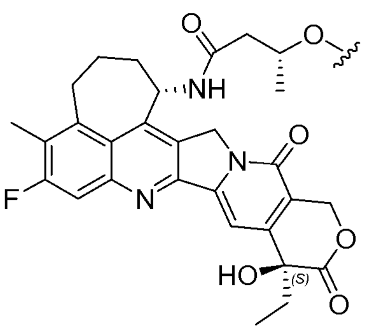
,
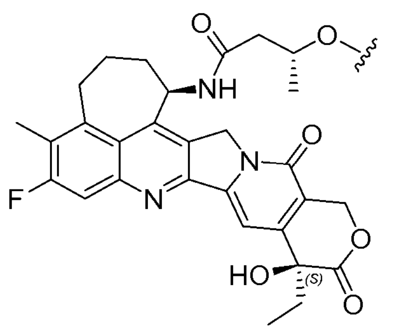
,
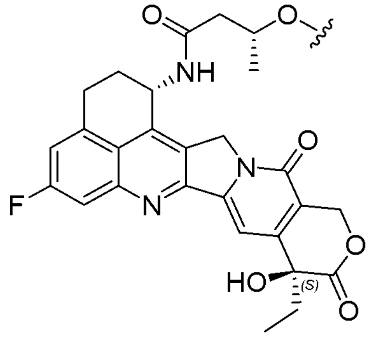
,
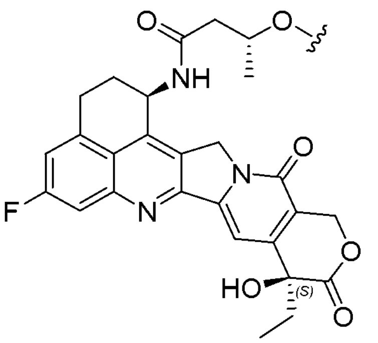
,
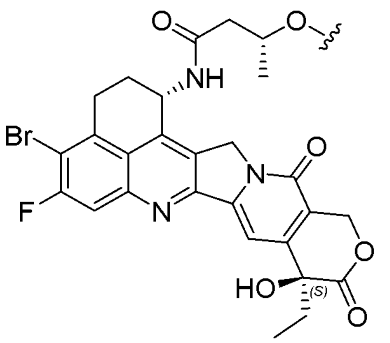
,
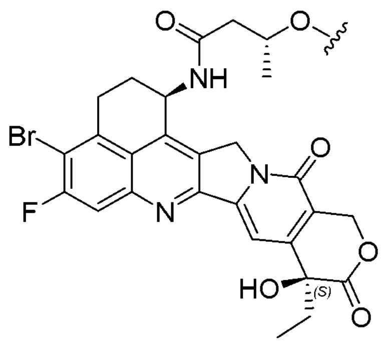
,
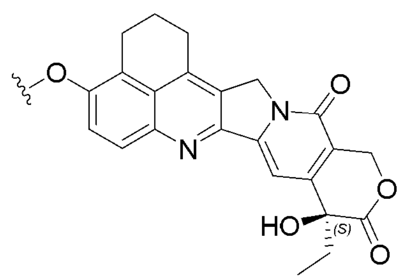
,
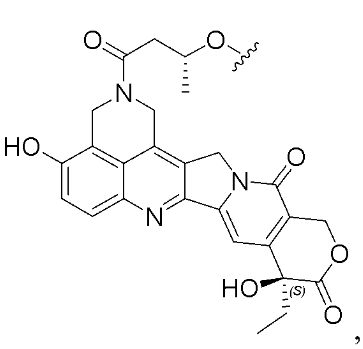
,
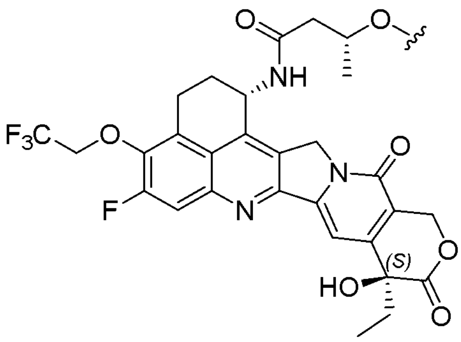

,
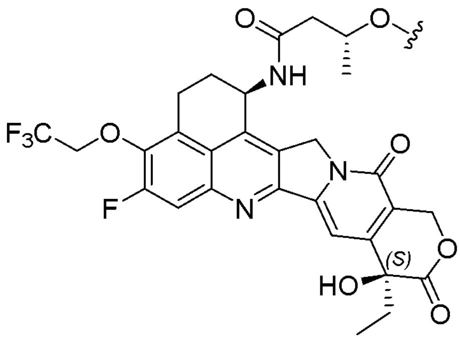
,
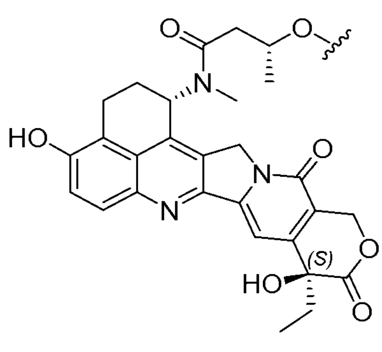
,
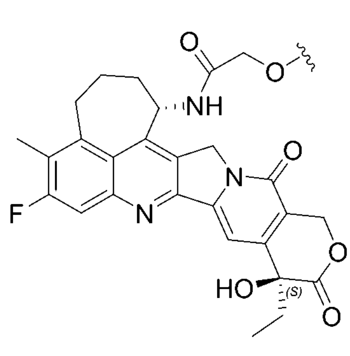

,

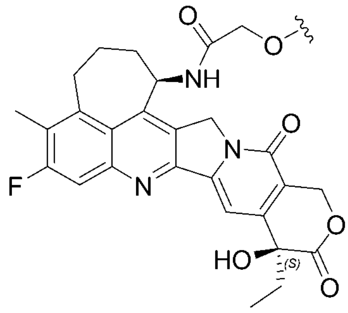

,

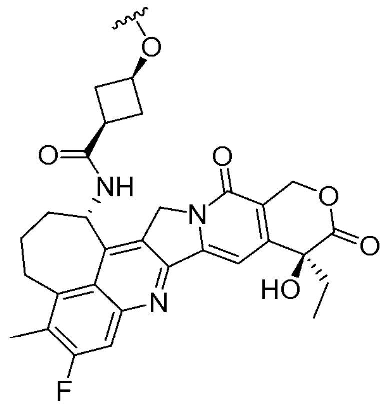

,

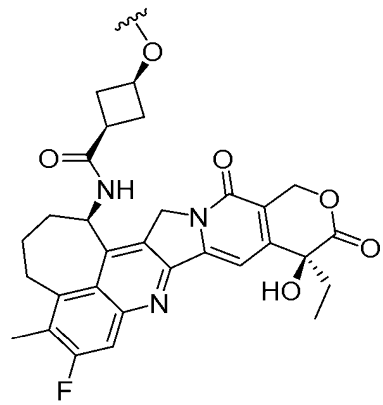

,

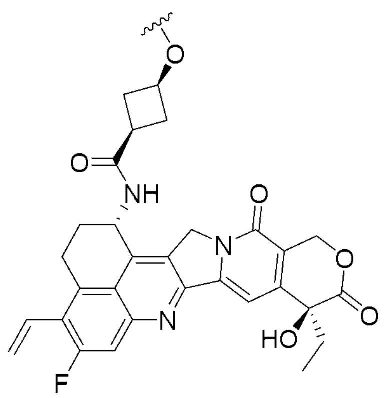

,

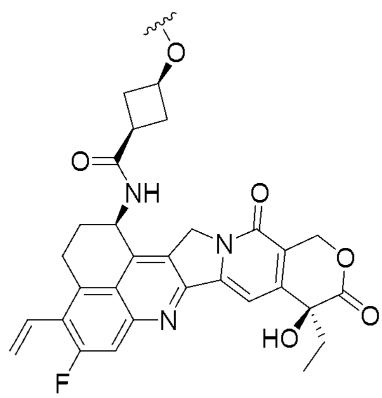

,

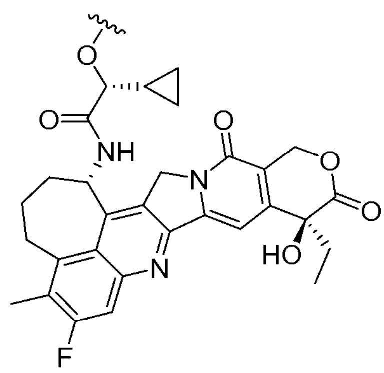

,

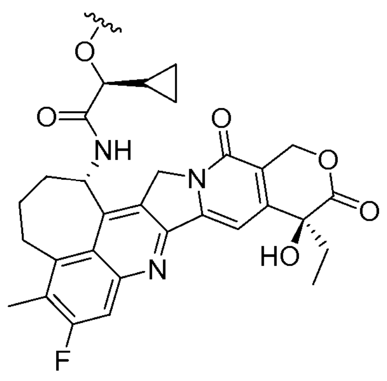

,

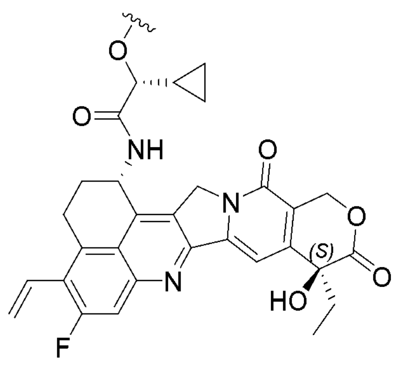

,

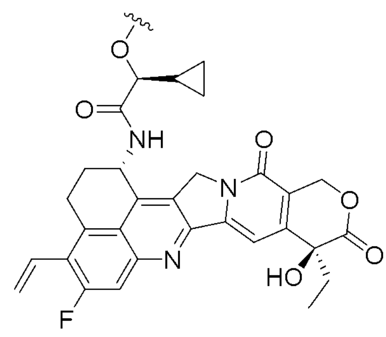

,

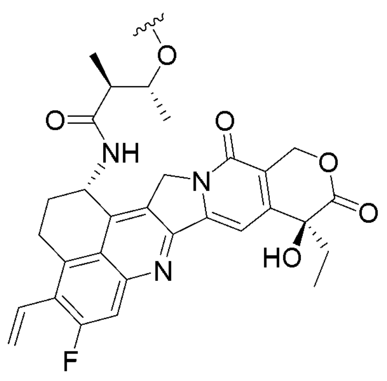

,

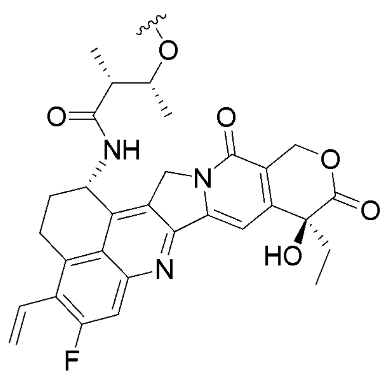

,

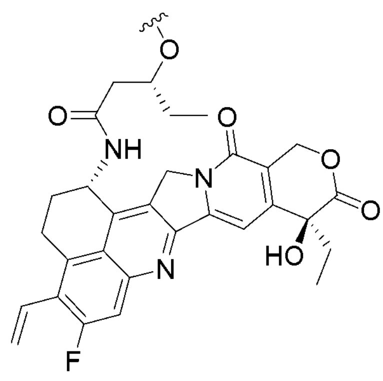

,

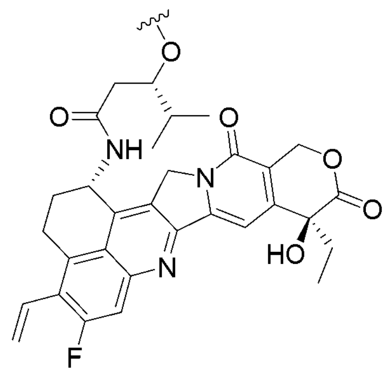,
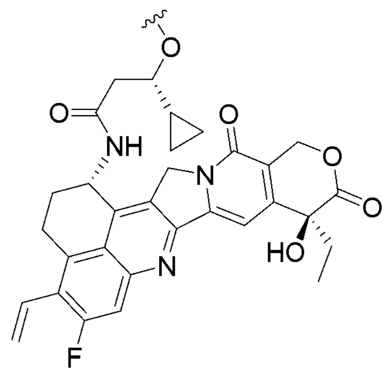,
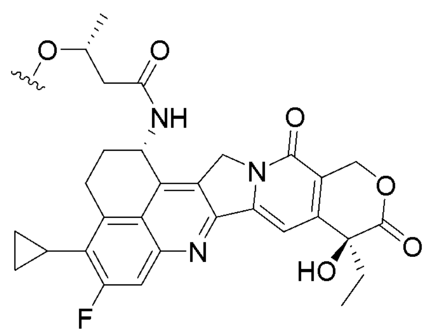,

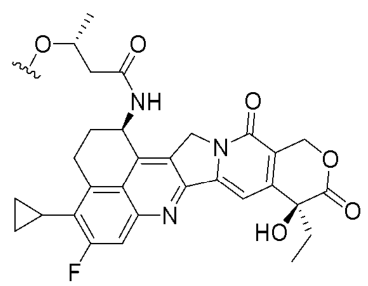,
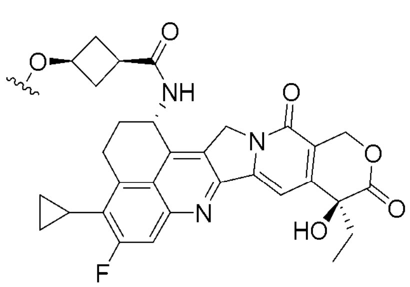,
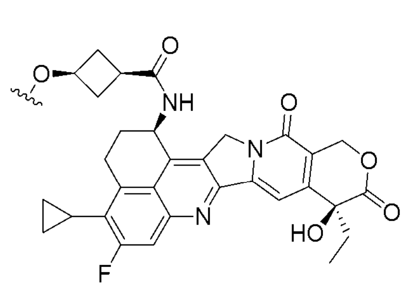,

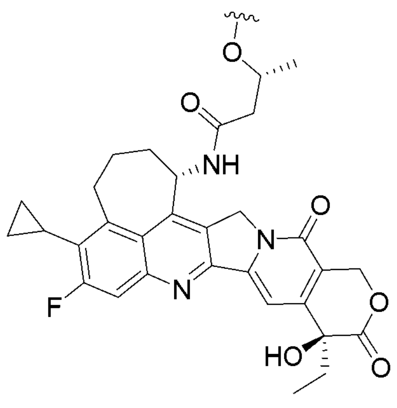,
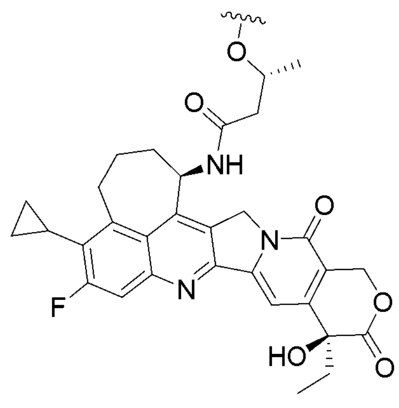,
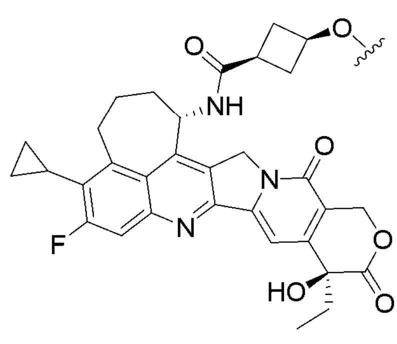,

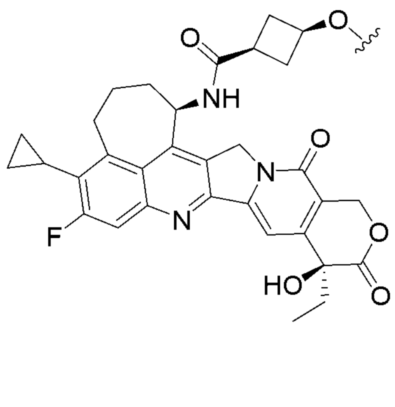,
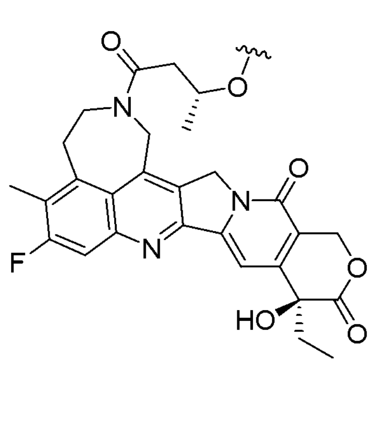,
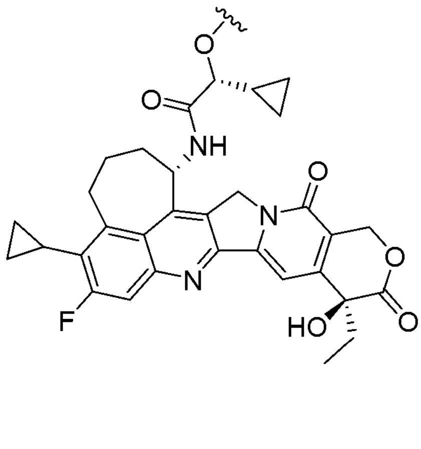,

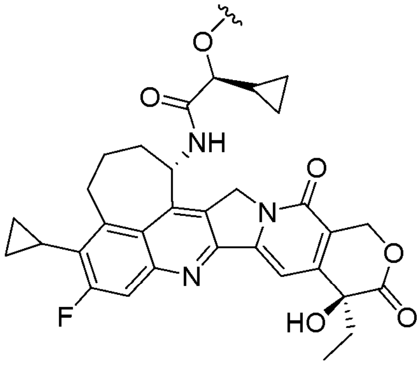,
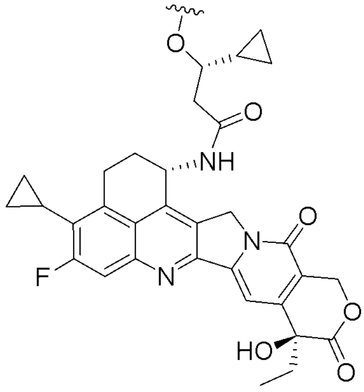,
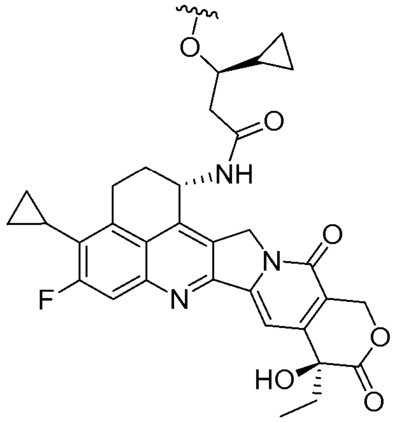,

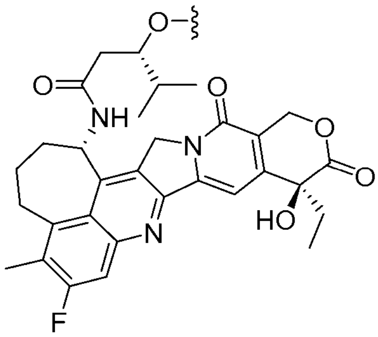,
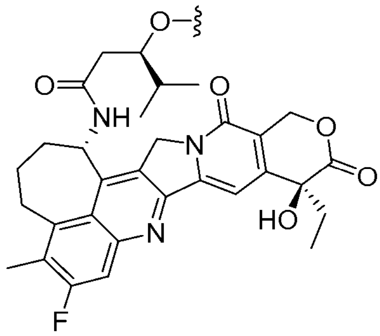,
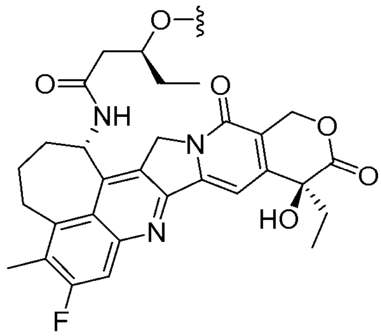,

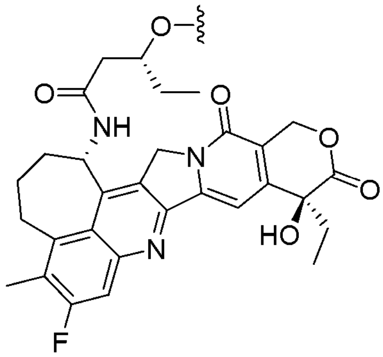,
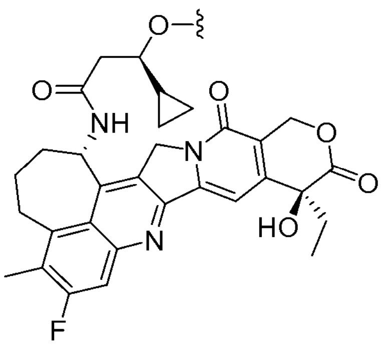,
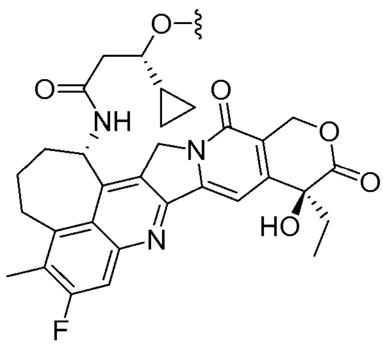,

or

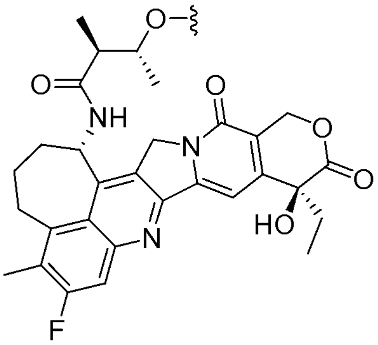.

The present invention further provides a compound represented by formula V, a racemate, a stereoisomer, a tautomer, an isotopically labeled compound, a solvate, a polymorph, a pharmaceutically acceptable salt, or a prodrug compound thereof:

L'-D (formula V)

wherein L' is a linker containing a linker site M capable of reacting with an antibody or an antigen-binding fragment thereof, and after L'-D reacts with the antibody or the antigen-binding fragment thereof, the L' thereof forms a linker L; and

preferably, the L' comprises a peptide residue $L_1$ and a fragment $L_2$ in which the peptide residue is connected to D.

The D is as defined herein.

The present invention further provides an antibody-drug conjugate represented by formula VI,

Ab-[L-D]$_\beta$ (formula VI)

wherein Ab is an antibody or an antigen-binding fragment thereof, D is as defined in the text of the present application, L is a linker linking the Ab to the D, and β is an integer or a decimal selected from 1-10.

The present invention further provides a method for preparing the compound of formula I, comprising solution I or solution II below:

solution I: comprising steps of:

(1) removing a protecting group $PG_4$ from compound I-41 to give compound I-42; and

(2) allowing the compound I-42 to react with compound I-43 to give the compound represented by formula I;

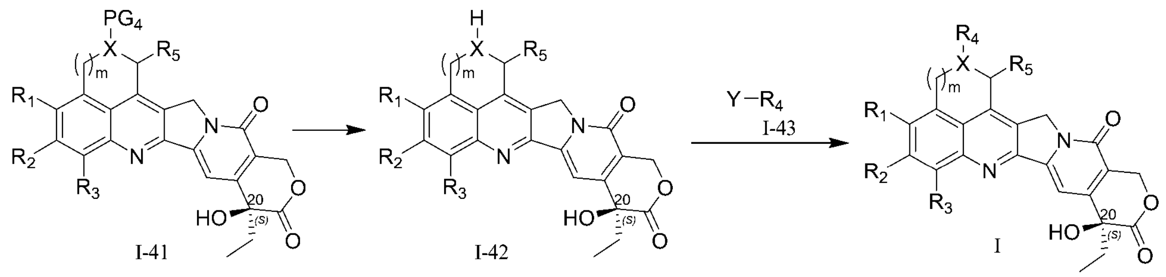

I-41 I-42 I

wherein the $R_1$, $R_2$, $R_3$, $R_4$, $R_5$, X, and m are as defined herein; Y is selected from a leaving group, for example, OH, Cl, Br, or I; and the $PG_4$ is selected from an amino protecting group, for example, Fmoc, Boc, Bn, or Cbz; or

solution II: comprising steps of:

(1) removing a protecting group $PG_5$ from compound I-51 to give compound I-52; and

(2) allowing the compound I-52 to react with compound I-53 to give the compound represented by formula I;

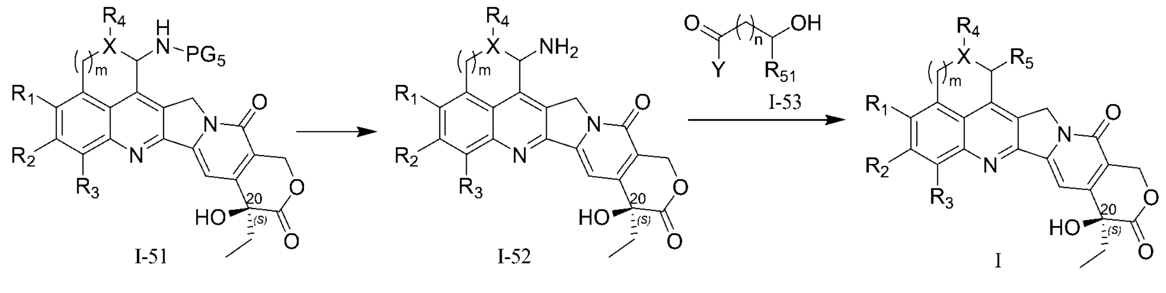

I-51 I-52 I

wherein the $R_1$, $R_2$, $R_3$, $R_4$, $R_5$, $R_{51}$, X, m, and n are as defined herein; Y is selected from a leaving group, for example, OH, Cl, Br, or I; and the $PG_5$ is selected from an amino protecting group, for example, Fmoc, Boc, Bn, or Cbz.

The present invention further provides a pharmaceutical composition, comprising a therapeutically effective amount of at least one of the compound represented by formula I or formula V, a racemate, a stereoisomer, a tautomer, an isotopically labeled compound, a solvate, a polymorph, a pharmaceutically acceptable salt, or a prodrug compound thereof.

The present invention further provides a pharmaceutical composition, comprising a therapeutically effective

amount of the antibody-drug conjugate represented by formula VI.

According to an embodiment of the present invention, the pharmaceutical composition further comprises one or more pharmaceutically acceptable excipients.

According to an embodiment of the present invention, the pharmaceutical composition may further comprise one or more additional therapeutic agents.

The present invention further provides a method for treating a tumor disease, comprising administering to a patient a prophylactically or therapeutically effective amount of at least one of a compound represented by formula I or formula V, or an antibody-drug conjugate represented by formula VI, a racemate, a stereoisomer, a tautomer, an isotopically labeled compound, a solvate, a polymorph, a pharmaceutically acceptable salt, or a prodrug compound thereof.

The present invention further provides a method for treating a tumor disease, comprising administering to a patient a prophylactically or therapeutically effective amount of the above pharmaceutical composition.

The tumor disease is selected from breast cancer, gastric cancer, lung cancer, colorectal cancer, large intestine cancer, ovarian cancer, liver cancer, kidney cancer, esophageal cancer, cervical cancer, bladder cancer, pancreatic cancer, prostate cancer, nasopharyngeal carcinoma, melanoma, or leukemia.

In some embodiments, the patient comprises a mammal, and is preferably a human.

The present invention further provides at least one of a compound represented by formula I or formula V, or an antibody-drug conjugate represented by formula VI, a racemate, a stereoisomer, a tautomer, an isotopically labeled compound, a solvate, a polymorph, a pharmaceutically acceptable salt, or a prodrug compound thereof, or a pharmaceutical composition thereof for treating a tumor disease.

The present invention further provides use of at least one of the compound represented by formula I or formula V, or the antibody-drug conjugate represented by formula VI, a racemate, a stereoisomer, a tautomer, an isotopically labeled compound, a solvate, a polymorph, a pharmaceutically acceptable salt, or a prodrug compound thereof, or the above pharmaceutical composition for the manufacture of a topoisomerase I inhibitor and/or for the manufacture of a medicament for preventing or treating a disease or condition associated with topoisomerase I.

In some embodiments, the disease or condition is a tumor, comprising breast cancer, gastric cancer, lung cancer, colorectal cancer, large intestine cancer, ovarian cancer, liver cancer, kidney cancer, esophageal cancer, cervical cancer, bladder cancer, pancreatic cancer, prostate cancer, nasopharyngeal carcinoma, melanoma, or leukemia.

## Beneficial effects

The compound provided in the present invention has good tumor inhibiting effects, can be used for treating or preventing a cancer (for example, breast cancer or gastric cancer), and can be used for preparing a medicament for treating or preventing such a condition or disease.

## Definitions and description of terms

Unless otherwise indicated, the definitions of radicals and terms recorded in the specification and claims of the present application, comprising their definitions as examples, exemplary definitions, preferred definitions, definitions recorded in tables, definitions of specific compounds in examples, etc., may be arbitrarily combined and associated with each other. Such combined and associated radical definitions and compound structures should be understood to be within the scope of the specification and/or claims of the present application.

Unless otherwise indicated, the numerical ranges recited in the present specification and claims are equivalent to at least reciting each specific integer value therein. For example, the numerical range "1-12" is equivalent to reciting each integer value in the numerical range "1-12," namely 1, 2, 3, 4, 5, 6, 7, 8, 9, 10, 11, and 12. In addition, when a numerical range is defined as a "number," it should be understood that both endpoints of the range, each integer within the range, and each decimal within the range are recited.

The term "halogen" represents fluorine, chlorine, bromine, and iodine.

The "$C_{1-10}$ alkyl" represents a linear and branched alkyl having 1, 2, 3, 4, 5, 6, 7, 8, 9, or 10 carbon atoms, the "$C_{1-8}$ alkyl" represents a linear and branched alkyl having 1, 2, 3, 4, 5, 6, 7, or 8 carbon atoms, and the "$C_{1-6}$ alkyl" represents a linear and branched alkyl having 1, 2, 3, 4, 5, or 6 carbon atoms. The alkyl is, e.g., methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, etc., or isomers thereof.

The "$C_{2-10}$ alkenyl" should be construed to represent preferably a linear or branched monovalent hydrocarbon radical comprising one or more double bonds and having 2, 3, 4, 5, 6, 7, 8, 9, or 10 carbon atoms, and is more preferably "$C_{2-8}$ alkenyl". The "$C_{2-10}$ alkenyl" should be construed to represent preferably a linear or branched monovalent

hydrocarbon radical comprising one or more double bonds and having 2, 3, 4, 5, 6, 7, or 8 carbon atoms, for example, having 2, 3, 4, 5, or 6 carbon atoms (namely, $C_{2-6}$ alkenyl) or having 2 or 3 carbon atoms (namely, $C_{2-3}$ alkenyl). It should be understood that in the case where the alkenyl comprises more than one double bond, the double bonds may be separated from each other or conjugated. The alkenyl is, e.g., ethenyl, allyl, (E)-2-methylethenyl, (Z)-2-methylethenyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propyethenyl, or 1-isopropylethenyl.

The "$C_{2-10}$ alkynyl" should be construed to represent preferably a linear or branched monovalent hydrocarbon radical comprising one or more triple bonds and having 2, 3, 4, 5, 6, 7, 8, 9, or 10 carbon atoms, for example, having 2, 3, 4, 5, 6, 7, or 8 carbon atoms (namely, "$C_{2-8}$ alkynyl"), having 2, 3, 4, 5, or 6 carbon atoms (namely, "$C_{2-6}$ alkynyl"), or having 2 or 3 carbon atoms (namely, "$C_{2-3}$ alkynyl"). The alkynyl is, e.g., ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl.

The term "$C_{3-10}$ cycloalkyl" should be construed to represent saturated monovalent monocyclic, bicyclic (e.g., bridged cyclic or spirocyclic) hydrocarbon ring or tricyclic alkane having 3, 4, 5, 6, 7, 8, 9, or 10 carbon atoms. The $C_{3-10}$ cycloalkyl may be a monocyclic hydrocarbon radical such as cyclopropyl, cyclobutyl, cyclopentyl, cyclohexyl, cycloheptyl, cyclooctyl, cyclononyl, or cyclodecyl, or may be a bicyclic hydrocarbon radical such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonyl, or 2,6-diazaspiro[3,4]octyl, or may be a tricyclic hydrocarbon radical such as adamantyl.

Unless otherwise defined, the term "3-6-membered heterocyclyl" refers to a saturated or unsaturated non-aromatic ring or ring system, for example, is a 4-, 5-, or 6-membered monocyclic ring, and contains at least one, for example, 1, 2, 3, 4, 5, or more heteroatoms selected from O, S, and N, wherein N and S may further be optionally oxidized to various oxidation states to form nitrogen oxides, -S(O)-, or $-S(O)_2-$ states. The heterocyclyl may comprise a fused or bridged ring and a spirocyclic ring. In particular, the heterocyclyl may include, but is not limited to: a 4-membered ring, such as azetidinyl or oxetanyl; a 5-membered ring, such as tetrahydrofuryl, dioxolyl, pyrrolidyl, imidazolidinyl, pyrazolidyl, or pyrrolinyl; or a 6-membered ring, such as tetrahydropyranyl, piperidyl, morpholinyl, dithianyl, thiomorpholinyl, piperazinyl, or trithianyl.

The term "$C_{6-14}$ aryl" should be construed to represent preferably a monovalent aromatic or partially aromatic monocyclic, bicyclic, or tricyclic hydrocarbon ring having 6, 7, 8, 9, 10, 11, 12, 13, or 14 carbon atoms ("$C_{6-14}$ aryl"), particularly a ring having 6 carbon atoms ("$C_6$ aryl"), such as phenyl; or biphenyl, or a ring having 9 carbon atoms ("$C_9$ aryl"), such as indanyl or indenyl, or a ring having 10 carbon atoms ("$C_{10}$ aryl"), such as tetralyl, dihydronaphthyl, or naphthyl, or a ring having 13 carbon atoms ("$C_{13}$ aryl"), such as fluorenyl, or a ring having 14 carbon atoms ("$C_{14}$ aryl"), such as anthranyl. When the $C_{6-20}$ aryl is substituted, it can be monosubstituted or polysubstituted. Further, the substitution site is not limited, for example, may be ortho-, para-, or meta-substitution.

The term "5-14-membered heteroaryl" should be construed to comprise such a monovalent monocyclic, bicyclic, or tricyclic aromatic ring system that has 5, 6, 7, 8, 9, 10, 11, 12, 13, or 14 ring atoms, particularly 5 or 6 or 9 or 10 carbon atoms, and comprises from 1 to 5, preferably from 1 to 3, heteroatoms each independently selected from N, O, and S, and, further, may be additionally benzo-fused in each circumstance. "Heteroaryl" also means a radical in which a heteroaromatic ring is fused with one or more aryl, alicyclic, or heterocyclic rings, wherein the radical or site of attachment is on the heteroaromatic ring. Non-limiting examples comprise 1-, 2-, 3-, 5-, 6-, 7-, or 8-indolizinyl, 1-, 3-, 4-, 5-, 6-, or 7-isoindolyl, 2-, 3-, 4-, 5-, 6-, or 7-indolyl, 2-, 3-, 4-, 5-, 6-, or 7-indazolyl, 2-, 4-, 5-, 6-, 7-, or 8-purinyl, 1-, 2-, 3-, 4-, 6-, 7-, 8-, or 9-quinolizinyl, 2-, 3- , 4-, 5-, 6-, 7- or 8-quinolinyl, 1-, 3-, 4-, 5-, 6-, 7- or 8-isoquinolinyl, 1-, 4-, 5-, 6-, 7- or 8-phthalazinyl, 2-, 3-, 4-, 5-, or 6-naphthyridinyl, 2-, 3-, 5-, 6-, 7- or 8-quinazolinyl, 3-, 4-, 5-, 6-, 7- or 8-cinnolinyl, 2-, 4-, 6-, or 7-pteridinyl, 1-, 2-, 3-, 4-, 5-, 6-, 7-, or 8-4aH carbazolyl, 1-, 2-, 3-, 4-, 5-, 6-, 7-, or 8-carbazolylcarbazolyl, 1-, 3-, 4-, 5-, 6-, 7-, 8-, or 9-carbolinyl, 1-, 2-, 3-, 4-, 6-, 7-, 8-, 9-, or 10-phenanthridinyl, 1-, 2-, 3-, 4-, 5-, 6-, 7-, 8-, or 9-acridinyl, 1-, 2-, 4-, 5-, 6-, 7-, 8-, or 9-pyridyl, 2-, 3-, 4-, 5-, 6-, 8-, 9-, or 10-phenanthrolinyl, 1-, 2-, 3-, 4-, 6-, 7-, 8-, or 9-phenazinyl, 1-, 2-, 3-, 4-, 6-, 7-, 8-, 9-, or 10-phenothiazinyl, 1-, 2-, 3-, 4-, 6-, 7-, 8-, 9-, or 10-phenazinyl , 2-, 3-, 4-, 5-, 6-or 1-, 3-, 4-, 5-, 6-, 7-, 8-, 9- or 10-benzoisoquinolyl, 2-, 3-, 4- or thieno[2,3-b]furanyl, 2-, 3-, 5-, 6-, 7-, 8-, 9-, 10- or 11-7H-pyrazino[2,3-c]carbazolyl, 2-, 3-, 5-, 6- or 7-2H-furo[3,2-b]pyranyl ,

2-, 3-, 4-, 5-, 7-, or 8-5H-pyrido[2,3-d]-o-oxazinyl, 1-, 3-, or 5-1H-pyrazolo[4,3-d]-oxazolyl, 2-, 4-, or 54H-imidazo[4,5-d] thiazolyl, 3-, 5-, or 8-pyrazino[2,3-d]pyridazinyl, 2-, 3-, 5-, or 6-imidazo[2,1-b]thiazolyl, 1-, 3-, 6-, 7-, 8-, or 9 -furo[3,4-c] cinnolinyl, 1-, 2-, 3-, 4-, 5-, 6-, 8-, 9-, 10 or 11-4H-pyrido[2,3-c]carbazolyl, 2-, 3-, 6- or 7-imidazo[1,2-b][1,2,4]triazinyl, 7-benzo[b]thienyl, 2-, 4-, 5-, 6-, or 7-benzoxazolyl, 2-, 4-, 5-, 6-, or 7-benzimidazolyl, 2-, 4- , 4-, 5-, 6-, or 7-benzothiazolyl, 1-, 2-, 4-, 5-, 6-, 7-, 8-, or 9-benzoxapinyl, 2-, 4-, 5-, 6-, 7-, or 8-benzoxazinyl, 1-, 2-, 3-, 5-, 6-, 7-, 8-, 9-, 10-, or 11-4H-pyrrolo [1,2-b][2]benzazepinyl. A typical fused heteroaryl includes, but is not limited to, 2-, 3-, 4-, 5-, 6-, 7-, or 8-quinolyl, 1-, 3-, 4-, 5-, 6-, 7-, or 8-isoquinolyl, 2-, 3-, 4-, 5-, 6-, or 7-indolyl, 2-, 3-, 4-, 5-, 6-, or 7-benzo[b]thienyl, 2-, 4-, 5-, 6-, or 7-benzoxazolyl, 2-, 4-, 5-, 6-, or 7-benzimidazolyl, and 2-, 4-, 5-, 6-, or 7-benzothiazolyl. The 5-20-membered heteroaryl may be connected with other radicals to form the compound of the present invention by connecting a carbon atom on a 5-20-membered heteroaryl ring with the other radicals, or by connecting a heteroatom on a 5-20-membered heteroaryl ring with the other radicals. When the 5-20-membered heteroaryl is substituted, it may be monosubstituted or polysubstituted. Further, the substitution site is not limited, for example, hydrogen attached to a carbon atom on the heteroaryl ring may be substituted, or hydrogen attached to a heteroatom on the heteroaryl ring may be substituted.

The term "spirocyclic ring" refers to a ring system in which two rings share 1 ring-forming atom.

The term "fused ring" refers to a ring system in which two rings share 2 ring atoms.

The term "bridged ring" refers to a ring system in which two rings share more than 3 ring-forming atoms.

Unless otherwise specified, the heterocyclyl, heteroaryl, or heteroarylene comprises all possible isomeric forms thereof, such as positional isomers thereof. Therefore, for some illustrative non-limiting examples, the substitution or bonding to other radicals may be comprised at, e.g., 1, 2, or more of its 1-, 2-, 3-, 4-, 5-, 6-, 7-, 8-, 9-, 10-, 11-, 12-C, (if present), comprising pyridin-2-yl, pyridyliden-2-yl, pyridin-3-yl, pyridyliden-3-yl, pyridin-4-yl, and pyridyliden-4-yl; and the thienyl or thienylenyl comprises thien-2-yl, thienylen-2-yl, thien-3-yl, and thienylen-3-yl; pyrazol-1-yl, pyrazol-3-yl, pyrazol-4-yl, and pyrazol-5-yl.

The term "alkoxy" refers to -O-(alkyl), wherein the alkyl is as defined above. Non-limiting examples of alkoxy comprise: methoxy, ethoxy, propoxy, and butoxy. The alkoxy may be optionally substituted or unsubstituted, and may be, when substituted, substituted with a substituent which is preferably one or more radicals independently selected from alkyl, alkenyl, alkynyl, alkoxy, alkylamino, halogen, sulfydryl, hydroxyl, nitro, cyano, cycloalkyl, heterocycloalkyl, aryl, heteroaryl, cycloalkoxy, or heterocycloalkoxy.

The term "alkylamino" refers to -NH-(alkyl), wherein the alkyl is as defined above. Non-limiting examples of alkylamino comprise, for example, methylamino, ethylamino, propylamino, isopropylamino, and butylamino.

The term "$(alkyl)_2$amino" refers to -N-$(alkyl)_2$, wherein the alkyl is as defined above. Non-limiting examples of $(alkyl)_2$amino comprise: for example, dimethylamino, methylethylamino, diethylamino, dipropylamino, methylpropylamino, diisopropylamino, and dibutylamino.

The "haloalkyl" refers to an alkyl substituted with one or more halogens, wherein the alkyl is as defined above.

The term peptide residue refers to an oligopeptide fragment in an ADC linker, which often serves as a substrate for enzymes in tumor cells or the microenvironment to control the release of a drug, such as GGFG, and has a structure of

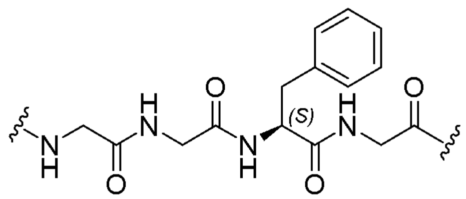

GGFG

.

Those skilled in the art will understand that the compound represented by formula (I) may exist in the form of various pharmaceutically acceptable salts. If the compounds have a basic center, they can form acid addition salts; if they have an acidic center, they can form base addition salts; if the compounds contain both an acidic center (e.g., carboxyl) and a basic center (e.g., amino), they can also form inner salts.

The compounds of the present invention may be present in the form of solvates, such as hydrates, wherein the compounds of the present invention comprise polar solvents, such as water, methanol or ethanol, as structural elements of the lattice of the compounds. The polar solvent, especially water, may be present in a stoichiometric or non-stoichiometric amount.

Depending on their molecular structure, the compounds of the present invention may be chiral and may therefore exist in various enantiomeric forms. These compounds may therefore exist in racemic or optically active forms. The compounds of the present invention encompass isomers in which the chiral carbons are each in R or S configuration, or mixtures and racemates thereof. The compounds of the present invention or their intermediates can be separated into enantiomeric compounds by chemical or physical methods known to those skilled in the art, or used in synthesis in this

form. In the case of racemic amines, diastereomers are prepared from the mixture by reaction with an optically active resolving reagent. Examples of suitable resolving reagents are optically active acids, such as the R and S forms of tartaric acid, diacetyltartaric acid, dibenzoyltartaric acid, mandelic acid, malic acid, lactic acid, appropriate N-protected amino acids (e.g., N-benzoylproline or N-phenylsulfonylproline) or various optically active camphorsulfonic acids. Chromatographic enantiomeric resolution can also be advantageously carried out by means of optically active resolving reagents, such as dinitrobenzoylphenylglycine, cellulose triacetate or other carbohydrate derivatives or chirally derivatized methacrylate polymers immobilized on silica gel. Suitable eluents for this purpose are aqueous or alcoholic solvent mixtures, for example, hexane/isopropanol/acetonitrile.

The corresponding stable isomers can be separated according to known methods, for example by extraction, filtration or column chromatography.

The term "patient" refers to any animal, comprising mammals, preferably mice, rats, other rodents, rabbits, dogs, cats, pigs, cows, sheep, horses or primates, and most preferably humans.

The term "therapeutically effective amount" refers to that amount of an active compound or drug which causes a biological or medical response sought by a researcher, veterinarian, physician or other clinician in a tissue, system, animal, individual or human, and which comprises one or more of the following: (1) prevention of disease: for example, prevention of a disease, disorder or condition in an individual who is susceptible to the disease, disorder or condition but has not yet experienced or developed the pathology or symptoms of the disease. (2) Inhibition of disease: for example, inhibition of a disease, disorder or condition (i.e., prevention of further progression of pathology and/or symptoms) in an individual undergoing or exhibiting the pathology or symptoms of the disease, disorder or condition. (3) Alleviation of disease: for example, alleviation of a disease, disorder or condition (i.e., reversion of pathology and/or symptoms) in an individual undergoing or exhibiting the pathology or symptoms of the disease, disorder or condition.

## DETAILED DESCRIPTION OF THE INVENTION

The technical solution of the present invention will be further described in detail below in conjunction with specific embodiments. It should be understood that the following embodiments are only intended to exemplify and explain the present invention and should not be construed as limiting the scope of protection of the present invention. All technologies realized based on the above contents of the present invention are comprised in the scope that the present invention intends to protect.

Unless otherwise stated, the raw materials and reagents used in the following examples are commercially available, or may be prepared in accordance with known methods.

The structures of the compounds are determined by nuclear magnetic resonance (NMR) or/and mass spectrum (MS). NMR shifts ($\delta$) are given in $10^{-6}$ (ppm). A nuclear magnetic resonance spectrometer (Bruker AVANCE-400) is used for NMR measurement with a solvent of deuterated dimethyl sulfoxide (DMSO-$d_6$), deuterated chloroform ($CDCl_3$), or deuterated methanol ($CD_3OD$), and with an internal standard of tetramethylsilane (TMS).

Agilent 1200/1290 DAD-6110/6120 Quadrupole MS/LC/MS (manufacturer: Agilent, MS model: 6110/6120 Quadrupole MS), waters ACQuity UPLC-QD/SQD (manufacturer: waters, MS model: waters ACQuity Qda Detector/-waters SQ Detector), and THERMO Ultimate 3000-Q Exactive (manufacturer: THERMO, MS model: THERMO Q Exactive) are used for MS measurement.

Agilent 1260II HPLC and Waters Acquity UPLC H-Class high performance liquid chromatograph are used for high performance liquid chromatography (HPLC) analysis.

Waters Acquity UPCC high performance liquid chromatograph is used for chiral HPLC analysis and determination.

Waters MS-triggered Prep-LC with SQD2 detector, Waters MS triggered Prep-LC with Acquity QDA detector, Waters MS-triggered Prep-LC with QDA detector, and GILSON Prep LC with UV detector are used for preparative high performance liquid chromatography.

Combiflash Rf200 (TELEDYNE ISCO) is used for CombiFlash fast preparative chromatograph.

Yantai Huanghai HSGF254 or Qingdao GF254 silica gel plate is used as the silica gel plate for thin layer chromatography, the specification of the silica gel plate for thin layer chromatography (TLC) is from 0.15 mm to 0.2 mm, and the specification for thin layer chromatography for separation and purification of products is from 0.4 mm to 0.5 mm.

Yantai Huanghai silica gel of 200-300 mesh is generally used as the carrier for silica gel column chromatography.

The average inhibition rate and $IC_{50}$ value of kinases are determined using NovoStar microplate reader (German BMG).

Known starting materials of the present invention may be synthesized using or in accordance with methods known in the art, or may be purchased from, e.g., ABCR GmbH & Co. KG, Acros Organics, Aldrich Chemical Company, Accela ChemBio Inc, or Chembee Chemicals.

Unless otherwise specified in the examples, reactions can all be carried out in an argon atmosphere or a nitrogen atmosphere.

The argon atmosphere or the nitrogen atmosphere means that the reaction flask is connected to an argon or nitrogen balloon with a volume of about 1 L.

A hydrogen atmosphere means that the reaction flask is connected to a hydrogen balloon with a volume of about 1 L.

Parr 3916EKX hydrogenator and QL-500 hydrogen generator or HC2-SS hydrogenator are used for a pressurized hydrogenation reaction.

Vacuumization and filling with hydrogen are usually repeatedly operated 3 times prior to the hydrogenation reaction.

CEM Discover-S 908860 microwave reactor is used for a microwave reaction.

Unless otherwise specified in the examples, the solution refers to an aqueous solution.

Unless otherwise specified in the examples, the reaction temperature is room temperature (20 °C-30 °C).

Thin layer chromatography (TLC) is used for monitoring of reaction processes in the examples. A developing agent used in a reaction, an eluent system of column chromatography used for purifying a compound, and a developing agent system of the thin layer chromatography comprise: A: dichloromethane/methanol system, and B: n-hexane/ethyl acetate system, wherein a volume ratio of the solvent is adjusted based on polarity of the compound, or adjusted optionally by addition of a small amount of an alkaline or acidic reagent such as triethylamine or acetic acid.

**Example 1**

(*R*)-*N*-((1*S*,9*S*)-9-ethyl-5-fluoro-4,9-dihydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-9-ethyl-5-fluoro-4,9-dihydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

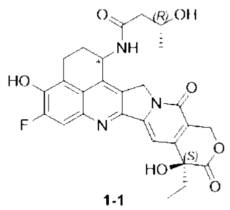

**1-1**
**1-2**

*-C represents that the chirality of the atom here is R or S

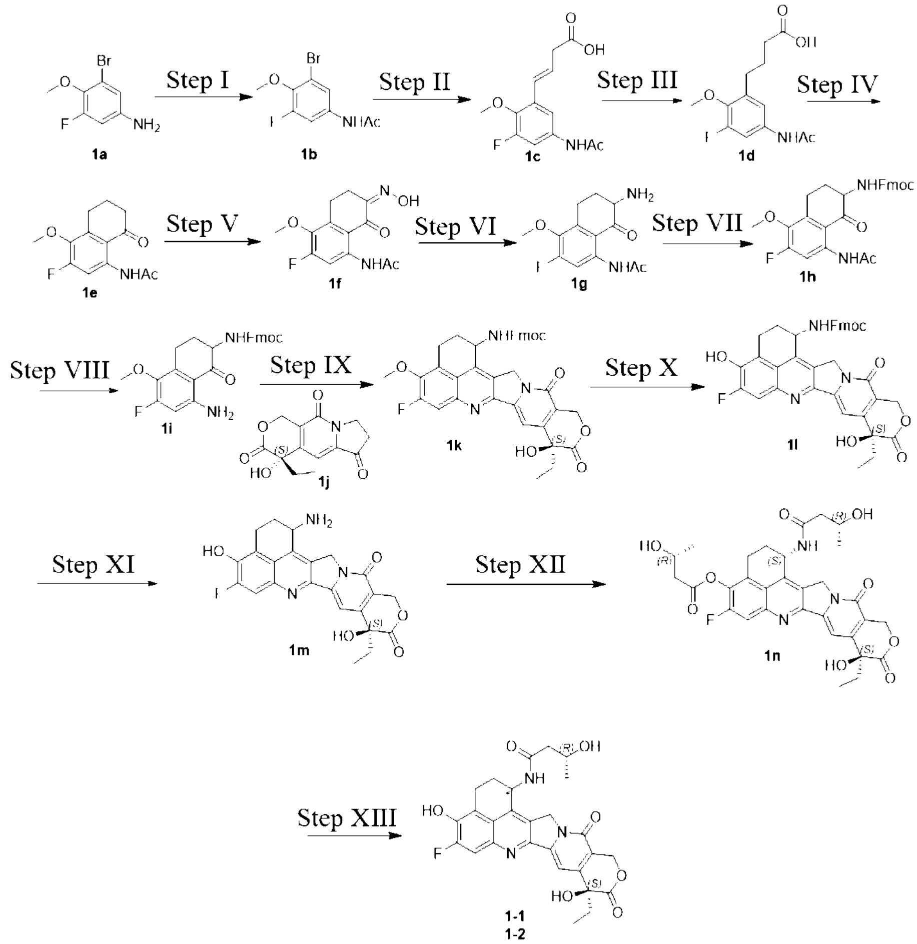


*-C represents that the chirality of the atom here is R or S

Step I *N*-(3-bromo-5-fluoro-4-methoxyphenyl)acetamide **1b**

3-bromo-5-fluoro-4-methoxyaniline **1a** (20 g, 90.8 mmol) was dissolved in dichloromethane (40 mL). After the mixture was cooled to 0°C, acetyl chloride (14.3 g, 181.6 mmol) and triethylamine (27.6 mg, 272.4 mmol) were slowly added. The reaction mixture was stirred at 0 °C for 0.5 h. After the reaction was complete, water (30 mL) was added to the reaction mixture. The mixture was extracted with dichloromethane (50 mL×3). The organic phases were combined, washed with saturated brine, dried, and concentrated. The resulting residue was purified by silica gel column chromatography with an eluent system B, to give the title compound **1b** (20 g, yield: 84%).
MS m/z (ESI): 262.1 (M+H) $^+$.

Step II (*E*)-4-(5-acetylamino-3-fluoro-2-methoxyphenyl)but-3-enoic acid **1c**

The compound **1b** (20 g, 76.3 mmol) was dissolved in dioxane (30 mL) and water (10 mL), to which but-3-enoic acid (7.23 g, 83.9 mmol), palladium acetate (1.71 g, 7.6 mmol), tris(o-methylphenyl)phosphorus (4.64 g, 15.2 mmol), and *N,N*-diisopropylethylamine (30 mg, 229 mmol) were added. The reaction mixture was stirred at 100 °C for 16 h. After the reaction was complete, the reaction mixture was filtered, and the resulting residue was purified by silica gel column chromatography with the eluent system B to give the title compound **1c** (20 g, yield: 87%).
MS m/z (ESI): 268.1 (M+H) $^+$.

Step III 4-(5-acetylamino-3-fluoro-2-methoxyphenyl)butyric acid **1d**

The compound **1c** (20 g, 74.8 mmol) was dissolved in tetrahydrofuran (50 mL), and 10% Pd/C (0.8 g, 7.4 mmol) was added. The reaction mixture was stirred in a hydrogen atmosphere at room temperature for 2 h. After the reaction was complete, the reaction mixture was filtered, and the filtrate was concentrated under reduced pressure to give the crude product. The resulting residue was purified by silica gel column chromatography with an eluent system A to give the title

compound **1d** (20 g, yield: 99%).
MS m/z (ESI): 270.1 (M+1) $^+$.

Step IV *N*-(3-fluoro-4-methoxy-8-oxo-5,6,7,8-tetralin-1-yl)acetamide **1e**

The compound **1d** (20 g, 74.4 mmol) was dissolved in trifluoroacetic acid (60 mL), the mixture was cooled to 0 °C, and then trifluoroacetic anhydride (31.24 g, 148.8 mmol) was slowly added. The reaction mixture was stirred at room temperature for 7 h. After the reaction was complete, the reaction mixture was slowly poured into water (50 mL), and extracted with ethyl acetate (50 mL×3). The organic phases were combined, washed with a saturated aqueous solution of sodium bicarbonate until neutral, then washed with saturated brine, dried, and concentrated. The resulting residue was purified by silica gel column chromatography with the eluent system B, to give the title compound **1e** (9.2 g, yield: 46%).
MS m/z (ESI): 252.1 (M+1) $^+$.

Step V (*Z*)-*N*-(3-fluoro-7-(hydroxyimino)-4-methoxy-8-oxo-5,6,7,8-tetralin-1-yl)acetamide **1f**

Potassium tert-butoxide (9.83 g, 87.5 mmol) was dissolved in tetrahydrofuran (40 mL) and tert-butanol (10 mL), and the mixture was cooled to 0 °C. Then, *N*-(3-fluoro-4-methoxy-8-oxo-5,6,7,8-tetralin-1-yl)acetamide **1e** (10 g, 39.8 mmol) was dissolved in tetrahydrofuran (10 mL), the resulting mixture was slowly added to the reaction mixture, and ten minutes later, isoamyl nitrite (7.46 g, 63.6 mmol) was added. The reaction mixture was stirred at 0 °C for 1 h. After the reaction was complete, a saturated ammonium chloride solution was added to the reaction mixture to quench the reaction. The mixture was extracted with ethyl acetate (50 mL×3). The organic phases were combined, then washed with saturated brine, dried, and concentrated. The resulting residue was purified by silica gel column chromatography with the eluent system B, to give the title compound **1f** (6 g, yield: 51%).
MS m/z (ESI): 281.1 (M+1) $^+$.

Step VI *N*-(7-amino-3-fluoro-4-methoxy-8-oxo-5,6,7,8-tetralin-1-yl)acetamide **1g**

(Z)-*N*-(3-fluoro-7-(hydroxyimino)-4-methoxy-8-oxo-5,6,7,8-tetralin-1-yl)acetamide **1f** (6 g, 21.4 mmol) was dissolved in dioxane (60 mL) and 2 *N* hydrochloric acid solution (20 mL), and 10% Pd/C (1.13 g, 10.7 mmol) was added. The reaction mixture was stirred in a hydrogen atmosphere at room temperature for 5 h. After the reaction was complete, the reaction mixture was filtered and concentrated to give the crude product **1g** (5 g), which was directly used in the next step without purification.
MS m/z (ESI): 267.1 (M+1) $^+$.

Step VII (9*H*-fluoren-9-yl)methyl(8-acetamido-6-fluoro-5-methoxy-1-oxo-1,2,3,4-tetralin-2-yl)carbamate **1h**

The compound **1g** (5 g, 18.8 mmol) obtained from the previous step was dissolved in dioxane (50 mL), and after the solution was adjusted with sodium bicarbonate to pH=7-8, 9-fluorenylmethyl-*N*-succinimidyl carbonate (6.36 g, 18.8 mmol) was slowly added. The reaction mixture was stirred at room temperature for 1 h. After the reaction was complete, the reaction mixture was slowly poured into water (40 mL), and extracted with ethyl acetate (40 mL×3). The organic phases were combined, washed with saturated brine, dried, and concentrated.
The resulting residue was purified by silica gel column chromatography with the eluent system B, to give the title compound **1h** (5.3 g, yield: 54%).
MS m/z (ESI): 489.2 (M+1) $^+$.

Step VIII (9*H*-fluoren-9-yl)methyl(8-amino-6-fluoro-5-methoxy-1-oxo-1,2,3,4-tetralin-2-yl)carbamate **1i**

The compound **1h** (5 g, 10.25 mmol) was dissolved in dioxane (50 mL) and 12 *N* hydrochloric acid (10 mL). The reaction mixture was stirred at 60 °C for 2 h. After the reaction was complete, the reaction mixture was slowly poured into water (50 mL), and extracted with ethyl acetate (40 mL×3). The organic phases were combined, washed with saturated brine, dried, and concentrated. The resulting residue was purified by silica gel column chromatography with the eluent system B, to give the title compound **1i** (3.75 g, yield: 78%).
MS m/z (ESI): 447.2 (M+1) $^+$.

Step IX (9*H*-fluoren-9-yl)methyl((9S)-9-ethyl-5-fluoro-9-hydroxy-4-methoxy-10,13-dioxo-2,3,9,10,13,15-hexahydro-1*H*,12*H*-benzo[*de*]pyrano[3',4',6,7]indolizino[1,2-*b*]quinolin-1-yl)carbamate **1k**

The compound **1i** (3 g, 6.6 mmol) was dissolved in toluene (30 mL), and (*S*)-4-ethyl-4-hydroxy-7,8-dihydro-1*H*-pyranindolizine-3,6,10(4*H*)-trione **1j** (2.61 g, 9.9 mmol) and p-toluenesulfonic acid (2.52 g, 13.2 mmol) were added. The reaction mixture was stirred at 110 °C for 5 h. After the reaction was complete, the mixture was extracted with ethyl acetate (30 mL×3). The organic phases were combined, washed with saturated brine, dried, and concentrated. The resulting residue was purified by silica gel column chromatography with the eluent system B, to give the title compound **1k** (1.8 g, yield: 36%).

MS m/z (ESI): 674.2 $(M+1)^+$.

Step X (9*H*-fluoren-9-yl)methyl((9S)-9-ethyl-5-fluoro-4,9-dihydroxy-10,13-dioxo-2,3,9,10,13,15-hexahydro-1*H*,12*H*-benzo[*de*]pyrano[3',4',6,7]indolizino[1,2-*b*]quinolin-1-yl)carbamate **1l**

The compound **1k** (1.8 g, 2.7 mmol) was dissolved in 40% hydrobromic acid (40 mL). The reaction mixture was stirred at 100 °C for 2 h. After the reaction was complete, the reaction mixture was extracted with ethyl acetate (50 mL×3). The organic phases were combined, washed with saturated brine, dried, and concentrated. The resulting residue was purified by silica gel column chromatography with the eluent system B, to give the title compound **1l** (1.08 g, yield: 60%).

MS m/z (ESI): 660.2 $(M+1)^+$.

Step XI (9*S*)-1-amino-9-ethyl-5-fluoro-4,9-dihydroxy-1,2,3,9,12,15-hexahydro-10*H*,13*H*-benzo[*de*]pyrano[3',4',6,7]indolizino[1,2-*b*]quinoline-10,13-dione **1m**

The compound **1l** (500 mg, 0.7 mmol) was dissolved in *N,N*-dimethylformamide (5 mL), and diethylamine (166 mg, 2.3 mmol) was added. The reaction mixture was stirred at room temperature for 1 h. After the reaction was complete, the reaction mixture was spin-dried using an oil pump to remove diethylamine, thus giving the crude product **1m**, which was slurried with ethyl acetate to give a solid. The solid was directly used in the next reaction step.

MS m/z (ESI): 438.1 $(M+1)^+$.

Step XII (1*S*,9*S*)-9-ethyl-5-fluoro-9-hydroxy-1-((*R*)-3-hydroxybutyrylamino)-10,13-dioxo-2,3,9,10,13,15-hexahydro-1*H*,12*H*-benzo[*de*]pyrano[3',4',6,7]indolizino[1,2-*b*]quinolin-4-yl-(*R*)-3-hydroxybutyrate **1n**

The compound **1m** (100 mg, 0.2 mmol) was dissolved in *N,N*-dimethylformamide (3 mL), and (*R*)-3-hydroxybutyric acid (36 mg, 0.34 mmol), 2-(7-azabenzotriazole)-*N,N,N',N'*-tramethyluronium hexafluorophosphate (174 mg, 0.46 mmol), and *N,N*-diisopropylethylamine (88 mg, 0.68 mmol) were added. The reaction mixture was stirred at room temperature for 1 h. After the reaction was complete, the reaction mixture was extracted with ethyl acetate (50 mL×3). The organic phases were combined, washed with saturated brine, dried, and concentrated. The resulting residue was purified by silica gel column chromatography with the eluent system B, to give the title compound **1n** (90 mg, yield: 64%).

MS m/z (ESI): 610.2 $(M+1)^+$.

Step XIII

(*R*)-*N*-((1*S*,9*S*)-9-ethyl-5-fluoro-4,9-dihydroxy-10,13-dioxo-2,3,9,10,13,15-hexahydro-1*H*,12*H*-benzo[*de*]pyrano [3',4',6,7]indolizino[1,2-*b*]quinolin-1-yl)-3-hydroxybutanamide

(*R*)-*N*-((1*R*,9*S*)-9-ethyl-5-fluoro-4,9-dihydroxy-10,13-dioxo-2,3,9,10,13,15-hexahydro-1*H*,12*H*-benzo[*de*]pyrano [3',4',6,7]indolizino[1,2-*b*]quinolin-1-yl)-3-hydroxybutanamide

The compound **1n** (90 mg, 0.15 mmol) was dissolved in methanol (5 mL), and then an aqueous solution of lithium hydroxide (2 mL, 1 M) was added at room temperature. The mixture was stirred at room temperature for reaction for 15 min. After the reaction was complete, methanol was spun away, and the remaining aqueous phase was lyophilized to give the crude product, which was purified by preparative high performance liquid chromatography (Waters MS-triggered Prep-LC with SQD2 detector, chromatographic column: Xbridge C18 150*19 mm, 5 μm; mobile phase 1: water (containing 0.1% TFA); mobile phase 2: acetonitrile; 15-min gradient, gradient proportion: acetonitrile phase 23%-33%, flow rate: 20 mL/min) to give compound **1-1** (35 mg, yield: 30%) and compound **1-2** (28 mg, yield: 24%).

Monoconfigurational compound **1-1** (short retention time):

MS m/z (ESI): 524.2 $(M+1)^+$.

$^1$H NMR (400 MHz, DMSO-$d_6$) δ 10.42 (s, 1H), 8.39 (d, 1H), 7.84 (d, 1H), 7.26 (s, 1H), 6.50 (s, 1H), 5.57-5.49 (m, 1H), 5.41 (s, 2H), 5.27-5.11 (m, 2H), 4.04 (dd, 1H), 3.18-3.04 (m, 2H), 2.36-2.17 (m, 2H), 2.15-1.96 (m, 2H), 1.92-1.77 (m, 2H), 1.08 (d, 3H), 0.93-0.81 (m, 3H).

Monoconfigurational compound **1-2** (long retention time):

MS m/z (ESI): 524.2 (M+1) $^+$.

$^1$H NMR (400 MHz, DMSO-$d_6$) δ 10.42 (s, 1H), 8.42 (d, 1H), 7.84 (d, 1H), 7.26 (s, 1H), 5.59-5.51 (m, 1H), 5.42 (s, 2H), 5.29-5.14 (m, 2H), 4.04 (dd, 1H), 3.17 (dd, 1H), 3.10-2.92 (m, 1H), 2.28 (dd, 1H), 2.18 (dd, 1H), 2.13-1.95 (m, 2H), 1.93-1.78 (m, 2H), 1.08 (d, 3H), 0.91-0.82 (m, 3H).

**Example 2**

(*R*)-*N*-((1*S*,9*S*)-9-ethyl-4,9-dihydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-9-ethyl-4,9-dihydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

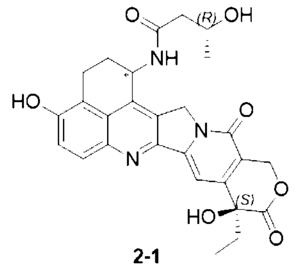

**2-1**
**2-2**

*-C represents that the chirality of the atom here is R or S

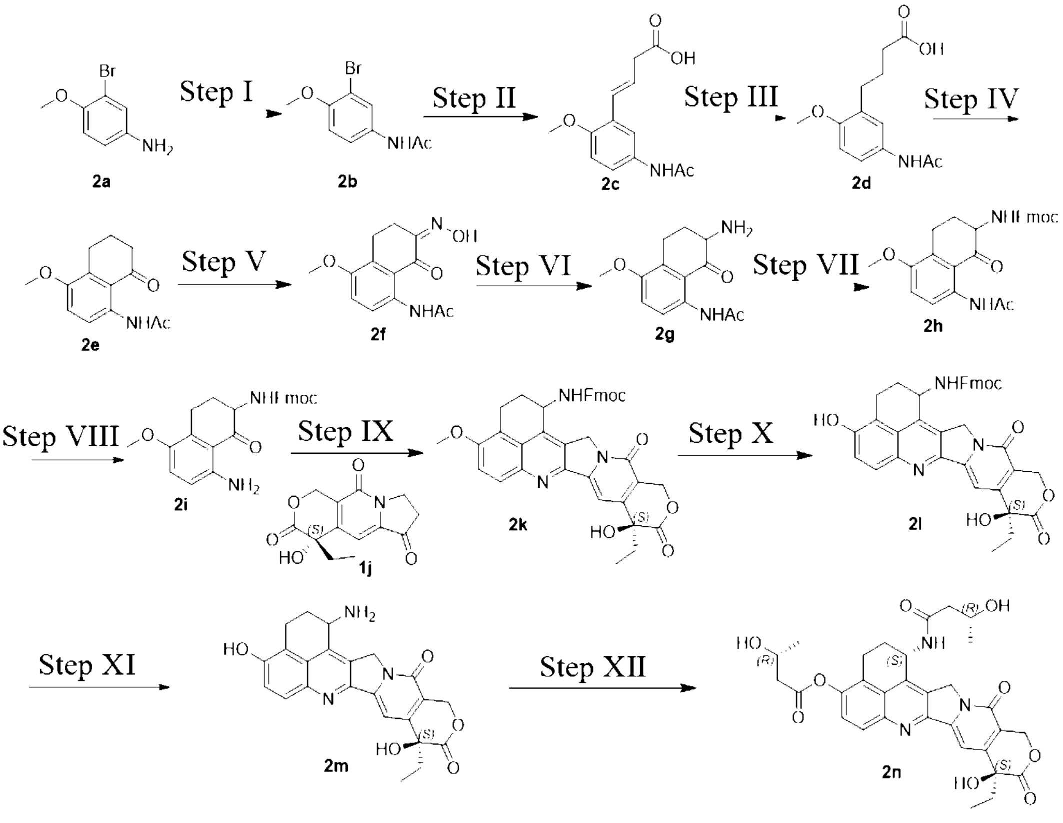


2a 2b 2c 2d 2e 2f 2g 2h 2i 1j 2k 2l 2m 2n

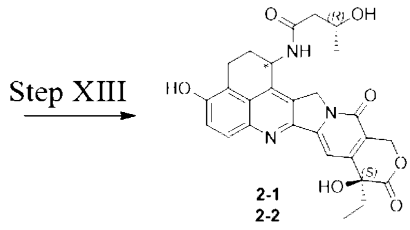


2-1
2-2

*-C represents that the chirality of the atom here is R or S

In accordance with the synthetic route in Example 1, the raw material from the step I was replaced with 3-bromo-4-methoxyaniline 2a (25 g, 0.12 mol), and the purification was performed by preparative high performance liquid chromatography (Waters MS-triggered Prep-LC with SQD2 detector, chromatographic column: Xbridge C18 150*19 mm, 5 μm; mobile phase 1: water (0.1% TFA); mobile phase 2: acetonitrile; 15-min gradient, gradient proportion: acetonitrile phase 23%-33%, flow rate: 20 mL/min) to give the captioned products **2-1** (19 mg, yield: 28%) and **2-2** (30 mg, yield: 44%).

Monoconfigurational compound 2-1 (short retention time):

MS m/z (ESI): 506 (M+1) $^+$.

$^1$H NMR (400 MHz, DMSO-$d_6$) $\delta$ 10.21 (s, 1H), 8.39-8.37 (m, 1H), 7.92-7.90 (m, 1H), 7.51-7.50 (m, 1H), 7.24 (s, 1H), 6.49 (s, 1H), 5.57-5.48 (m, 1H), 5.41(s, 2H), 5.27-5.11 (m, 2H), 4.65 (s, 1H), 4.09-3.98 (m, 1H), 3.09-2.96 (m, 2H), 2.25-2.23 (m, 2H), 2.05-2.00 (m, 2H), 1.95-1.77 (m, 2H), 1.23 (s, 2H),1.08-1.07 (m, 3H), 0.86-0.85(m, 3H).

Monoconfigurational compound **2-2** (long retention time):

MS m/z (ESI): 506 (M+1) $^+$.

$^1$H NMR (400 MHz, DMSO-$d_6$) $\delta$ 10.22 (s, 1H), 8.42 - 8.40 (d, 1H), 7.93-7.90 (d, 1H), 7.51-7.50 (m, 1H), 7.25 (s, 1H), 5.58-5.46 (m,1H), 5.42 (s, 2H), 5.21-5.20 (m, 2H), 4.04-4.01 (m, 1H), 2.96-2.94 (m, 2H), 2.28 (s, 2H), 2.10-1.97 (m, 2H), 1.86 (s, 2H), 1.23 (s, 2H), 1.08-1.06 (m, 3H), 0.87-0.85 (m, 3H).

**Example 3**

(*R*)-*N*-((1*S*,9*S*)-4-bromo-9-ethyl-5-fluoro-9-hydroxy-10,13-dioxo-2,3,9,10,13,15-hexahydro-1*H*,12*H* benzo[*de*]pyrano [3',4',6,7]indolizino[1,2-*b*]quinolin-1-yl)-3-hydroxybutanamide

(*R*)-*N*-((1R,9*S*)-4-bromo-9-ethyl-5-fluoro-9-hydroxy-10,13-dioxo-2,3,9,10,13,15-hexahydro-1*H*,12*H*-benzo[*de*]pyrano [3',4',6,7]indolizino[1,2-*b*]quinolin-1-yl)-3-hydroxybutanamide

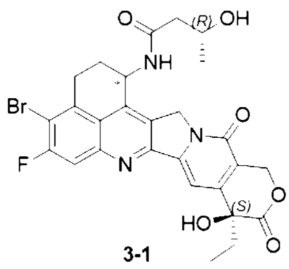

3-1
3-2

*-C represents that the chirality of the atom is R or S

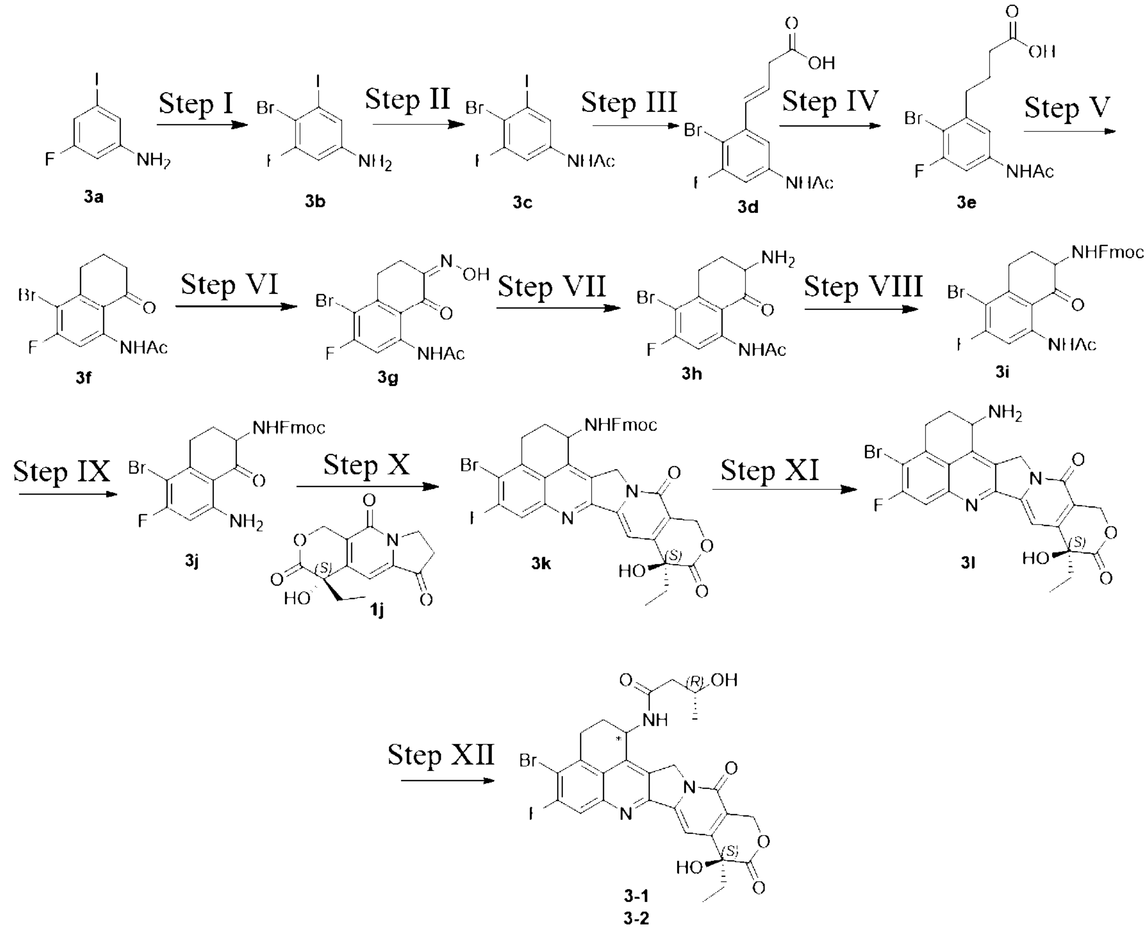


*-C represents that the chirality of the atom is R or S

Step I 4-bromo-3-fluoro-5-iodoaniline 3b

3-fluoro-5-iodoaniline **3a** (50 g, 210.95 mmol) was dissolved in DMF (250 mL), and NBS (41.30 g, 232.04 mmol) was slowly added under an ice bath condition. The mixture was stirred for reaction at room temperature for 16 h. After the reaction was complete, water was added, and the system was extracted with dichloromethane. The organic phase was washed with saturated brine, collected, dried over anhydrous sodium sulfate, and spin-dried to remove the solvent, thus giving the crude product **3b** (66 g).

MS m/z (ESI): 315.9, 317.9 (M+1) $^+$.

$^1$H NMR (400 MHz, DMSO-$d_6$) $\delta$ 7.01 (d, 1H), 6.47 (dd, 1H), 5.74 (s, 2H).

Step II *N*-(4-bromo-3-fluoro-5-iodophenyl)acetamide **3c**

The compound **3b** (66 g, 208.92 mmol) was dissolved in dichloromethane (660 mL), and triethylamine (42.28 g, 417.84 mmol) was added. After the system was cooled to 0 °C, acetyl chloride (19.68 g, 250.70 mmol) was slowly added dropwise. After the dropwise addition was complete, the system was stirred at room temperature for 4 h. After the reaction was complete, the mixture was spin-dried to remove the solvent, thus giving the crude product, which was redissolved in ethyl acetate. Then, the system was adjusted with dilute hydrochloric acid to a pH of 2-3, then extracted with ethyl acetate, and washed with saturated brine. The organic phase was collected, dried over anhydrous sodium sulfate, and spin-dried to remove the solvent, thus giving the crude product, which was slurried with a mixed solvent of dichloromethane/methanol (10:1) to give the title compound **3c** (60 g, yield: 80%).

MS m/z (ESI): 357.9, 359.9 (M+1) $^+$.

$^1$H NMR (400 MHz, DMSO-$d_6$) $\delta$ 10.27 (s, 1H), 7.96 (s, 1H), 7.66 (dd, 1H), 2.06 (s, 3H).

Step III (*E*)-4-(5-acetylamino-2-bromo-3-fluorophenyl)but-3-enoic acid **3d**

The compound **3c** (20 g, 55.87 mmol) was dissolved in a mixed solvent of dioxane (200 mL) and water (40 mL), and but-3-enoic acid (4.81 g, 55.87 mmol), DIPEA (14.45 g, 111.74 mmol), palladium acetate (630 mg, 2.79 mmol), and tris(o-methylphenyl)phosphorus (1.7 g, 5.59 mmol) were added. The mixture was stirred for reaction under the protection of nitrogen at 100 °C for 16 h. After the reaction was complete, water and dichloromethane were added, and the system was washed with a saturated sodium bicarbonate solution 3-5 times. The aqueous phase was collected, adjusted with hydrochloric acid to a pH of 2-3, and extracted with ethyl acetate 5-7 times. The organic phase was collected, dried over anhydrous sodium sulfate, and spin-dried to remove the solvent, thus giving the crude compound **3d** (17 g).

MS m/z (ESI): 316.0, 317.9 $(M+1)^+$.

Step IV 4-(5-acetylamino-2-bromo-3-fluorophenyl)butyric acid **3e**

The compound **3d** (8 g, 25.3 mmol) was dissolved in methanol (80 mL), and a platinum-carbon catalyst (800 mg) was added. The mixture was stirred for reaction in a hydrogen environment at room temperature for 2 h. After the reaction was complete, the mixture was filtered. The filtrate was collected, and spin-dried to remove the solvent, thus giving the crude product 3e (8 g), which was directly used in the next reaction step without purification.

MS m/z (ESI): 318.0, 320.0 $(M+1)^+$.

$^1$H NMR (400 MHz, DMSO-$d_6$) δ 12.14 (s, 1H), 10.24 (s, 1H), 7.63 (dd, 1H), 7.25 (s, 1H), 2.75-2.67 (m, 2H), 2.29 (t, 2H), 2.05 (s, 3H), 1.79 (dd, 2H).

Step V N-(4-bromo-3-fluoro-8-oxo-5,6,7,8-tetralin-1-yl)acetamide **3f**

The compound **3e** (8 g, 25.1 mmol) was dissolved in trifluoroacetic acid (80 mL), and trifluoroacetic anhydride (15.82 g, 75.3 mmol) was slowly added under an ice bath condition. The mixture was stirred for reaction at 0 °C for 4 h. After the reaction was complete, water was added in an ice bath. The system was adjusted with 15% sodium hydroxide solution to a pH of 9-10, and then extracted with dichloromethane. The organic phase was washed with saturated brine, collected, dried over anhydrous sodium sulfate, and spin-dried to remove the solvent, thus giving the compound **3f** (4.8 g, yield: 53%).

MS m/z (ESI): 329.0, 331.0 $(M+1)^+$.

Step VI (*Z*)-*N*-(4-bromo-3-fluoro-7-(hydroxyimino)-8-oxo-5,6,7,8-tetralin-1-yl)acetamide **3g**

Potassium tert-butoxide (1.62 g, 14.4 mmol) was dissolved in a mixed solvent of tetrahydrofuran (80 mL) and tert-butanol (20 mL), and a solution of the compound **3f** (2.15 g, 7.2 mmol) in tetrahydrofuran (20 mL) was slowly added in an ice bath. After the mixture was stirred for reaction at 0°C for 10 min, isoamyl nitrite (1.27 g, 10.8 mmol) was added, and the reaction was continued by stirring at 0 °C for 50 min. After the reaction was complete, the system was adjusted with dilute hydrochloric acid to a pH of 4-5, and extracted with ethyl acetate. The organic phase was washed with saturated brine, collected, dried over anhydrous sodium sulfate, and spin-dried to remove the solvent, thus giving the crude product, which was slurried with methyl tert-butyl ether to give the title compound **3g** (1.1 g, yield: 46%).

MS m/z (ESI): 329.0, 331.0 $(M+1)^+$.

$^1$H NMR (400 MHz, $CD_3OD$) δ 8.52 (d, 1H), 3.21 (dd, 2H), 3.07 (dd, 2H), 2.24 (s, 3H).

Step VII *N*-(7-amino-4-bromo-3-fluoro-8-oxo-5,6,7,8-tetralin-1-yl)acetamide **3h**

The compound **3g** (500 mg, 1.52 mmol) was dissolved in dioxane (10 mL), and 1 mL of 1M hydrochloric acid and a platinum-carbon catalyst (100 mg) were added. The mixture was stirred for reaction in a hydrogen environment at room temperature for 4 h. After the reaction was complete, the mixture was filtered. The filtrate was collected and concentrated to give the crude product **3h** (500 mg), which was directly used in the next reaction step.

MS m/z (ESI): 315.1, 317.1 $(M+1)^+$.

Step VIII (9*H*-fluoren-9-yl)methyl(8-acetylamino-5-bromo-6-fluoro-1-oxo-1,2,3,4-tetralin-2-yl)carbamate **3i**

The compound **3h** (500 mg) was dissolved in dioxane (10 mL), the filtrate from the step VII was adjusted with a saturated sodium carbonate solution to a pH of 8-9, and then fluorenylmethoxycarbonylchloride (432 mg, 1.67 mmol) was added. The mixture was stirred for reaction at room temperature for 1 h. After the reaction was complete, the system was extracted with ethyl acetate. The organic phase was washed with saturated brine, collected, dried over anhydrous sodium

sulfate, and spin-dried to remove the solvent, thus giving the crude product. The resulting residue was purified by silica gel column chromatography with the eluent system B, to give the title compound **3i** (300 mg, yield: 37%).

MS m/z (ESI): 537.0, 539.0 (M+1) +.

Step IX (9*H*-fluoren-9-yl)methyl(8-amino-5-bromo-6-fluoro-1-oxo-1,2,3,4-tetralin-2-yl)carbamate **3j**

The compound **3i** (700 mg, 1.30 mmol) was dissolved in methanol (10 mL), and concentrated hydrochloric acid (12 mol/L, 2 mL) was added. The mixture was stirred for reaction at 60 °C for 1 h. After the reaction was complete, the resulting residue was purified by silica gel column chromatography with the eluent system B, to give the title compound **3j** (500 mg, yield: 77%).

Step X ((9*H*-fluoren-9-yl)methyl((9S)-4-bromo-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)carbamate **3k**

The compound **3j** (200 mg, 0.40 mmol) was dissolved in toluene (5 mL), and (*S*)-4-ethyl-4-hydroxy-7,8-dihydro-1*H*-pyrano[3,4-f]indolizine-3,6,10(4H)-trione **1j** (117 mg, 0.44 mmol) and p-toluenesulfonic acid monohydrate (77 mg, 0.40 mmol) were added. The mixture was stirred for reaction at 120 °C for 2 h. After the reaction was complete, the mixture was spin-dried to remove the solvent, thus giving the crude product. The resulting residue was purified by silica gel column chromatography with the eluent system B to give the title compound **3k** (250 mg, yield: 86%).

MS m/z (ESI): 722.0, 724.0 (M+1) +.

$^1H$ NMR (400 MHz, $CDCl_3$) δ 8.19-7.31 (m, 9H), 5.68 (dd, 1H), 5.33-5.18 (m, 2H), 4.66 (s, 2H), 4.34 (d, 1H), 4.21-4.04 (m, 2H), 3.26 (s, 1H), 3.00-2.94 (m, 1H), 2.04 (s, 2H), 1.81 (s, 2H), 1.70-1.50 (m, 2H), 1.27 (dd, 3H).

Step XI (9*S*)-1-amino-4-bromo-9-ethyl-5-fluoro-9-hydroxy-1,2,3,9,12,15-hexahydro-10*H*,13*H*-benzo[*de*]pyrano [3',4',6,7]indolizino[1,2-*b*]quinoline-10,13-dione **3l**

The compound **3k** (200 mg, 0.28 mmol) was dissolved in DMF (1 mL), and diethylamine (0.1 mL) was added. The mixture was stirred for reaction at room temperature for 30 min. After the reaction was complete, the resulting residue was purified by silica gel column chromatography with the eluent system A, to give the title compound **3l** (100 mg, yield: 72%).

MS m/z (ESI): 500.0, 502.1 (M+1) +.

Step XII

(*R*)-*N*-((1*S*,9*S*)-4-bromo-9-ethyl-5-fluoro-9-hydroxy-10,13-dioxo-2,3,9,10,13,15-hexahydro-1H,12*H*-benzo[*de*]pyrano [3',4',6,7]indolizino[1,2-*b*]quinolin-1-yl)-3-hydroxybutanamide

(*R*)-*N*-((1*R*,9*S*)-4-bromo-9-ethyl-5-fluoro-9-hydroxy-10,13-dioxo-2,3,9,10,13,15-hexahydro-1*H*,12*H*-benzo[*de*]pyrano [3',4',6,7]indolizino[1,2-*b*]quinolin-1-yl)-3-hydroxybutanamide

The compound **3l** (100 mg, 0.20 mmoL) was dissolved in DMF (2 mL), and (*R*)-3-hydroxybutyric acid (25 mg, 0.24 mmol), 2-(7-azobenzotriazole)-*N,N,N',N'*-tramethyluronium hexafluorophosphate (114 mg, 0.30 mmol), and *N,N*-diisopropylethylamine (52 mg, 0.40 mmol) were added. The mixture was stirred for reaction at room temperature for 1 h. After the reaction was complete, the purification was performed by preparative high performance liquid chromatography (Waters MS-triggered Prep-LC with QDA detector, chromatographic column: WELCH Xtimate C18 21.2*250mm, 10 μm; mobile phase 1: water (0.1% FA); mobile phase 2: acetonitrile; 15-min gradient, gradient proportion: acetonitrile phase 38%-48%, flow rate: 25 mL/min) to give compound **3-1** (1.62 mg, yield: 6%) and compound **3-2** (1.32 mg, yield: 7.4%).

Monoconfigurational compound **3-1** (short retention time):

MS m/z (ESI): 586.0, 588.0 (M+1) +.

$^1H$ NMR (400 MHz, $CD_3OD$) δ 8.10 (d, 1H), 7.90 (s, 1H), 5.96-5.88 (m, 1H), 5.83 (d, 1H), 5.71-5.48 (m, 4H), 4.52-4.46 (m, 1H), 2.71-2.65 (m, 2H), 2.59-2.52 (m, 2H), 2.23-2.18 (m, 3H), 1.49 (d, 3H), 1.25 (t, 3H).

Monoconfigurational compound **3-2** (long retention time):

MS m/z (ESI): 586.0, 588.0 $(M+1)^+$.

$^1H$ NMR (400 MHz, $CD_3OD$) δ 7.79 (d, 1H), 7.59 (s, 1H), 5.67-5.59 (m, 1H), 5.52 (d, 1H), 5.41-5.33 (m, 2H), 5.32-5.23 (m, 2H), 4.24-4.18 (m, 1H), 2.34 (d, 2H), 2.28-2.21 (m, 2H), 1.93-1.86 (m, 3H), 1.17 (d, 3H), 0.94 (t, 3H).

**Example 4**

(*R*)-*N*-((1*S*,9*S*)-9-ethyl-5-fluoro-9-hydroxy-10,13-dioxo-2,3,9,10,13,15-hexahydro-1*H*,12*H*-benzo[*de*]pyrano[3',4',6,7] indolizino[1,2-*b*]quinolin-1-yl)-3-hydroxybutanamide

(*R*)-*N*-((1*R*,9*S*)-9-ethyl-5-fluoro-9-hydroxy-10,13-dioxo-2,3,9,10,13,15-hexahydro-1*H*,12*H*-benzo[*de*]pyrano[3',4',6,7] indolizino[1,2-*b*]quinolin-1-yl)-3-hydroxybutanamide

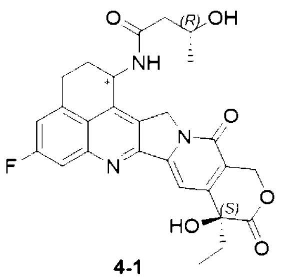

**4-1**
**4-2**

*-C represents that the chirality of the atom is R or S

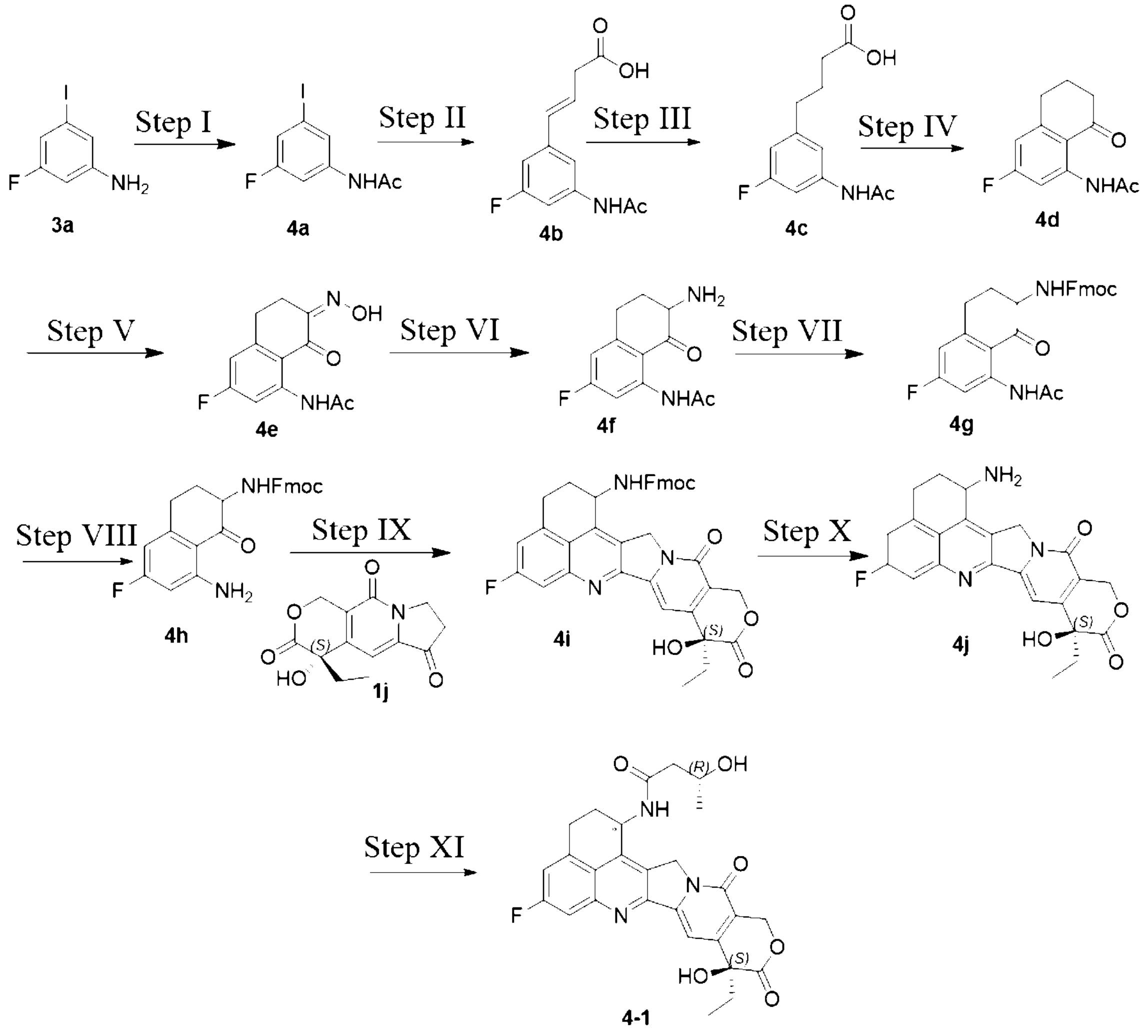


**3a** **4a** **4b** **4c** **4d** **4e** **4f** **4g** **4h** **1j** **4i** **4j**

**4-1**
**4-2**

*-C represents that the chirality of the atom is R or S

In accordance with the synthetic route in Example 3, 3-fluoro-5-iodoaniline (25 g, 105.5 mmol) was used as the starting material, and the purification was performed by preparative high performance liquid chromatography (Waters MS-triggered Prep-LC with QDA detector, chromatographic column: WELCH Xtimate C18 21.2*250 mm, 10 μm; mobile phase 1: water (0.1% FA); mobile phase 2: acetonitrile; 15-min gradient, gradient proportion: acetonitrile phase 38%-48%, flow rate: 25 mL/min) to give the captioned products **4-1** (2.5 mg, yield: 12%) and **4-2** (3.1 mg, yield: 15%).

Monoconfigurational compound **4-1** (short retention time):

MS m/z (ESI): 508.1 (M+1) $^+$.
$^1$H NMR (400 MHz, $CD_3OD$) δ 7.65 (d, 2H), 7.37 (d, 1H), 5.66 (s, 1H), 5.56 (d, 1H), 5.40-5.20 (m, 4H), 4.28-4.21 (m, 1H), 2.47-2.39 (m, 2H), 2.27 (s, 2H), 1.98-1.90 (m, 2H), 1.24 (d, 4H), 0.99 (t, 3H).

Monoconfigurational compound **4-2** (long retention time):

MS m/z (ESI): 508.1 (M+1) $^+$.
$^1$H NMR (400 MHz, $CD_3OD$) δ 7.57 (d, 2H), 7.30 (d, 1H), 5.60 (d, 1H), 5.48 (d, 1H), 5.36-5.15 (m, 5H), 4.22-4.15 (m, 1H), 2.36-2.30 (m, 2H), 2.18 (t, 3H), 1.90-1.83 (m, 2H), 1.15 (d, 3H), 0.91 (t, 3H).

**Example 5**

(*R*)-*N*-((1*S*,9*S*)-4-(difluoromethoxy)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-4-(difluoromethoxy)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

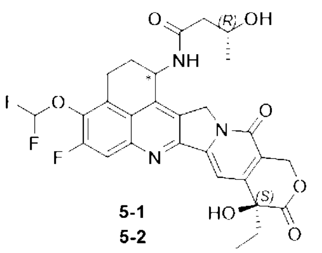

**5-1**
**5-2**

*-C represents that the chirality of the atom is R or S

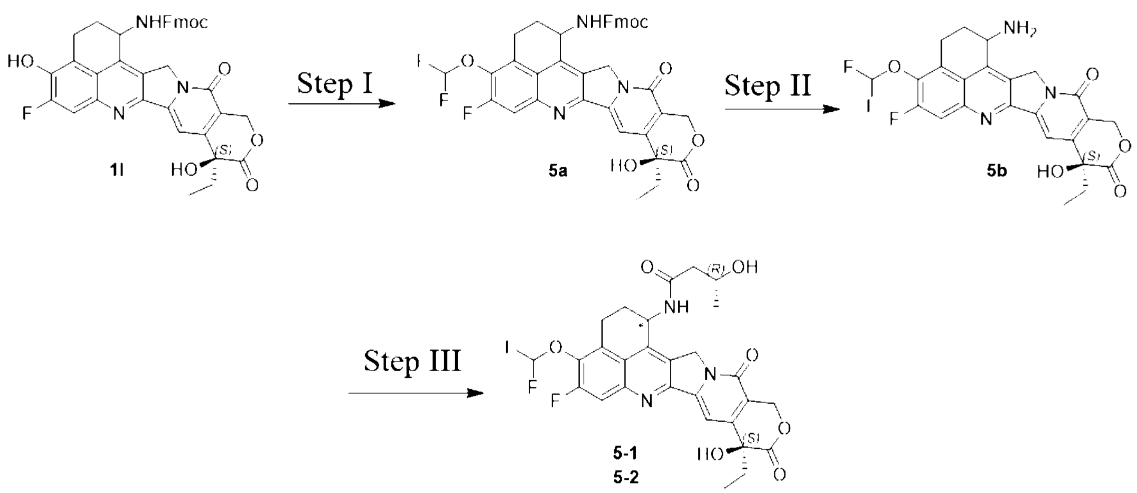


**1l** **5a** **5b**

**5-1**
**5-2**

*-C represents that the chirality of the atom is R or S

Step I (9*H*-fluoren-9-yl)methyl((9*S*)-4-(difluoromethoxy)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)carbamate **5a**

The compound **1l** (200 mg, 0.30 mmol) was dissolved in DMF (5 mL), and cesium carbonate (198 mg, 0.61 mmol) and diethyl (bromodifluoromethyl)phosphonate (121 mg, 0.45 mmol) were added in an ice bath. The mixture was stirred for reaction at 0 °C for 1 h. After the reaction was complete, water was added, and the system was extracted with ethyl acetate. The organic phase was washed with saturated brine, collected, dried over anhydrous sodium sulfate, and spin-dried to remove the solvent, thus giving the crude product, which was separated and purified by column chromatography to give the title compound **5a** (200 mg, yield: 93%).
MS m/z (ESI): 711.2 $(M+1)^+$.

Step II (9*S*)-1-amino-4-(difluoromethoxy)-9-ethyl-5-fluoro-9-hydroxy-1,2,3,9,12,15-hexahydro-10*H*,13*H*-benzo[*de*]pyrano[3',4',6,7]indolizino[1,2-*b*]quinoline-10,13-dione **5b**

The compound **5a** (140 mg, 0.20 mmol) was dissolved in *N,N*-dimethylformamide (1 mL), and diethylamine (0.1 mL) was added. The mixture was stirred for reaction at room temperature for 1 h. After the reaction was complete, the reaction mixture was spin-dried under vacuum using an oil pump to remove the solvent, thus giving the crude title compound **5b** (90 mg), which was directly used in the next reaction step without purification.
MS m/z (ESI): 488.1 $(M+1)^+$.

Step III

(*R*)-*N*-((1*S*,9*S*)-4-(difluoromethoxy)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-4-(difluoromethoxy)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

The crude product **5b** (90 mg, 0.18 mmol) was dissolved in *N,N*-dimethylformamide (1 mL), and (*R*)-3-hydroxybutyric acid (23 mg, 0.22 mmol), 2-(7-azobenzotriazole)-*N,N,N',N'*-tramethyluronium hexafluorophosphate (105 mg, 0.28 mmol), and N,N-diisopropylethylamine (48 mg, 0.37 mmol) were added. The mixture was stirred for reaction at room temperature for 1 h. After the reaction was complete, the mixture was spin-dried to remove the solvent, thus giving the crude product, which was purified by preparative high performance liquid chromatography (GILSON Prep LC with UV detector, chromatographic column: Xtimate C18 250*30 mm, 10 μm; mobile phase 1: water (0.1% FA); mobile phase 2: acetonitrile; 15-min gradient, gradient proportion: acetonitrile phase 37%-47%, flow rate: 50 mL/min) to give the title compound **5-1** (8.6 mg, yield: 16%) and the title compound **5-2** (7.8 mg, yield: 14.7%).

Monoconfigurational compound **5-1** (short retention time):

MS m/z (ESI): 574.1 $(M+1)^+$.
$^1$H NMR (400 MHz, DMSO-$d_6$) δ 8.49 (d, 2H), 8.06 (*d,* 1H), 7.34 (s, 1H),7.31 (t, 1H) 6.54 (s, 1H), 5.63-5.54 (m, 1H), 5.43 (s, 2H), 5.32-5.16 (m, 2H), 4.68 (d, 1H), 4.05 (s, 1H), 3.23 (d, 1H), 2.36-2.19 (m, 2H), 2.19-2.05 (m, 2H), 1.93-1.78 (m, 2H), 1.10 (d, 3H), 0.88 (t, 3H).

Monoconfigurational compound **5-2** (long retention time):

MS m/z (ESI): 574.1 $(M+1)^+$.
$^1$H NMR (400 MHz, DMSO-$d_6$) δ 8.52 (d, 2H), 8.10-8.04 (m, 1H), 7.35 (s, 1H), 7.32 (t, 1 H) 6.55 (s, 1H), 5.66-5.58 (m, 1H), 5.43 (d, 2H), 5.26 (t, 2H), 4.66 (d, 1H), 4.14-4.01 (m, 1H), 3.25 (s, 1H), 2.34-2.20 (m, 2H), 2.16-2.07 (m, 2H), 1.92-1.80 (m, 2H), 1.10 (d, 3H), 0.89 (t, 3H).

**Example 6**

(*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4',6,7]indolizino[1,2-*b*]quinolin-1-yl)-3-hydroxybutanamide

(*R*)-*N*-((1*R*,10*S*)-10-ethyl-6-fluoro-10-hydroxy-5-methyl-11,14-dioxo-1,2,3,4,10,11,14,16-octahydro-13*H*-cyclohepta[3',4',6,7]indolizino[1,2-*b*]quinolin-1-yl)-3-hydroxybutanamide

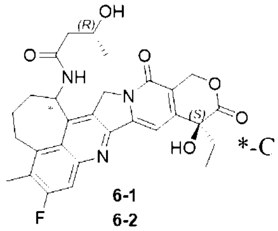

6-1
6-2

represents that the chirality of the atom is R or S

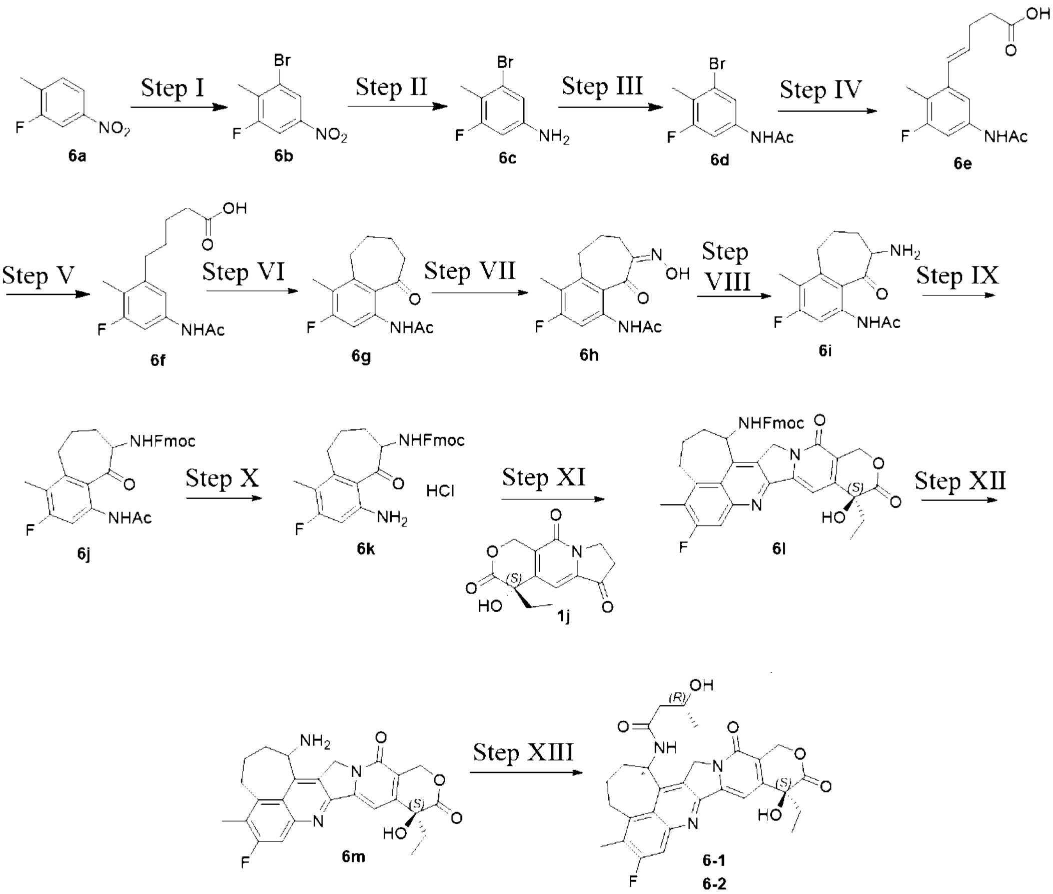


6a 6b 6c 6d 6e 6f 6g 6h 6i 6j 6k 1j 6l 6m 6-1 6-2

*-C represents that the chirality of the atom is R or S

Step I 1-bromo-3-fluoro-2-methyl-5-nitrobenzene **6b**

2-fluoro-1-methyl-4-nitrobenzene **6a** (20.0 g, 0.13 mol) was dissolved in n-heptane (50 mL), concentrated sulfuric acid (50 mL) was added, the mixture was heated to 60 °C, *N*-bromosuccinimide (35.6 g, 0.20 mol) was added batchwise at this temperature, and the mixture was kept for reaction at 60 °C for 2 h. The reaction mixture cooled to room temperature was added dropwise to ice water, and extracted with toluene. The organic phases were combined, successively washed with a sodium sulfite solution, water, and saturated brine, dried over anhydrous sodium sulfate, and then concentrated under reduced pressure, to give the title compound **6b** (30.0 g), which was directly used in the next reaction step without purification.

MS m/z (ESI): 233.9 $(M+1)^+$.

$^1$H NMR (400 MHz, CDCl3) $\delta$ 8.29-8.23 (m, 1H), 7.88 (dd, 1H), 2.44 (d, 3H).

Step II 3-bromo-5-fluoro-4-methylaniline **6c**

The compound **6b** (30.0 g, 0.13 mol) was dissolved in methanol (200 mL), platinum-carbon (3.0 g, 5% content) was added, and the mixture was, after hydrogen replacement, kept for reaction in a hydrogen atmosphere at room temperature for 16 h. After the reaction was complete, the reaction mixture was filtered, and the filtrate was concentrated to give the title compound **6c** (20.0 g, yellow oil), which was directly used in the next reaction step without purification.
MS m/z (ESI): 204.0 $(M+1)^+$.

Step III N-(3-bromo-5-fluoro-4-methylphenyl)acetamide **6d**

3-bromo-5-fluoro-4-methylaniline **6c** (20.0 g, 0.10 mol) was dissolved in dichloromethane (100 mL) under an ice bath condition, triethylamine (20.2 g, 0.20 mol) and acetyl chloride (11.8 g, 0.15 mol) were added, and the mixture was kept for reaction under an ice bath condition for 3 h. After the reaction was complete, water was added to the reaction mixture to quench the reaction, and the mixture was extracted with ethyl acetate. The organic phases were combined, dried over anhydrous sodium sulfate, and concentrated under reduced pressure to give the crude product, which was slurried with a mixed solvent of dichloromethane and petroleum ether (V/V=10:1) to give the title compound **6d** (10.0 g, yield: 32%).
MS m/z (ESI): 246.0 $(M+1)^+$.

Step IV (E)-5-(5-acetylamino-3-fluoro-2-methylphenyl)pent-4-enoic acid **6e**

*N*-(3-bromo-5-fluoro-4-methylphenyl)acetamide **6d** (10.0 g, 40.8 mmol) was dissolved in dioxane (40 mL) and water (10 mL), to which pent-4-enoic acid (6.1 g, 61.20 mmol), palladium acetate (0.7 g, 4.10 mmol), tris(o-methylphenyl) phosphorus (2.5 g, 8.20 mmol), and *N,N*-diisopropylethylamine (15.9 g, 122.01 mmol) were added. The reaction mixture was stirred for reaction at 100 °C for 16 h. After the reaction was complete, water and dichloromethane were added, and the system was washed with a saturated sodium bicarbonate solution 3 times. The aqueous phase was collected, adjusted with hydrochloric acid to a pH of 2-3, and extracted with ethyl acetate 3 times. The organic phase was collected, dried over anhydrous sodium sulfate, and spin-dried to remove the solvent, thus giving the title compound **6e** (10 g), which was directly used in the next reaction step without purification.
MS m/z (ESI): 266.1 $(M+1)^+$,
**[0176]** The synthetic route in Example 3 was used as the subsequent synthetic route, except that the intermediate **3d** in Example 3 was replaced with the intermediate **6e** (10 g, 0.04 mol). The final product was purified by preparative high performance liquid chromatography (Waters MS-triggered Prep-LC with SQD2 detector, chromatographic column: Xbridge C18 150*19 mm, 5 μm; mobile phase 1: water (0.1% TFA); mobile phase 2: acetonitrile; 15-min gradient, gradient proportion: acetonitrile phase 37%-47%, flow rate: 20 mL/min) to give the captioned products **6-1** (12.6 mg, yield: 14%) and **6-2** (17.2 mg, yield: 19%).

Monoconfigurational compound **6-1** (short retention time):

MS m/z (ESI): 536 $(M+1)^+$.
$^1$H NMR (400 MHz, DMSO-$d_6$) δ 8.60-8.55 (m, 1H), 7.73-7.72 (m, 1H), 7.28 (s, 1H), 6.51 (s, 1H), 5.54-5.53 (m, 1H), 5.42 (s, 2H), 5.32-5.26 (m, 1H), 5.24-5.22 (m, 1H), 4.65 (s, 1H), 4.01 (s, 1H), 3.22-3.18 (m, 2H), 2.42-2.40 (m, 3H), 2.28-2.24 (m, 4H), 2.10-1.67 (m, 4H), 1.06 (d, 3H), 0.85-0.82 (m, 3H).

Monoconfigurational compound **6-2** (long retention time):

MS m/z (ESI): 536 $(M+1)^+$.
$^1$H NMR (400 MHz, DMSO-$d_6$) δ 8.67-8.66 (m, 1H), 7.73-7.71 (m, 1H), 7.28 (s, 1H), 6.49 (s, 1H), 5.53-5.52 (m, 1H), 5.41-5.40 (m, 3H), 5.35-5.33 (m, 1H), 4.67-4.65 (m, 1H), 4.03-3.96 (m, 1H), 3.22-3.18 (m, 2H), 2.42 (s, 3H), 2.40-2.15 (m, 4H), 2.11-1.52 (m, 4H), 1.08-1.07 (m, 3H), 0.87-0.85 (m, 3H).

**Example 7**

(*R*)-*N*-((1*S*,9*S*)-9-ethyl-5-fluoro-9-hydroxy-10,13-dioxo-4-ethenyl-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-9-ethyl-5-fluoro-9-hydroxy-10,13-dioxo-4-ethenyl-2,3,9,10,13,15-hexahydro-1*H*,12*H*-benzo[*de*]pyrano[3',4',6,7]indolizino[1,2-*b*]quinolin-1-yl)-3-hydroxybutanamide

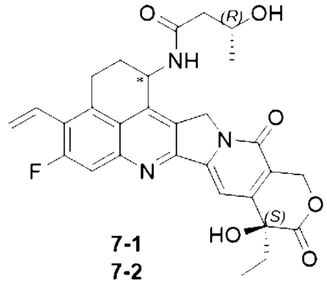

7-1
7-2

*-C represents that the chirality of the atom is R or S

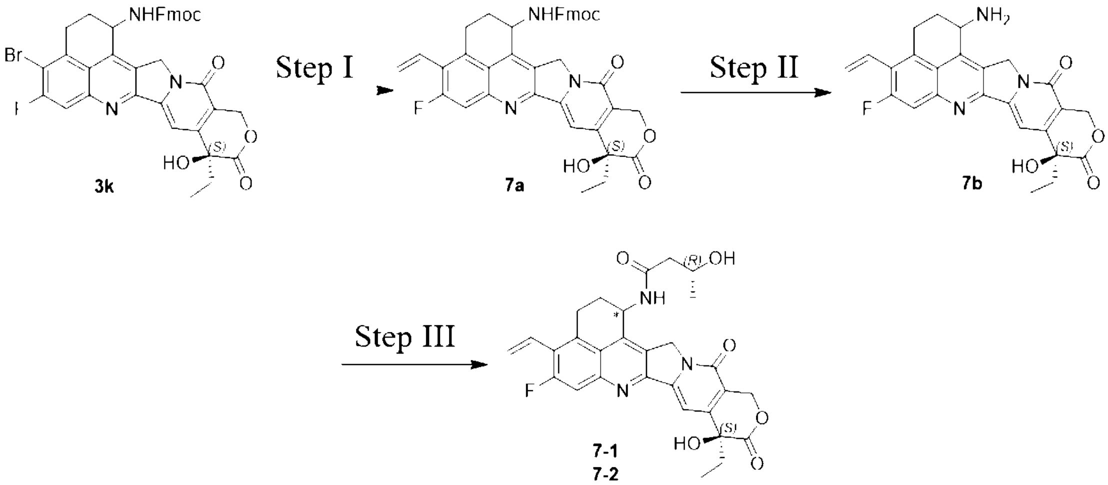


3k 7a 7b 7-1 7-2

*-C represents that the chirality of the atom is R or S

Step I (9*H*-fluoren-9-yl)methyl((9*S*)-9-ethyl-5-fluoro-9-hydroxy-10,13-dioxo-4-ethenyl-2,3,9,10,13,15-hexahydro-1*H*,12*H*-benzo[*de*]pyrano[3',4',6,7]indolizino[1,2-*b*]quinolin-1-yl)carbamate **7a**

The compound **3k** (140 mg, 0.20 mmol) and potassium vinyltrifluoroborate (54 mg, 0.40 mmol) were dissolved in dioxane (16 mL) and water (4 mL), and potassium phosphate (128 mg, 0.60 mmol) and methanesulfonato(2-dicyclohexylphosphino-2',6'-diisopropoxy-1,1'-biphenyl)(2-amino-1,1'-biphenyl-2-yl)palladium (II) (34 mg, 0.04 mmol) were added. After nitrogen replacement, the reaction mixture was stirred at 100 °C for 5 h. After the reaction was complete, water (20 mL) was added into the reaction mixture. The mixture was extracted with dichloromethane (15 mL×3). The organic phases were combined, washed with saturated brine, dried, and concentrated. The resulting residue was purified by silica gel column chromatography with the eluent system B, to give the title compound **7a** (80 mg, yield: 60%).

MS m/z (ESI): 670.2 (M+1)+.

Step II (*S*)-1-amino-9-ethyl-5-fluoro-9-hydroxy-4-ethenyl-1,2,3,9,12,15-hexahydro-10*H*,13*H*-benzo[*de*]pyrano[3',4',6,7]indolizino[1,2-*b*]quinoline-10,13-dione

The compound **7a** (80 mg, 0.12 mmol) was dissolved in *N,N*-dimethylformamide (10 mL), diethylamine (1 mL) was added, and the reaction mixture was stirred at room temperature for 1 h. After the reaction was complete, the mixture was vacuum concentrated, slurried with ethyl acetate, and filtered to give the title compound **7b** (30 mg, yield: 51%).

MS m/z (ESI): 448.2 (M+1)+.

Step III

(*R*)-*N*-((1*S*,9*S*)-9-ethyl-5-fluoro-9-hydroxy-10,13-dioxo-4-ethenyl-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-9-ethyl-5-fluoro-9-hydroxy-10,13-dioxo-4-ethenyl-2,3,9,10,13,15-hexahydro-1*H*,12*H*-benzo[*de*]pyrano[3',4',6,7]indolizino[1,2-*b*]quinolin-1-yl)-3-hydroxybutanamide

The compound **7b** (30 mg, 0.07 mmol) and (*R*)-3-hydroxybutyric acid (8 mg, 0.08 mmol) were dissolved in *N,N*-dimethylformamide (5 mL), and *N,N*-diisopropylethylamine (18.2 mg, 0.14 mmol) and 2-(7-azabenzotriazole)-*N,N,N',N'*-tramethyluronium hexafluorophosphate (39.9 mg, 0.11 mmol) were added. The reaction mixture was stirred at 25 °C for 5 h. After the reaction was complete, the reaction mixture was concentrated, and purified by preparative high performance liquid chromatography (Waters MS-triggered Prep-LC with SQD2 detector, chromatographic column: Xbridge C18 150*19 mm, 5 $\mu$m; mobile phase 1: water (containing 0.1% formic acid); mobile phase 2: acetonitrile; 15-min gradient, gradient proportion: acetonitrile phase 43%-53%, flow rate: 20 mL/min) to give the title compound **7-1** (1.76 mg, yield: 10%) and the title compound **7-2** (2.31 mg, yield: 10%).

Monoconfigurational compound **7-1** (short retention time):

MS m/z (ESI): 534.2 (M+1) $^+$.

$^1$H NMR (400 MHz, DMSO-$d_6$) $\delta$ 8.51 (d, 1H), 8.48 (s, 1H), 7.84 (d, 1H), 7.32 (s, 1H), 6.97 (dd, 1H), 6.58 (s, 1H), 5.91-5.77 (m, 2H), 5.61-5.50 (m, 1H), 5.43 (s, 2H), 5.22 (q, 2H), 4.72 (s, 1H), 4.04 (dd, 1H), 3.27 (d, 1H), 2.30-2.20 (m, 2H), 2.11 (t, 2H), 1.90-1.80 (m, 2H), 1.09 (d, 3H), 0.87 (t, 3H).

Monoconfigurational compound **7-2** (long retention time):

MS m/z (ESI): 534.2 (M+1) $^+$.

$^1$H NMR (400 MHz, DMSO-$d_6$) $\delta$ 8.55 (d, 1H), 8.47 (s, 1H), 7.85 (d, 1H), 7.32 (s, 1H), 6.97 (dd, 1H), 6.62 (s, 1H), 5.82 (dd, 2H), 5.57 (dt, 1H), 5.43 (d, 2H), 5.24 (s, 2H), 4.72 (s, 1H), 4.03 (dt, 1H), 3.27 (d, 1H), 2.23 (ddd, 2H), 2.08 (dd, 2H), 1.87 (tt, 2H), 1.08 (d, 3H), 0.87 (s, 3H).

## Example 8

(*R*)-*N*-((1*S*,9*S*)-9-ethyl-5-fluoro-9-hydroxy-10,13-dioxo-4-(2,2,2-trifluoroethoxy)-2,3,9,10,13,15-hexahydro-1*H*,12*H* benzo[*de*]pyrano[3',4',6,7]indolizino[1,2-*b*]quinolin-1-yl)-3-hydroxybutanamide

(*R*)-*N*-((1*R*,9*S*)-9-ethyl-5-fluoro-9-hydroxy-10,13-dioxo-4-(2,2,2-trifluoroethoxy)-2,3,9,10,13,15-hexahydro-1*H*,12*H*-benzo[*de*]pyrano[3',4',6,7]indolizino[1,2-*b*]quinolin-1-yl)-3-hydroxybutanamide

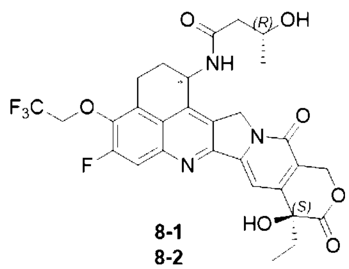

**8-1**
**8-2**

*-C represents that the chirality of the atom is R or S

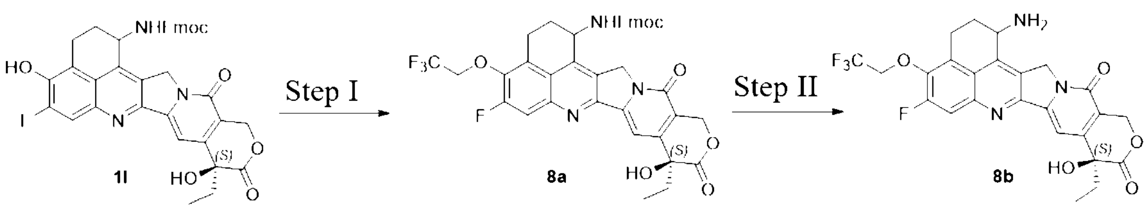

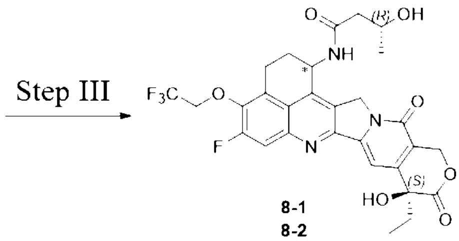


8-1
8-2

*-C represents that the chirality of the atom is R or S

Step I (9*H*-fluoren-9-yl)methyl((9*S*)-9-ethyl-5-fluoro-9-hydroxy-10,13-dioxo-4-(2,2,2-trifluoroethoxy)-2,3,9,10,13,15-hexahydro-1*H*,12*H*-benzo[*de*]pyrano[3',4',6,7]indolizino[1,2-*b*]quinolin-1-yl)carbamate **8a**

The compound **1l** (200 mg, 0.30 mmol) was dissolved in *N,N*-dimethylformamide (3 mL), and 2,2,2-trifluoroethyl trifluoromethanesulfonate (141 mg, 0.60 mmol) and cesium carbonate (296 mg, 0.90 mmol) were slowly added. The reaction mixture was stirred at room temperature for 10 min. After the reaction was complete, the mixture was diluted with water (10 mL), and extracted with ethyl acetate (10 mL×3). The organic phases were combined and concentrated. The resulting residue was purified by silica gel column chromatography with the eluent system B, to give the title compound **8a** (180 mg, yield: 75%).
MS m/z (ESI): 742.2 (M+H) +.

Step II (9*S*)-1-amino-9-ethyl-5-fluoro-9-hydroxy-4-(2,2,2-trifluoroethoxy)-1,2,3,9,12,15-hexahydro-10*H*,13*H*-benzo[*de*]pyrano[3',4',6,7]indolizino[1,2-*b*]quinoline-10,13-dione **8b**

The compound **8a** (180 mg, 0.24 mmol) was dissolved in *N,N*-dimethylformamide (3 mL), to which diethylamine (35 mg, 0.48 mmol) was added. The reaction mixture was kept for reaction at room temperature for 1 h. After the reaction was complete, the reaction mixture was concentrated under reduced pressure to give the crude product **8b** (100 mg), which was directly used in the next reaction step without purification.
MS m/z (ESI): 520.1 (M+H) +.

Step III

(*R*)-*N*-((1S,9S)-9-ethyl-5-fluoro-9-hydroxy-10,13-dioxo-4-(2,2,2-trifluoroethoxy)-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-9-ethyl-5-fluoro-9-hydroxy-10,13-dioxo-4-(2,2,2-trifluoroethoxy)-2,3,9,10,13,15-hexahydro-1*H*,12*H*-benzo[*de*]pyrano[3',4',6,7]indolizino[1,2-*b*]quinolin-1-yl)-3-hydroxybutanamide

The compound **8b** (50 mg, 0.09 mmol) was dissolved in *N,N*-dimethylformamide (2 mL), and (*R*)-3-hydroxybutyric acid (15 mg, 0.14 mmol), 2-(7-azabenzotriazole)-*N,N,N'N'*-tramethyluronium hexafluorophosphate (73 mg, 0.19 mmol), and *N,N*-diisopropylethylamine (37 mg, 0.28 mmol) were added. The mixture was stirred for reaction at room temperature for 1 h. After the reaction was complete, the mixture was concentrated under reduced pressure to give the crude product, which was purified by preparative high performance liquid chromatography (GILSON Prep LC with UV detector, chromatographic column: Xtimate C18 150*19 mm, 10 μm; mobile phase 1: water (containing 0.1% FA); mobile phase 2: acetonitrile; 15-min gradient, gradient proportion: acetonitrile phase 38%-48%, flow rate: 50 mL/min) to give the title compound **8-1** (2.5 mg, yield: 4.6%) and the title compound **8-2** (3.5 mg, yield: 6.4%).

Monoconfigurational compound **8-1** (short retention time):

MS m/z (ESI): 606.2 (M+1) +.
$^1$H NMR (400 MHz, DMSO-$d_6$) δ 8.43 (d, 1H), 7.98 (d, 1H), 7.31 (s, 1H), 6.52 (s, 1H), 5.59-5.53 (m, 1H), 5.42 (s, 2H), 5.23 (s, 2H), 4.93-4.85 (m, 2H), 4.66 (d, 1H), 4.08-4.03 (m, 1H), 3.21 (d, 2H), 2.34-2.27 (m, 1H), 2.22 (d, 1H), 2.10 (dd, 2H), 1.89-1.81 (m, 2H), 1.10 (d, 3H), 0.90-0.84 (m, 3H).

Monoconfigurational compound **8-2** (long retention time):

MS m/z (ESI): 606.2 (M+1)+.

$^1H$ NMR (400 MHz, DMSO-$d_6$) δ 8.45 (d, 1H), 7.98 (d, 1H), 7.32 (s, 1H), 6.52 (s, 1H), 5.63-5.55 (m, 1H), 5.43 (s, 2H), 5.33-5.18 (m, 2H), 4.88 (dd, 2H), 4.65 (d, 1H), 4.09-4.00 (m, 1H), 3.24-3.12 (m, 2H), 2.28 (dd, 1H), 2.19 (dd, 1H), 2.09 (dd, 2H), 1.93-1.78 (m, 2H), 1.09 (d, 3H), 0.94-0.78 (m, 3H).

**Example 9**

(*S*)-9-ethyl-4,9-dihydroxy-1,2,3,9,12,15-hexahydro-10*H*,13*H*-benzopyrano[3',4',6,7]indolizino[1,2-*b*]quinoline-10,13-dione **9**

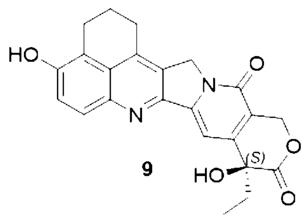

**9**

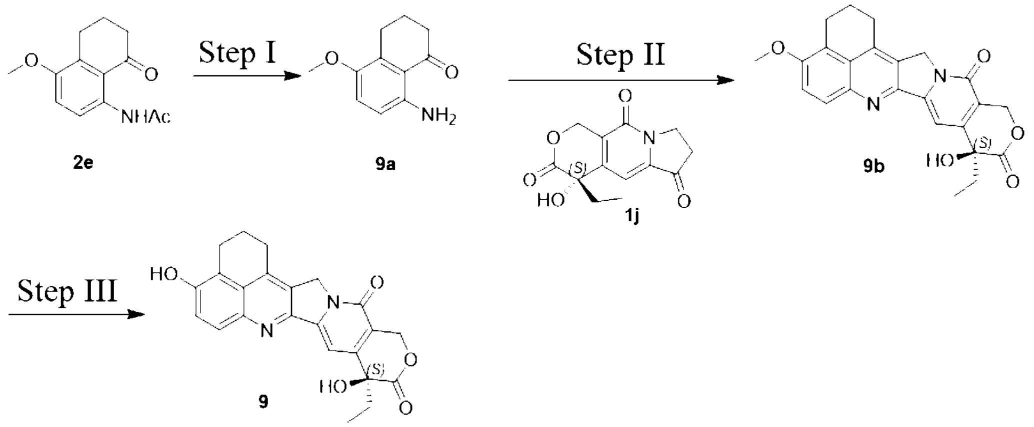


**2e** **9a** **1j** **9b** **9**

Step I 8-amino-5-methoxy-3,4-dihydronaphthalen-1(2*H*)-one **9a**

The compound **2e** (500 mg, 2.14 mmol) was dissolved in a mixed solvent of methanol and concentrated hydrochloric acid (36 mL, V/V=5:1). The reaction mixture was kept for reaction at 60 °C for 2 h. After the reaction was complete, the reaction mixture was directly concentrated, to give the title compound **9a** (400 mg, yield: 88%).

MS m/z (ESI): 192.1 $(M+1)^+$.

Step II (*S*)-9-ethyl-9-hydroxy-4-methoxy-1,2,3,9,12,15-hexahydro-10*H*,13*H*-benzopyrano[3',4',6,7]indolizino[1,2-*b*]quinoline-10,13-dione **9b**

The compound **9a** (400 mg, 2.09 mmol) was dissolved in toluene (15 mL), and the compound **1j** (661 mg, 2.51 mmol) was added. The reaction mixture was stirred at 110 °C for 16 h. After the reaction was complete, water (20 mL) was added into the reaction mixture. The mixture was extracted with dichloromethane (15 mL×3). The organic phases were combined, washed with saturated brine, dried, and concentrated. The purification was performed by silica gel column chromatography with the eluent system B to give the title compound **9b** (698 mg, yield: 80%).

MS m/z (ESI): 419.1 $(M+1)^+$.

Step III (*S*)-9-ethyl-4,9-dihydroxy-1,2,3,9,12,15-hexahydro-10*H*,13*H*-benzopyrano[3',4',6,7]indolizino[1,2-b]quinoline-10,13-dione **9**

The compound **9b** (100 mg, 0.24 mmol) was dissolved in hydrobromic acid (30 mL). The reaction mixture was kept for reaction at 100 °C for 2 h. After the reaction was complete, the reaction mixture was directly concentrated, and then purified by preparative high performance liquid chromatography (Waters MS triggered Prep-LC with Acquity QDA detector, chromatographic column: Welch C18 250×21.2 mm, 10 μm; mobile phase 1: water (containing 0.1% ammonia

water); mobile phase 2: acetonitrile; 15-min gradient, gradient proportion: acetonitrile phase 10%-30%, flow rate: 25 mL/min) to give the title compound 9 (19.4 mg, yield: 20%).

MS m/z (ESI): 405.1 (M+1)$^+$.

$^1$H NMR (400 MHz, $CD_3OD$) $\delta$ 7.79-7.71 (m, 2H), 7.29 (d, 1H), 5.01 (d, 2H), 4.95 (d, 2H), 3.01-2.86 (m, 4H), 2.43-2.35(m, 1H), 2.28-2.19 (m, 1H), 2.06-2.00 (m, 2H), 1.10-1.00 (m, 3H).

**Example 10**

(1*S*,3*R*)-N-((1*S*,10*S*)-10-ethyl-6-fluoro-10-hydroxy-5-methyl-11,14-dioxo-1,2,3,4,10,11,14,16-octahydro-13*H*-cyclohepta[*de*]pyrano[3',4':6,7]indolizino[1,2-*b*]quinolin-1-yl)-3-hydroxycyclobutane-1-carboxamide

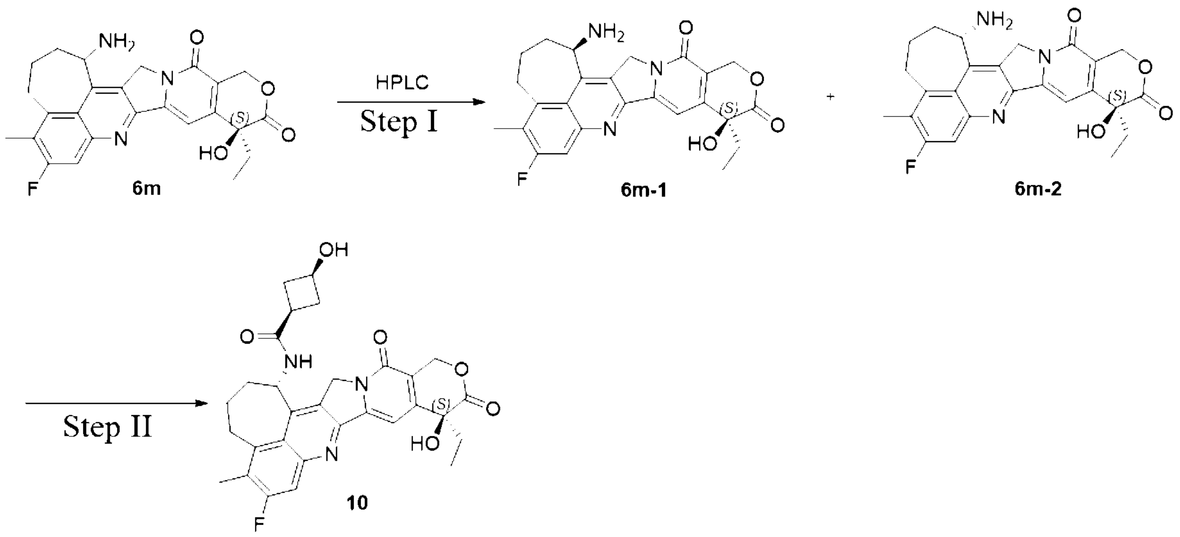

6m, 6m-1, 6m-2, 10

Step I

(1*R*,10*S*)-1-amino-10-ethyl-6-fluoro-10-hydroxy-5-methyl-2,3,4,10,13,16-hexahydro-14*H*-cyclohepta[3',4':6,7]indolizino[1,2-*b*]quinoline-11,14-(1*H*)-dione

(1*S*,10*S*)-1-amino-10-ethyl-6-fluoro-10-hydroxy-5-methyl-2,3,4,10,13,16-hexahydro-14*H*-cyclohepta[3',4':6,7]indolizine[1,2-*b*]quinoline-11,14-(1*H*)-dione

Compound **6m** (700 mg) was purified by preparative high performance liquid chromatography (GILSON Prep LC with UV detector, chromatographic column: Xbridge 5 μm C18 150x30 mm; mobile phase 1: water (0.1% TFA); mobile phase 2: acetonitrile; 15-min gradient, gradient proportion: acetonitrile phase 22%-32%, flow rate: 50 mL/min), to give compounds **6m-1** (290 mg, short retention time) and **6m-2** (300 mg, long retention time).

MS m/z (ESI): 450.2 (M+H)$^+$

Step II

(1*S*,3*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta[*de*]pyrano[3',4':6,7]indolizino[1,2-*b*]quinolin-1-yl)-3-hydroxycyclobutane-1-carboxamide **10**

The compound **6m-2** (30 mg, 0.06 mmol) was dissolved in *N*,*N*-dimethylformamide (3 mL), and (15,35)-3-hydroxycyclobutane-1-carboxylic acid (8.5 mg, 0.07 mmol), 2-(7-azabenzotriazole)-*N*,*N*,*N'*,*N'*-tramethyluronium hexafluorophosphate (38 mg, 0.10 mmol), and N,N-diisopropylethylamine (17.2 mg, 0.13 mmol) were added. The reaction mixture was stirred at room temperature for 1 h. After the reaction was complete, the reaction mixture was diluted with water (10 mL), and extracted with ethyl acetate (10 mL×3). The organic phases were combined, washed with saturated brine, dried, and concentrated to give the crude product, which was purified by preparative high performance liquid chromatography (GILSON Prep LC with UV detector, chromatographic column: Xbridge 5 μm C18 150x30 mm; mobile phase 1: water (containing 0.1% TFA); mobile phase 2: acetonitrile; 15-min gradient, gradient proportion: acetonitrile

phase 30%-95%, flow rate: 50 mL/min) to give the compound **10** (2 mg, yield: 5%).

MS m/z (ESI): 548.2 $(M+H)^+$.

$^1H$ NMR (400 MHz, $CD_3OD$) δ 8.71 (d, 1H), 7.57-7.48 (m, 2H), 5.65 (s, 1H), 5.55 (d, 1H), 5.35 (dd, 2H), 5.10 (d, 1H), 4.13 (dd, 1H), 2.73 (dd, 1H), 2.61-2.33 (m, 8H), 2.20-2.08 (m, 4H), 1.96 (dd, 4H), 1.02-0.97 (m, 3H).

**Example 11**

(*R*)-2-cyclopropyl-N-((1*S*,10*S*)-10-ethyl-6-fluoro-10-hydroxy-5-methyl-11,14-dioxo-1,2,3,4,10,11,14,16-octahydro-13*H*-cyclohepta[de]pyrano[3',4':6,7]indolizino[1,2-*b*]quinolin-1-yl)-2-hydroxyacetamide

(*S*)-2-cyclopropyl-N-((1*S*,10*S*)-10-ethyl-6-fluoro-10-hydroxy-5-methyl-11,14-dioxo-1,2,3,4,10,11,14,16-octahydro-13*H*-cyclohepta[de]pyrano[3',4':6,7]indolizino[1,2-*b*]quinolin-1-yl)-2-hydroxyacetamide

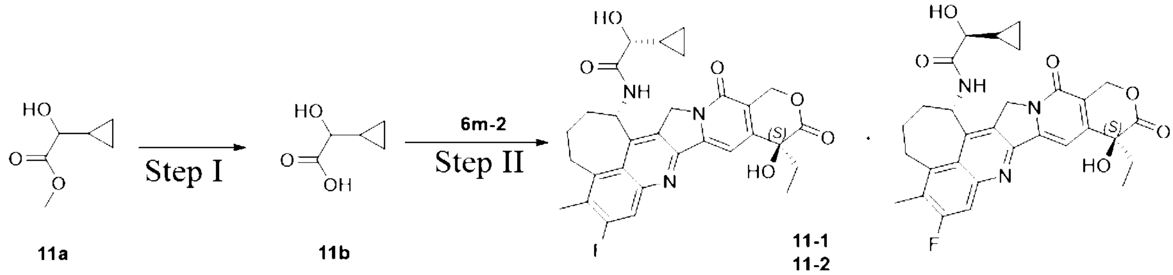


**11a** **11b** **11-1** **11-2**

Step I 2-cyclopropyl-2-hydroxyacetic acid **11b**

Methyl 2-cyclopropyl-2-hydroxyacetate **11a** (50 mg, 0.38 mmol) was dissolved in tetrahydrofuran (6 mL), and water (0.5 mL) and lithium hydroxide (27 mg, 1.15 mmol) were added. The mixture was stirred for reaction at room temperature for 1 h. After the reaction was complete, the mixture was spin-dried to remove the solvent, thus giving the crude product 2-cyclopropyl-2-hydroxyacetic acid **11b** (50 mg, white solid), which was directly used in the next reaction step without purification.

MS m/z (ESI): 117.1 $(M+H)^+$.

Step II

(*R*)-2-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-2-hydroxyacetamide

(*S*)-2-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-2-hydroxyacetamide

The compound **6m-2** (50 mg, 0.11 mmol) was dissolved in *N*,*N*-dimethylformamide (2 mL), and 2-cyclopropyl-2-hydroxyacetic acid **11b** (44 mg, 0.38 mmol), *N*,*N*-diisopropylethylamine (29 mg, 0.22 mmol), and 2-(7-azabenzotriazole)-*N*,*N*,*N*',*N*'-tetramethyluronium hexafluorophosphate (63 mg, 0.17 mmol) were added. The mixture was stirred for reaction at room temperature for 15 min. After the reaction was complete, the purification was performed by preparative high performance liquid chromatography (Waters MS-triggered Prep-LC with SQD2 detector, chromatographic column: Xbridge 5 μm C18 150x19 mm; mobile phase 1: water (containing 0.1% FA); mobile phase 2: acetonitrile; 10-min gradient, gradient proportion: acetonitrile phase 40%-50%, flow rate: 20 mL/min) to give compound **11-1** (2.3 mg, yield: 4%) and compound **11-2** (2.5 mg, yield: 4%).

Monoconfigurational compound **11-1** (compound with short retention time)

MS m/z (ESI): 548.2 $(M+H)^+$.

$^1H$ NMR (400 MHz, DMSO-$d_6$) δ 8.49 (d, 1H), 7.74 (d, 1H), 7.28 (s, 1H), 6.50 (s, 1H), 5.56 (s, 1H), 5.49-5.40 (m, 4H), 5.29 (d, 1H), 3.61 (t, 1H), 3.22 (s, 2H), 2.42 (s, 3H), 2.12-1.63 (m, 6H), 1.11 (d, 1H), 0.87 (t, 3H), 0.47-0.27 (m, 4H).

Monoconfigurational compound **11-2** (compound with long retention time)

MS m/z (ESI): 548.2 $(M+H)^+$.

$^1H$ NMR (400 MHz, DMSO-$d_6$) δ 8.43 (d, 1H), 7.74 (d, 1H), 7.28 (s, 1H), 6.51 (s, 1H), 5.54 (s, 1H), 5.42 (t, 4H), 5.31 (d, 1H), 3.68-3.53 (m, 1H), 3.22 (s, 2H), 2.42 (s, 3H), 2.12-1.66 (m, 6H), 1.10 (d, 1H), 0.87 (t, 3H), 0.48-0.28 (m, 4H).

**Example 12**

(1*S*,3*R*)-*N*-((1*S*,9*S*)-9-ethyl-5-fluoro-9-hydroxy-10,13-dioxo-4-ethenyl-2,3,9,10,13,15-hexahydro-1*H*,12*H*-benzo[*d*]pyrano[3',4':6,7]indolizino[1,2-*b*]quinolin-1-yl)-3-hydroxycyclobutane-1-carboxamide

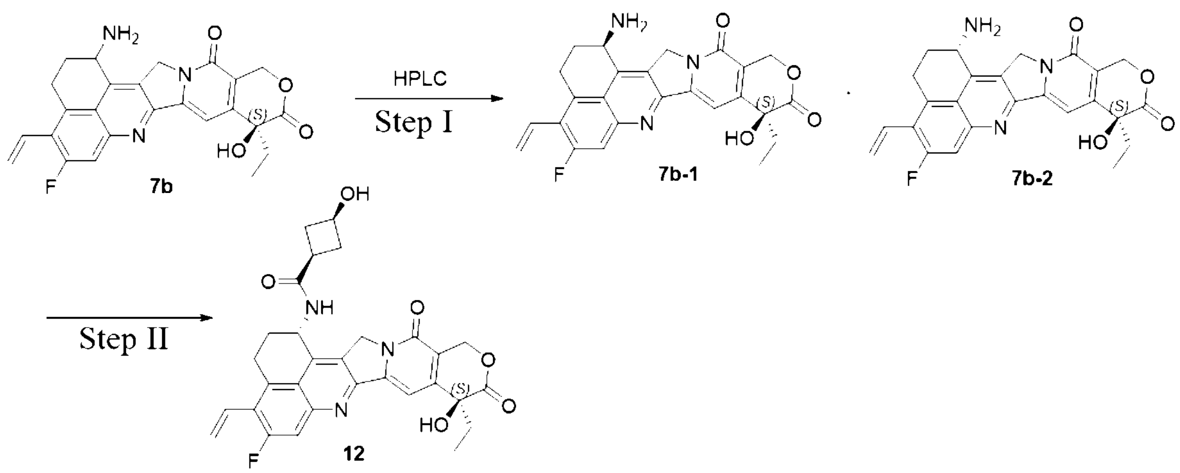


7b, 7b-1, 7b-2, 12

Step I (1*R*,9*S*)-1-amino-9-ethyl-5-fluoro-9-hydroxy-4-ethenyl-1,2,3,9,12,15-hexahydro-10*H*,13*H*-benzo[*de*]pyrano[3',4':6,7]indolizino[1,2-*b*]quinoline-10,13-dione

(1*S*,9*S*)-1-amino-9-ethyl-5-fluoro-9-hydroxy-4-ethenyl-1,2,3,9,12,15-hexahydro-10*H*,13*H*-benzo[*de*]pyrano[3',4':6,7]indolizino[1,2-*b*]quinoline-10,13-dione

The compound **7b** (500 mg) was purified by preparative high performance liquid chromatography (GILSON Prep LC with UV detector, chromatographic column: Xbridge 5 μm C18 150x30 mm; mobile phase 1: water (0.1% TFA); mobile phase 2: acetonitrile; 15-min gradient, gradient proportion: acetonitrile phase 22%-32%, flow rate: 50 mL/min), to give compounds **7b-1** (180 mg, short retention time) and **7b-2** (195 mg, long retention time).

MS m/z (ESI): 448.2 $(M+H)^+$.

Step II (1*S*,3*R*)-*N*-((1*S*,9*S*)-9-ethyl-5-fluoro-9-hydroxy-10,13-dioxo-4-ethenyl-2,3,9,10,13,15-hexahydro-1*H*,12*H*-benzo[*d*]pyrano[3',4':6,7]indolizino[1,2-*b*]quinolin-1-yl)-3-hydroxycyclobutane-1-carboxamide **12**

The compound **7b-2** (22 mg, 0.05 mmol) was dissolved in *N*,*N*-dimethylformamide (0.5 mL), and (1S,3S)-3-hydroxycyclobutane-1-carboxylic acid (7 mg, 0.06 mmol), 2-(7-azabenzotriazole)-*N*,*N*,*N'*,*N'*-tramethyluronium hexafluorophosphate (30 mg, 0.08 mmol), and *N,N*-diisopropylethylamine (13 mg, 0.10 mmol) were added. The mixture was stirred for reaction at room temperature for 15 min. After the reaction was complete, the reaction mixture was purified by preparative high performance liquid chromatography (Waters MS-triggered Prep-LC with SQD2 detector, chromatographic column: Xbridge 5 μm C18 150x19 mm; mobile phase 1: water (containing 0.1% FA); mobile phase 2: acetonitrile; 10-min gradient, gradient proportion: acetonitrile phase 45%-100%, flow rate: 20 mL/min) to give the compound **12** (3 mg, yield: 11%).

MS m/z (ESI): 546.2 $(M+H)^+$.

$^1H$ NMR (400 MHz, $CD_3OD$) δ 8.50 (s, 1H), 7.71 (d, 1H), 7.64 (s, 1H), 6.95 (dd, 1H), 5.83 (dd, 2H), 5.67-5.61 (m, 1H), 5.57 (d, 1H), 5.39 (d, 1H), 5.24 (d, 2H), 4.16-4.05 (m,1H), 3.45-3.35 (m, 2H), 2.73-2.42 (m, 4H), 2.29-2.17 (m, 4H), 2.01-1.91 (m, 2H), 1.00 (t, 3H).

**Example 13**

(*R*)-*N*-((1*R*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxybutanamide

(*R*)-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxybutanamide

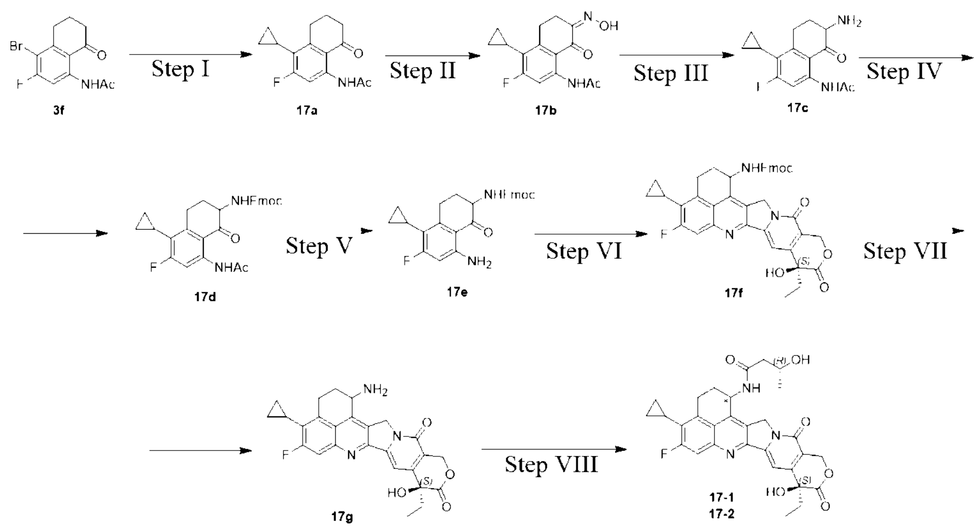


*-C represents that the chirality of the atom is R or S

Step I *N*-(4-cyclopropyl-3-fluoro-8-oxo-5,6,7,8-tetralin-1-yl)acetamide **17a**

The compound **3f** (1.5 g, 5.02 mmol), cyclopropylboronic acid (1.3 g, 15.06 mmol), [1,1-bis(diphenylphosphino) ferrocene]dichloropalladium (2.2 g, 3.01 mmol), and cesium carbonate (1.6 g, 15.06 mmol) were dissolved in dioxane (15 mL). The mixture was stirred for reaction in a microwave reactor at 110 °C for 2 h. After the reaction was complete, the reaction mixture was poured into water, extracted with ethyl acetate three times, and dried over anhydrous sodium sulfate. The organic phases were combined, concentrated, and purified by silica gel column chromatography with the eluent system B to give the title compound **17a** (1.1 g, yield: 84%).
MS m/z (ESI): 262.1 $(M+1)^+$.

Step II (Z)-N-(4-cyclopropyl-3-fluoro-7-(hydroxyimino)-8-oxo-5,6,7,8-tetralin-1-yl)acetamide **17b**

Potassium tert-butoxide (0.94 g, 8.4 mmol) was dissolved in a mixed solvent of tetrahydrofuran and tert-butanol (50 mL, V/V=4:1), the compound **17a** (1.1 g, 4.2 mmol) was dissolved in 10 mL of the tetrahydrofuran solution, and the mixture was slowly added to the reaction system at 0 °C. After the mixture was stirred for reaction at 0 °C for 10 min, isoamyl nitrite (0.74 g, 6.3 mmol) was added, and the reaction was continued by stirring at 0 °C for 50 min. After the reaction was complete, the system was adjusted with dilute hydrochloric acid to a pH of 4-5, and extracted with ethyl acetate 3 times. The organic phase was washed with saturated brine, collected, dried over anhydrous sodium sulfate, and spin-dried to remove the solvent, thus giving the crude product **17b,** which was directly used in the next reaction step without purification.
MS m/z (ESI): 291.0 $(M+1)^+$.

Step III N-(7-amino-4-cyclopropyl-3-fluoro-8-oxo-5,6,7,8-tetralin-1-yl)acetamide **17c**

The compound **17b** was dissolved in methanol (50 mL), and a palladium-carbon catalyst (500 mg) was added. The mixture was stirred for reaction in a hydrogen environment at room temperature for 2 h. After the reaction was complete, the mixture was filtered. The filtrate was collected to give the crude product **17c,** which was directly used in the next reaction step without purification.
MS m/z (ESI): 277.1 $(M+1)^+$.

Step IV (9*H*-fluoren-9-yl)methyl(8-acetylamino-5-cyclopropyl-6-fluoro-1-oxo-1,2,3,4-tetralin-2-yl)carbamate **17d**

The filtrate obtained from the previous step was adjusted with a saturated sodium carbonate solution to a PH of 8-9, and then Fmoc-Cl (1.19 g, 4.6 mmol) was added. The mixture was stirred for reaction at room temperature for 1 h. After the reaction was complete, the system was extracted with ethyl acetate. The organic phase was washed with saturated brine, collected, dried over anhydrous sodium sulfate, and spin-dried to remove the solvent, thus giving the crude product, which was purified through the silica gel column chromatography system A to give the compound **17d** (1 g, yield: 48%).

MS m/z (ESI): 499.1 $(M+1)^+$.

Step V (9*H*-fluoren-9-yl)methyl(8-amino-5-cyclopropyl-6-fluoro-1-oxo-1,2,3,4-tetralin-2-yl)carbamate **17e**

The compound **17e** (1 g, 2.01 mmol) was dissolved in dioxane (20 mL), and concentrated hydrochloric acid (12 mol/L, 5 mL) was added. The mixture was stirred for reaction at 60 °C for 1 h. After the reaction was complete, the mixture was spin-dried to remove the solvent, thus giving the crude product, which was purified through the silica gel column chromatography system A to give compound **17f** (700 mg, yield: 77%).

MS m/z (ESI): 457.1 $(M+1)^+$.

Step VI (9*H*-fluoren-9-yl)methyl((9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)carbamate **17g**

The compound **17f** (700 mg, 1.54 mmol) was dissolved in toluene (10 mL), and (S)-4-ethyl-4-hydroxy-7,8-dihydro-1*H*-pyrano[3,4-f]indolizine-3,6,10(4*H*)-trione (484 mg, 1.84 mmol), and p-toluenesulfonic acid monohydrate (292 mg, 1.54 mmol) were added. The reaction system was stirred for reaction at 140 °C for 4 h. After the reaction was complete, the mixture was spin-dried to remove the solvent, thus giving the crude product, which was purified through a silica gel column chromatography system Achu to give the title compound **17g** (700 mg, yield: 66%).

MS m/z (ESI): 684.2 $(M+1)^+$.

Step VII (9*S*)-1-amino-4-cyclopropyl-9-ethyl-5-fluoro-9-hydroxy-1,2,3,9,12,15-hexahydro-10*H*,13*H*-benzo[*d*]pyrano[3',4':6,7]indolizino[1,2-b]quinoline-10,13-dione **17h**

The compound **17g** (100 mg, 0.15 mmol) was dissolved in *N*,*N*-dimethylformamide (5 mL), and diethylamine (0.5 mL) was added. The mixture was stirred for reaction at room temperature for 30 min. After the reaction was complete, the mixture was vacuum concentrated to give the crude compound **17h** (60 mg), which was directly used in the next reaction step without purification.

MS m/z (ESI): 462.1 $(M+1)^+$.

Step VIII (*R*)-*N*-((1*R*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxybutanamide

(*R*)-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxybutanamide

The compound **17h** (60 mg, 0.13 mmol) was dissolved in DMF (5 mL), and then (*R*)-3-hydroxybutyric acid (19 mg, 0.18 mmol), 2-(7-azabenzotriazole)-*N*,*N*,*N'*,*N'*-tramethyluronium hexafluorophosphate (87 mg, 0.23 mmol), and *N*,*N*-dimethylacetamide (59 mg, 0.45 mmol) were added. The mixture was stirred for reaction at room temperature for 30 min. After the reaction was complete, the reaction mixture was purified by preparative high performance liquid chromatography (Waters MS-triggered Prep-LC with SQD2 detector, chromatographic column: Xbridge 5 μm C18 150x19 mm; mobile phase 1: water (containing 0.1% FA); mobile phase 2: acetonitrile; 15-min gradient, gradient proportion: acetonitrile phase 38%-48%, flow rate: 20 mL/min) to give compounds **17-1** (6 mg, yield: 11%) and **17-2** (4 mg, yield: 8%).

Monoconfigurational compound **17-1** (short retention time):

MS m/z (ESI): 548.2 $(M+1)^+$.

$^1$H NMR (400 MHz, DMSO-*$d_6$*) δ 8.44 (d, 1H), 7.75 (d, 1H), 7.47 (d, 1H), 7.30 (s, 1H), 7.11 (d, 1H), 5.60-5.52 (m, 1H), 5.43 (s, 2H), 5.29-5.13 (m, 2H), 4.09-4.00 (m, 1H), 2.48 (s, 1H), 2.29 (s, 2H), 2.24-2.18 (m, 1H), 2.14 (d, 2H), 1.99-1.92 (m, 1H), 1.91-1.79 (m, 2H), 1.15-1.10 (m, 2H), 1.09 (d, 3H),0.87 (s, 3H), 0.76 (s, 2H).

Monoconfigurational compound **17-2** (long retention time):

MS m/z (ESI): 548.2 $(M+1)^+$.

$^1H$ NMR (400 MHz, DMSO-$d_6$) δ 8.46 (d, 1H), 7.76 (d, 1H), 7.47 (d, 1H), 7.30 (s, 1H), 7.13-7.06 (m, 1H), 5.59 (dd, 1H), 5.43 (d, 2H), 5.24 (d, 2H), 4.05 (dd, 1H), 2.29 (t, 1H), 2.25 (d, 1H), 2.19 (d, 1H), 2.18-2.06 (m, 3H), 1.95 (d, 1H), 1.85 (dd, 2H), 1.16-1.10 (m, 2H), 1.08 (d, 3H), 0.87 (s, 4H), 0.76 (d, 2H).

**Example 14**

(1*S*,3*S*)-*N*-((1*R*,9*S*)-4-cyclopropyl-9-ethyl-5-fluoro-9-hydroxy-10,13-dioxo-2,3,9,10,13,15-hexahydro-1*H*,12*H*-benzo[d]pyrano[3',4':6,7]indolizino[1,2-*b*]quinolin-1-yl)-3-hydroxycyclobutane-1-carboxamide

(1*S*,3*R*)-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxycyclobutane-1-carboxamide

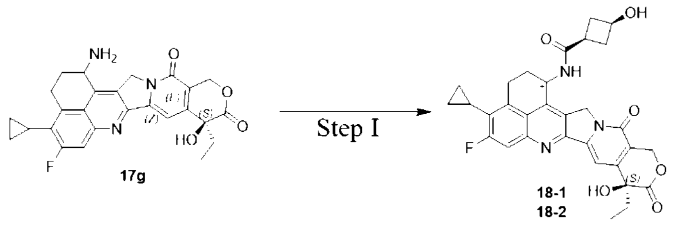


*-C represents that the chirality of the atom is R or S

Step I

(1*S*,3*S*)-*N*-((1*R*,9*S*)-4-cyclopropyl-9-ethyl-5-fluoro-9-hydroxy-10,13-dioxo-2,3,9,10,13,15-hexahydro-1*H*,12*H*-benzo[d]pyrano[3',4':6,7]indolizino[1,2-*b*]quinolin-1-yl)-3-hydroxycyclobutane-1-carboxamide

(1*S*,3*R*)-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxycyclobutane-1-carboxamide

The compound **17g** (69 mg, 0.15 mmol) was dissolved in *N,N*-dimethylformamide (5 mL), and (1S,3S)-3-hydroxycyclobutane-1-carboxylic acid (21 mg, 0.18 mmol), 2-(7-azabenzotriazole)-*N,N,N',N'*-tramethyluronium hexafluorophosphate (87 mg, 0.23 mmol), and *N,N*-dimethylacetamide (59 mg, 0.45 mmol) were added. The mixture was stirred for reaction at room temperature for 30 min. After the reaction was complete, the reaction mixture was purified by preparative high performance liquid chromatography (Waters MS-triggered Prep-LC with QDA detector, chromatographic column: WELCH Xtimate C18 21.2x250 mm 10 μm; mobile phase 1: water (containing 0.1% FA); mobile phase 2: acetonitrile; 18-min gradient, gradient proportion: acetonitrile phase 35%-65%, flow rate: 20 mL/min) to give compounds **18-1** (4 mg, yield: 6%) and **18-2** (2.4 mg, yield: 4%:)

Monoconfigurational compound **18-1** (short retention time):

MS m/z (ESI): 560.2 $(M+1)^+$.

$^1H$ NMR (400 MHz, DMSO-$d_6$) δ 8.44 (d, 1H), 7.71 (d, 1H), 7.29 (d, 1H), 6.54 (d, 1H), 5.56 (s, 1H), 5.42 (s, 2H), 5.17-5.09 (m, 3H), 3.94 (d, 1H), 2.44-2.30 (m, 4H), 2.08-2.03 (m, 4H), 1.94-1.84 (m, 4H), 1.11 (d, 2H), 0.87 (t, 3H), 0.76 (s, 2H).

Monoconfigurational compound **18-2** (long retention time):

MS m/z (ESI): 560.2 $(M+1)^+$.

$^1H$ NMR (400 MHz, DMSO-$d_6$) δ 8.45 (d, 1H), 7.74 (d, 1H), 7.30 (s, 1H), 6.53 (s, 1H), 5.57 (s, 1H), 5.43 (s, 2H), 5.14 (dd, 3H), 3.97-3.91 (m, 1H), 2.40-2.27 (m, 4H), 2.15-2.05 (m, 4H), 1.96-1.84 (m, 4H), 1.11 (d, 2H), 0.87 (t, 3H), 0.75 (d, 2H).

**Example 15**

(*R*)-*N*-((1*S*,10*S*)-5-cyclopropyl-10-ethyl-6-fluoro-10-hydroxy-11,14-dioxo-1,2,3,4,10,14,16-octahydro-13*H*-cyclohepta [de]pyrano[3',4':6,7]indolizino[1,2-b]quinolin-1-yl)-3-hydroxybutanamide

(*R*)-*N*-((1R,10*S*)-5-cyclopropyl-10-ethyl-6-fluoro-10-hydroxy-11,14-dioxo-1,2,3,4,10,14,16-octahydro-13*H*-cyclohepta [*de*]pyrano[3',4':6,7]indolizino[1,2-*b*]quinolin-1-yl)-3-hydroxybutanamide

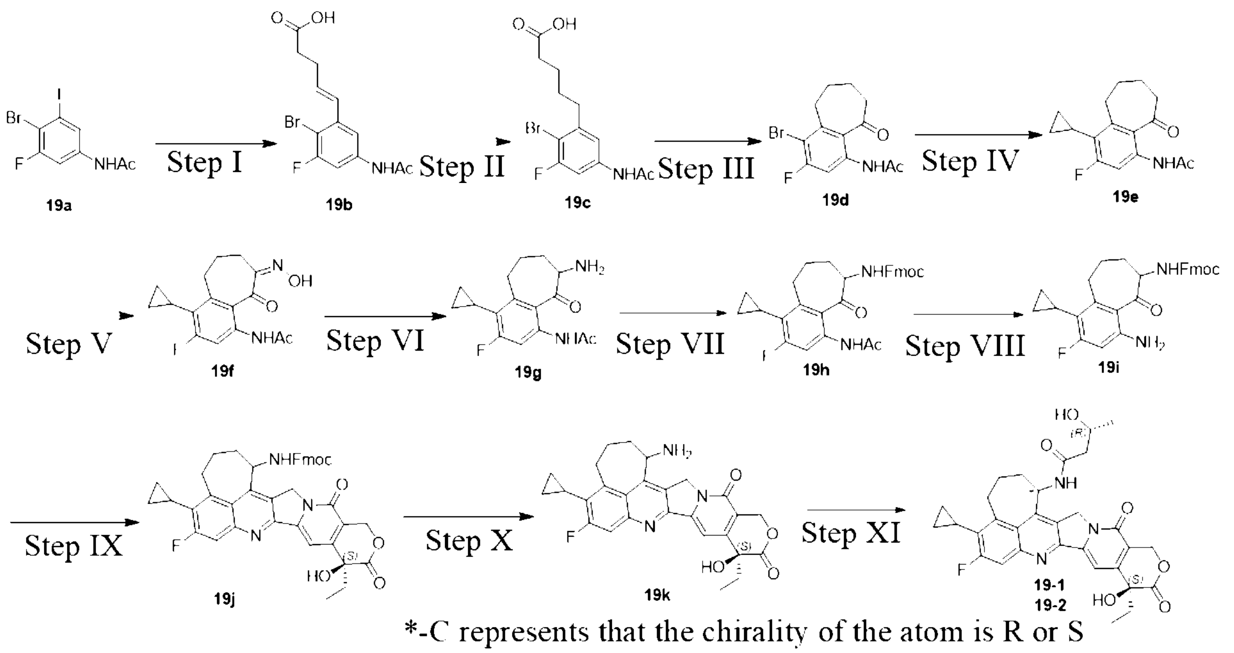


Step I 5-(5-acetylamino-2-bromo-3-fluorophenyl)pent-4-enoic acid **19b**

The compound **19a** (7.0 g, 19.5 mmol) was dissolved in dioxane (60 mL) and water (15 mL), to which pent-4-enoic acid (2.2 g, 22.0 mol), bis(triphenylphosphine)palladium chloride (700 mg, 1.0 mmol), and sodium carbonate (6.2 g, 58.4 mmol) were added. The reaction mixture was stirred under the protection of nitrogen at 90 °C for 16 h. After the reaction was complete, the reaction mixture was filtered through diatomite, and washed with ethyl acetate. The resulting filtrate was directly concentrated. The crude product was diluted with water (200 mL), and extracted with diethyl ether 3 times (100 mLx3). The aqueous phase was adjusted with dilute hydrochloric acid to pH=2, and extracted with ethyl acetate (100 mLx3). The organic phases were combined and concentrated to give the crude product **19b** (6.0 g).
MS m/z (ESI): 330.1 $(M+1)^+$.

Step II 5-(5-acetylamino-2-bromo-3-fluorophenyl)pentanoic acid **19c**

The compound **19b** (6.0 g, 18.2 mmol) was dissolved in anhydrous methanol (100 mL), to which platinum/carbon (5%, 600 mg) was added. The reaction mixture was subjected to hydrogen replacement three times, and stirred for reaction at room temperature for 16 h. After the reaction was complete, the reaction mixture was filtered through diatomite, and washed with ethyl acetate. The resulting filtrate was directly concentrated to give the crude compound **19c** (5.0 g).
MS m/z (ESI): 332.0 $(M+1)^+$.

Step III *N*-(4-bromo-3-fluoro-9-oxo-6,7,8,9-tetrahydro-5*H*-benzo[7]cycloalken-1-yl)acetamide **19d**

The compound **19c** (2.5 g, 3.3 mmol) was dissolved in polyphosphoric acid (25 mL). The reaction mixture was stirred for reaction at 140 °C for 2 h. After the reaction was complete, water (100 mL) was added to the reaction mixture, and the mixture was stirred for 4 h. The aqueous phase was extracted with ethyl acetate (100 mLx3). The organic phases were combined and concentrated. The resulting crude product was purified through the silica gel column chromatography system B to give the compound **19d** (920 mg, yield: 39%).

MS m/z (ESI): 314.0 $(M+1)^+$.

A synthetic route same as that in Example **17** was used as the subsequent synthetic route, except that the compound **3f** was replaced with the compound **19d** (720 mg, 2.3 mmol). The final product was purified by preparative high performance liquid chromatography (Waters MS-triggered Prep-LC with QDA detector, chromatographic column: WELCH Xtimate C18 21.2x250 mm 10 μm; mobile phase 1: water (containing 0.1% FA); mobile phase 2: acetonitrile; 15-min gradient, gradient proportion: acetonitrile phase 40%-95%, flow rate: 30 mL/min) to give products **19-1** (6.5 mg, yield: 9%) and **19-2** (11mg, yield: 16%).

Monoconfigurational compound **19-1** (short retention time):

MS m/z (ESI): 562.2 $(M+1)^+$.

$^1H$ NMR (400 MHz, $CDCl_3$) δ 7.63-7.50 (m, 3H), 7.07 (s, 1H), 5.73-5.69 (m, 2H), 5.43 (d, 1H), 5.36-5.15 (m, 2H), 4.24 (s, 1H), 3.81 (s, 1H),3.64-3.50 (m, 1H), 3.47-3.32 (m, 1H), 2.52-2.41 (m, 2H), 2.36 -2.12 (m, 3H), 1.88-1.83 (m, 4H), 1.25 (d, 3H), 1.19-1.15 (m, 2H), 1.02 (t, 3H), 0.81-0.77 (m, 2H).

Monoconfigurational compound **19-2** (long retention time):

MS m/z (ESI): 562.2 $(M+1)^+$.

$^1H$ NMR (400 MHz, $CDCl_3$) δ 7.50-7.35 (m, 3H), 5.72-5.55 (m, 1H), 5.47-5.29 (m, 2H), 5.26-5.05 (m, 2H), 4.51-4.31 (m, 1H), 4.05-3.91 (m, 1H), 3.70-3.54 (m, 1H), 3.53-3.37 (m, 1H), 2.66-2.50 (m, 2H), 2.49-2.37 (m, 1H), 2.28-2.14 (m, 1H), 2.11-1.75 (m, 5H), 1.41-1.29 (m, 3H), 1.29-1.10 (m, 2H),1.04-0.95 (m, 3H), 0.88-0.71 (m, 2H).

**Example 16**

(1*S*,3*S*)-*N*-((1*R*,10*S*)-5-cyclopropyl-10-ethyl-6-fluoro-10-hydroxy-11,14-dioxo-1,2,3,4,10,11,14,16-octahydro-13*H*-cyclohepta[*de*]pyrano[3',4':6,7]indolizino[1,2-*b*]quinolin-1-yl)-3-hydroxybutane-1-carboxamide

(1*S*,3*S*)-*N*-((1*S*,10*S*)-5-cyclopropyl-10-ethyl-6-fluoro-10-hydroxy-11,14-dioxo-1,2,3,4,10,11,14,16-octahydro-13*H*-cyclohepta[de]pyrano[3',4':6,7]indolizino[1,2-*b*]quinolin-1-yl)-3-hydroxybutane-1-carboxamide

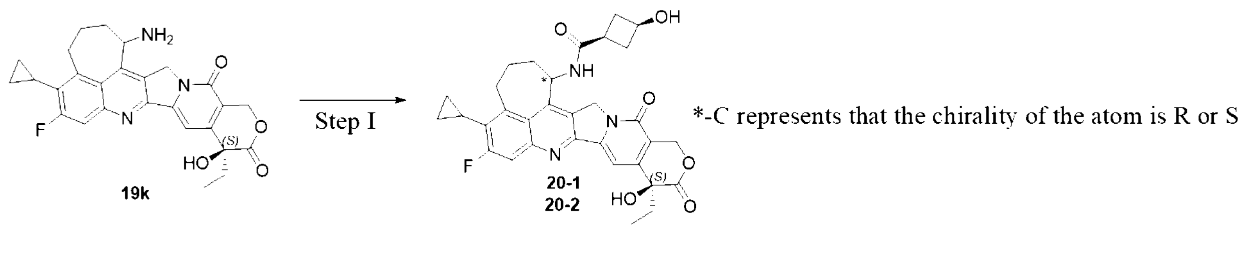


19k

20-1
20-2

Step I

(1*S*,3*S*)-*N*-((1*R*,10*S*)-5-cyclopropyl-10-ethyl-6-fluoro-10-hydroxy-11,14-dioxo-1,2,3,4,10,11,14,16-octahydro-13*H*-cyclohepta[de]pyrano[3',4':6,7]indolizino[1,2-*b*]quinolin-1-yl)-3-hydroxybutane-1-carboxamide

(1*S*,3*S*)-*N*-((1*S*,10*S*)-5-cyclopropyl-10-ethyl-6-fluoro-10-hydroxy-11,14-dioxo-1,2,3,4,10,11,14,16-octahydro-13*H*-cyclohepta[de]pyrano[3',4':6,7]indolizino[1,2-*b*]quinolin-1-yl)-3-hydroxybutane-1-carboxamide

The compound **19k** (75 mg, 0.16 mmol) was dissolved in *N,N*-dimethylformamide (3 mL), and (1S,3S)-3-hydroxycyclobutane-1-carboxylic acid (22 mg, 0.19 mmol), 2-(7-azabenzotriazole)-*N,N,N',N'*-tramethyluronium hexafluorophosphate (91 mg, 0.24 mmol), and *N,N*-diisopropylethylamine (171 mg, 1.32 mmol) were added. The reaction mixture was stirred for reaction at room temperature for 1 h. After the reaction was complete, the reaction mixture was diluted with water (50 mL), and extracted with ethyl acetate (30 mLx3). The organic phases were combined, washed with saturated brine, dried, and concentrated. The crude product was purified by preparative high performance liquid chromatography (GILSON Prep LC with UV detector, chromatographic column: WELCH Xtimate C18 21.2x250mm 10 μm; mobile phase 1: water (containing 0.1% NH3·H2O); mobile phase 2: acetonitrile; 15-min gradient, gradient proportion: acetonitrile phase 40%-100%, flow rate: 30 mL/min) to give compounds **20-1** (2.9 mg, 7%) and **20-2** (1.5 mg,

2%).

Monoconfigurational compound **20-1** (short retention time):

MS m/z (ESI): 574.7 $(M+1)^+$.

1H NMR (400 MHz, $CDCl_3$) δ 7.59 (d, 1H), 7.53 (s, 1H), 6.49 (s, 1H), 5.72-5.64 (m, 1H), 5.59 (d, 1H), 5.35 (d, 1H), 5.14 (d, 1H), 4.93 (d, 1H), 4.25-4.19(m, 1H), 3.77 (s, 1H), 3.64-3.52 (m, 1H), 3.46 -3.41 (m, 1H), 2.77-2.68 (m, , 3H), 2.42 -2.10 (m, 5H), 1.94-1.78 (m, 4H), 1.25-1.75 (m, 2H), 1.05 (t, 3H), 0.87-0.72 (m, 2H).

Monoconfigurational compound **20-2** (long retention time):

MS m/z (ESI): 574.7 $(M+1)^+$.

$^1$H NMR (400 MHz, $CDCl_3$) δ 7.53 (d, 1H), 7.46 (s, 1H), 6.91 (br, 1H), 5.59 (t, 1H), 5.30-5.19(m, 2H), 5.11-5.04 (m, 2H), 4.30-4.21 (m, 1H), 3.76 (s, 1H), 3.68 (d, 1H), 3.48-3.34 (m, 1H), 2.88-2.75 (m, 3H), 2.37-2.21 (m, 5H), 1.93 (s, 3H), 1.80-1.72 (m, 2H), 1.23-1.13 (m, 2H), 0.96 (t, 3H), 0.87-0.75 (m, 2H).

**Example 17**

(*R*)-2-cyclopropyl-*N*-((1*S*,10*S*)-5-cyclopropyl-10-ethyl-6-fluoro-10-hydroxy-11,14-dioxo-1,2,3,4,10,11,14,16-octahydro-13*H*-cyclohepta[3',4':6,7]indolizino[1,2-*b*]quinolin-1-yl)-2-hydroxyacetamide

(*S*)-2-cyclopropyl-*N*-((1*S*,10*S*)-5-cyclopropyl-10-ethyl-6-fluoro-10-hydroxy-11,14-dioxo-1,2,3,4,10,11,14,16-octahydro-13*H*-cyclohepta[3',4':6,7]indolizino[1,2-*b*]quinolin-1-yl)-2-hydroxyacetamide

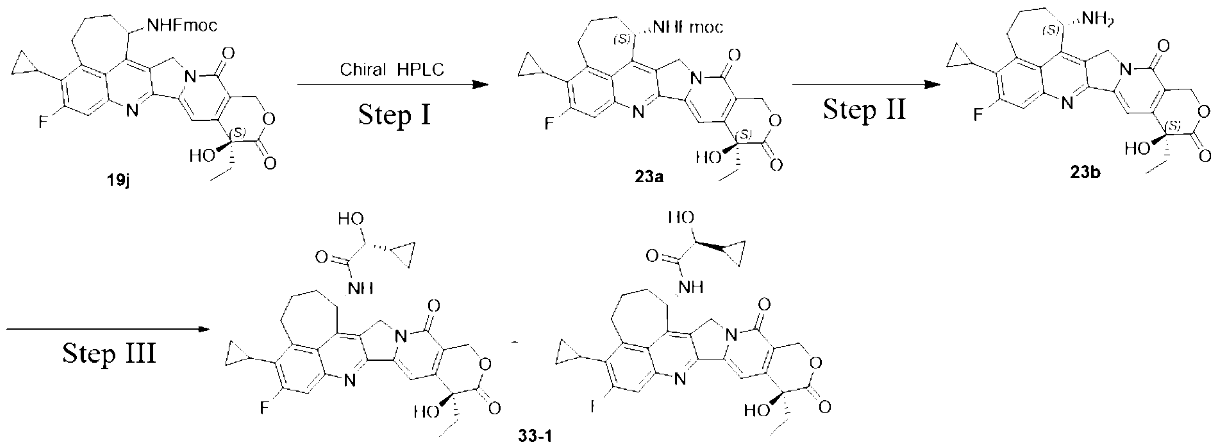


19j

23a

23b

33-1

33-2

Step I (9*H*-fluoro-9-yl)methyl((1*S*,10*S*)-5-cyclopropyl-10-ethyl-6-fluoro-10-hydroxy-11,14-dioxo-1,2,3,4,10,11,14,16-octahydro-13*H*-cyclohepta[3',4':6,7]indolizino[1,2-*b*]quinolin-1-yl)carbamate **23a**

Compound **19j** (500 mg, 0.72 mmol) was purified using a Gilson preparative instrument and a Daicel chiral column to separate chiral isomers (chromatographic column: CHIRALPAK IA 3.0 cm I.D.×25 cm,10 μm; mobile phase 1: MeOH; mobile phase 2: DCM; gradient proportion: MeOH/DCM=70/30, flow rate: 25 mL/min) to give the compound **23a** (220 mg, yield: 44%).

Step II (1*S*,10*S*)-1-amino-5-cyclopropyl-10-ethyl-6-fluoro-10-hydroxy-2,3,4,10,13,16-hexahydro-14*H*-cyclohepta [3',4':6,7]indolizine[1,2-b]quinoline-11,14-(1*H*)-dione **23b**

The compound **23a** (45 mg, 0.064 mmol) was dissolved in a mixed solvent of *N,N*-dimethylformamide and diethylamine (7 mL, V/V=5:2), and the reaction mixture was stirred at room temperature for 2 h. After the reaction was complete, the reaction mixture was directly concentrated to give the crude compound **23b** (30 mg), which was directly used in the next reaction step without purification.

MS m/z (ESI): 476.2 $(M+1)^+$,

Step III

(*R*)-2-cyclopropyl-*N*-((1*S*,10*S*)-5-cyclopropyl-10-ethyl-6-fluoro-10-hydroxy-11,14-dioxo-1,2,3,4,10,11,14,16-octahydro-13*H*-cyclohepta[3',4':6,7]indolizino[1,2-*b*]quinolin-1-yl)-2-hydroxyacetamide

(*S*)-2-cyclopropyl-*N*-((1*S*,10*S*)-5-cyclopropyl-10-ethyl-6-fluoro-10-hydroxy-11,14-dioxo-1,2,3,4,11,14,16-octahydro-13*H*-cyclohepta[3',4':6,7]indolizino[1,2-*b*]quinolin-1-yl)-2-hydroxyacetamide

The compound **23b** (20 mg, 0.042 mmol) was dissolved in *N*,*N*-dimethylacetamide (2 mL), and 2-cyclopropyl-2-hydroxyacetic acid (5.4 mg, 0.046 mmol), (benzotriazol-1-yloxy)tris(dimethylamino)phosphonium hexafluorophosphate (22 mg, 0.051 mmol), and *N,N*-diisopropylethylamine (11 mg, 0.084 mmol) were added. The reaction mixture was stirred at room temperature for 3 h. After the reaction was complete, the reaction mixture was purified by preparative high performance liquid chromatography (Waters MS-triggered Prep-LC with QDA detector, chromatographic column: WELCH Xtimate C18 21.2x250 mm 10 μm; mobile phase 1: water (containing 0.1% FA); mobile phase 2: acetonitrile; 15-min gradient, gradient proportion: acetonitrile phase 46%-100%, flow rate: 30 mL/min) to give products **33-1** (4.1 mg, yield: 17.2%) and **33-2** (1.7 mg, yield: 6.9%).

**33-1** (Monoconfigurational compound, short retention time)

MS m/z (ESI): 574.2 $(M+1)^+$.
$^1H$ NMR (400 MHz, DMSO-$d_6$) δ 8.62-8.37 (m, 2H), 7.69 (d, 1H), 7.28 (s, 1H), 6.53 (s, 1H), 5.59-5.48 (m, 2H), 5.43 (d, 2H), 5.28 (d, 1H), 3.62 (d, 1H), 3.49-3.46 (m, 1H), 2.19-1.72 (m, 6H), 1.69-1.53 (m, 1H), 1.23 (s, 1H), 1.18-1.00 (m, 3H), 0.85 (t, 3H), 0.83-0.77 (m, 1H), 0.74-0.62 (m, 1H), 0.46-0.28 (m, 4H).

**33-2** (Monoconfigurational compound, long retention time)

MS m/z (ESI): 574.2 $(M+1)^+$.
$^1H$ NMR (400 MHz, DMSO-$d_6$) δ 8.45 (d, 2H), 7.69 (d, 1H), 7.28 (s, 1H), 6.53 (s, 1H), 5.60-5.52 (m, 1H), 5.48 (s, 1H), 5.43 (d, 2H), 5.30 (d, 1H), 3.59 (d, 1H), 3.49-3.46 (m, 1H), 2.14-1.79 (m, 6H), 1.75-1.61 (m, 1H), 1.24 (s, 1H), 1.17-1.02 (m, 3H), 0.86 (t, *J*=8 Hz, 3H), 0.83-0.77 (m, 1H), 0.74-0.64 (m, 1H), 0.48-0.29 (m, 4H).

**Example 18**

(*S*)-3-cyclopropyl-*N*-((1*S*,9*S*)-4-cyclopropyl-9-ethyl-5-fluoro-9-hydroxy-10,13-dioxo-2,3,9,10,13,15-hexahydro-1*H*,12H-benzo[*d*]pyrano[3',4':6,7]indolizino[1,2-*b*]quinolin-1-yl)-3-hydroxypropanamide

(*R*)-3-cyclopropyl-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxypropanamide

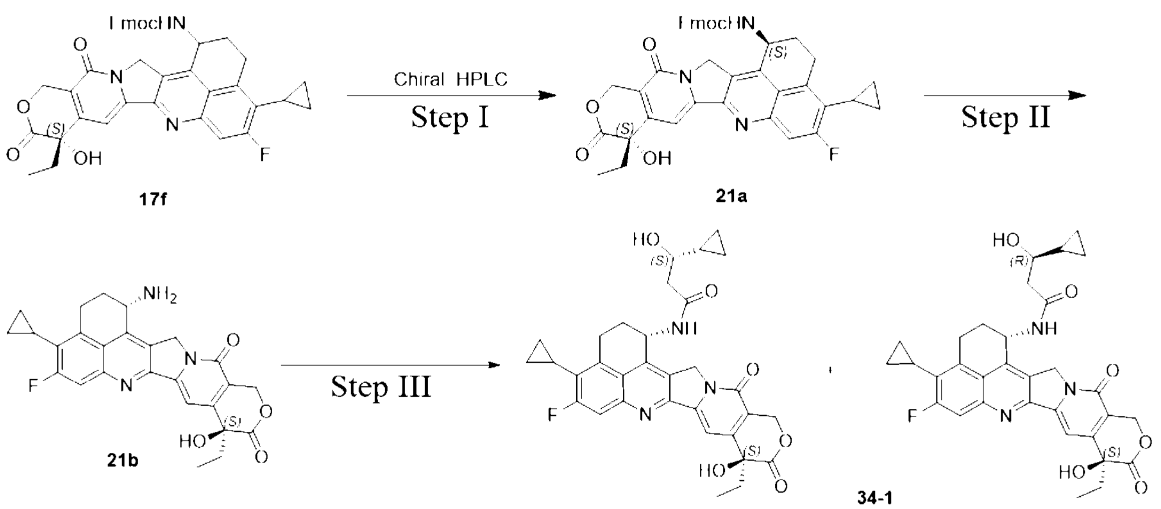


**17f** **21a** **21b** , **34-1** **34-2**

Step I (9*H*-fluoren-9-yl)methyl((1*S*,9*S*)-4-cyclopropyl-9-ethyl-5-fluoro-9-hydroxy-10,13-dioxo-2,3,9,10,13,15-hexahydro-1*H*,12*H*-benzo[d]pyrano[3',4':6,7]indolizino[1,2-*b*]quinolin-1-yl)carbamate **21a**

The compound **17f** (600 mg, 1.02 mmol) was purified using a Gilson preparative instrument and a Daicel chiral column to separate chiral isomers (chromatographic column: CHIRALPAK IB 3.0 cm I.D.×25 cm,10 μm; mobile phase 1: MeOH; mobile phase 2: DCM; gradient proportion: MeOH/DCM=90/10, flow rate: 25 mL/min) to give the compound **21a** (250 mg, yield: 42%).

Step II (1S,9S)-1-amino-4-cyclopropyl-9-ethyl-5-fluoro-9-hydroxy-1,2,3,9,12,15-hexahydro-10*H*,13*H*-benzo[*d*]pyrano[3',4':6,7]indolizino[1,2-*b*]quinoline-10,13-dione **21b**

The compound **21a** (50 mg, 0.07 mmol) was dissolved in DMF (5 mL), and diethylamine (0.5 mL) was added. The mixture was stirred for reaction at room temperature for 1 h. After the reaction was complete, the mixture was spin-dried under vacuum to remove the solvent, thus giving the title compound **21b** (34 mg), which was directly used in the next reaction step without purification.
MS m/z (ESI): 462.1 $(M+1)^+$.

Step III

(*S*)-3-cyclopropyl-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxypropanamide

(*R*)-3-cyclopropyl-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[d]pyrano[3',4':6,7]indolizino[1,2-*b*]quinolin-1-yl)-3-hydroxypropanamide

The compound **21b** (30 mg, 0.065 mmol) was dissolved in *N*,*N*-dimethylformamide (3 mL), and 3-cyclopropyl-3-hydroxypropionic acid (15 mg, 0.098 mmol), 2-(7-azabenzotriazole)-*N*,*N*,*N'*,*N'*-tramethyluronium hexafluorophosphate (37 mg, 0.096 mmol), and *N,N*-diisopropylethylamine (171 mg, 1.32 mmol) were added. The reaction mixture was stirred at room temperature for 2 h. After the reaction was complete, the mixture was purified by preparative high performance liquid chromatography (Waters MS-triggered Prep-LC with QDA detector, chromatographic column: WELCH Xtimate C18 21.2x250 mm 10 μm; mobile phase 1: water (containing 0.1% FA); mobile phase 2: acetonitrile; 15-min gradient, gradient proportion: acetonitrile phase 40%-100%, flow rate: 30 mL/min) to give products **34-1** (4.2 mg, yield: 11%) and **34-2** (3.9 mg, yield: 10%).

**34-1 (Monoconfigurational compound, compound with short retention time)**

MS m/z (ESI): 574.2 $(M+1)^+$.
$^1H$ NMR (400 MHz, DMSO-$d_6$) δ 8.53-8.39 (m, 2H), 7.75 (d, 1H), 7.30 (s, 1H), 6.53 (s, 1H), 5.55 (dd, 1H), 5.42 (s, 2H), 5.23 (s, 2H), 4.69 (s, 1H), 2.37 (d, 2H), 2.15 (d, 2H), 2.01-1.77 (m, 4H), 1.17-1.07 (m, 2H), 0.91-0.72 (m, 6H), 0.39-0.12 (m, 5H).

**34-2 (Monoconfigurational compound, compound with long retention time)**

MS m/z (ESI): 574.2 $(M+1)^+$.
$^1H$ NMR (400 MHz, DMSO-$d_6$) δ 8.45 (d, 2H), 7.75 (d, 1H), 7.31 (s, 1H), 6.53 (s, 1H), 5.64-5.54 (m, 1H), 5.43 (s, 2H), 5.24 (q, 2H), 4.65 (d, 1H), 2.33 (d, 2H), 2.27-1.73 (m, 6H), 1.12 (dd, 2H), 0.91-0.70 (m, 6H), 0.39-0.13 (m, 5H).

**Example 19**

(*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxypentanamide

(*S*)-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxypentanamide

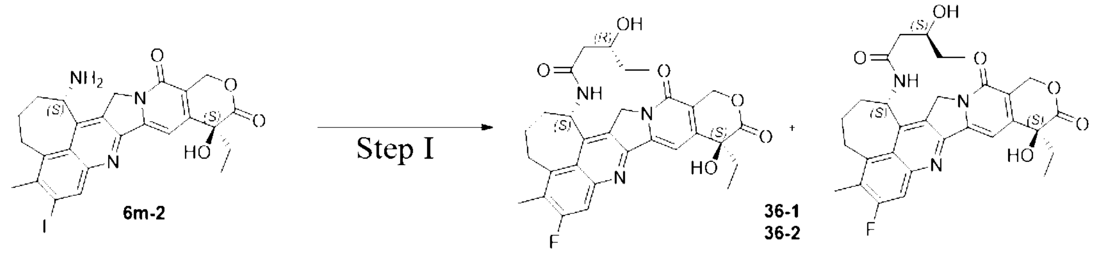


6m-2

36-1
36-2

Step I

(*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxypentanamide

(*S*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxypentanamide

The compound **6m-2** (30 mg, 0.07 mmol) was dissolved in *N*,*N*-dimethylformamide (3 mL), and then 3-hydroxypentanoic acid (9 mg, 0.07 mmol), *N*,*N*-diisopropylethylamine (17 mg, 0.13 mmol), and 2-(7-azabenzotriazole)-*N*,*N*,*N'*,*N'*-tramethyluronium hexafluorophosphate (38 mg, 0.10 mmol) were added. The reaction system was kept for reaction at normal temperature for 1 h. After the reaction was complete, the purification was performed by preparative high performance liquid chromatography (GILSON Prep LC with UV detector, chromatographic column: Xbridge 10 μm C18 250x30 mm, 10 μm; mobile phase 1: water (containing 10 mmol/LFA); mobile phase 2: acetonitrile; 15-min gradient, gradient proportion: acetonitrile phase 5%-57%, flow rate:50 mL/min) to give compounds **36-1**(6.98 mg, yield: 18%) and **36-2** (6.70 mg, yield: 18%).

**36-1 (Monoconfigurational compound, compound with short retention time)**

MS m/z (ESI): 550.2 $(M+1)^+$.
$^1H$ NMR (400 MHz, DMSO-$d_6$) δ 8.61 (d, 1H), 7.74 (d, 1H), 7.29 (s, 1H), 6.52 (s, 1H), 5.53 (s, 1H), 5.43 (s, 2H), 5.39-5.09 (m, 2H), 4.63 (s,1H), 2.43 (s, 3H), 2.32 (d, 3H), 2.10-1.93 (m, 3H), 1.92-1.81 (m, 2H), 1.75 (s, 1H), 1.40-1.32 (m, 2H), 1.24 (s, 2H), 0.91-0.76 (m, 6H).

**36-2 (Monoconfigurational compound, compound with long retention time)**

MS m/z (ESI): 550.2 $(M+1)^+$.
$^1H$ NMR (400 MHz, DMSO-$d_6$) δ 8.69 (d, 1H), 7.73 (d, 1H), 7.28 (s, 1H), 6.51 (s, 1H), 5.51 (d, 2H), 5.43 (s, 2H), 5.33 (d, 1H),4.64 (s, 1H), 2.42 (s, 4H), 2.34-2.22 (m, 4H), 2.08 (d, 1H), 2.01 (d, 1H), 1.92-1.78 (m, 2H), 1.65 (s, 1H), 1.44-1.31 (m, 2H), 1.24 (s, 1H), 0.95-0.66 (m, 6H).

**Example 20**

(*S*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxy-4-methylpentanamide

(*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxy-4-methylpentanamide

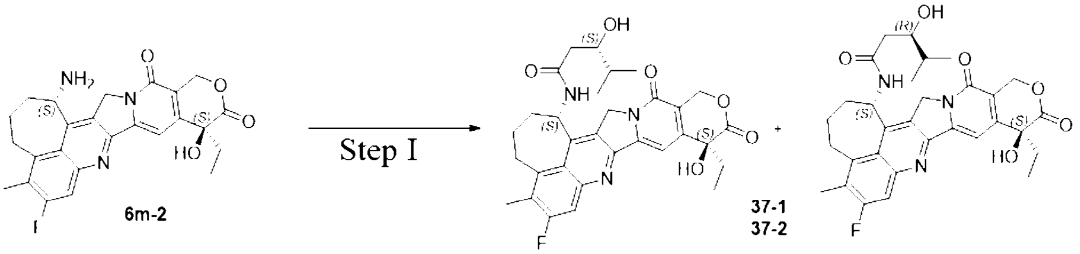


6m-2

37-1
37-2

Step I

(*S*)-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxy-4-methylpentanamide

(*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxy-4-methylpentanamide

The compound **6m-2** (30 mg, 0.07 mmol) was dissolved in *N*,*N*-dimethylformamide (3 mL), and then 3-hydroxy-4-methylpentanoic acid (9 mg, 0.07 mmol), *N*,*N*-diisopropylethylamine (17 mg, 0.13 mmol), and 2-(7-azabenzotriazole)-*N,N,N',N'*-tramethyluronium hexafluorophosphate (38 mg, 0.10 mmol) were added. The reaction system was kept for reaction at room temperature for 1 h. After the reaction was complete, the mixture was purified by preparative high performance liquid chromatography (Waters MS-triggered Prep-LC with SQD2 detector, chromatographic column: Xbridge 5 μm C18 150x19 mm; mobile phase 1: water (containing 0.1% FA); mobile phase 2: acetonitrile; 15-min gradient, gradient proportion: acetonitrile phase 42%-100%, flow rate: 20 mL/min) to give compound **37-1** (6.6 mg, yield: 18%) and **37-2** (6.9 mg, yield: 18%).

**37-1 (Monoconfigurational compound, compound with short retention time)**

MS m/z (ESI): 564.2 $(M+1)^+$.
$^1H$ NMR (400 MHz, DMSO-$d_6$) δ 8.68 (d, 1H), 8.48 (br, 1H), 7.73 (d, 1H), 7.28 (s, 1H), 6.56 (s, 1H), 5.60-5.49 (m, 1H), 5.42 (s, 2H), 5.35 (d, 1H), 5.21 (d, 1H), 4.64 (s, 1H), 2.42 (s, 3H), 2.29 (d, 3H), 2.13-1.91 (m, 3H), 1.90-1.80 (m, 2H), 1.79-1.67 (m,1H),1.60-1.47 (m, 1H), 1.23 (s, 1H), 0.87 (d, 3H), 0.82 (d, 6H).

**37-2 (Monoconfigurational compound, compound with long retention time)**

MS m/z (ESI): 564.2 $(M+1)^+$.
$^1H$ NMR (400 MHz, DMSO-$d_6$) δ 8.78 (s, 1H), 8.50 (s, 1H), 7.73 (s, 1H), 7.28 (s, 1H), 6.56 (s, 1H), 5.43 (s, 3H), 5.34 (d, 2H), 4.62 (s, 2H), 2.29 (s, 3H), 2.07 (s, 2H), 2.02-1.97 (m, 2H), 1.92-1.77 (m, 2H), 1.71-1.49 (m, 1H), 1.23 (s, 1H), 0.99-0.66 (m, 11H).

**Example 21**

(*R*)-3-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxypropanamide

(*S*)-3-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxypropanamide

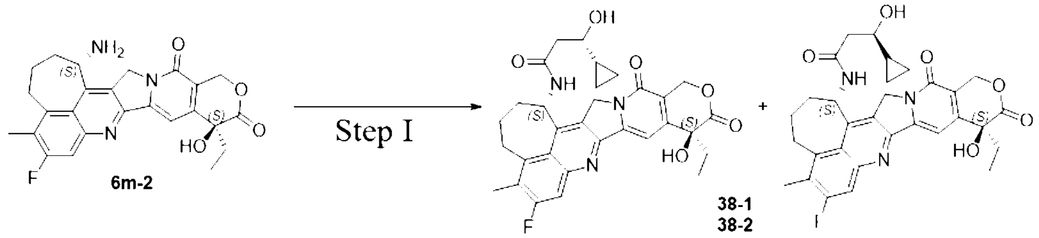


6m-2

38-1
38-2

(*R*)-3-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxypropanamide

(*S*)-3-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxypropanamide

The compound **6m-2** (30 mg, 0.07 mmol) was dissolved in *N,N*-dimethylformamide (3 mL), and then 3-cyclopropyl-3-hydroxypropionic acid (18 mg, 0.14 mmol), *N,N*-diisopropylethylamine (17 mg, 0.13 mmol), and 2-(7-azabenzotriazole)-*N,N,N',N'*-tramethyluronium hexafluorophosphate (38 mg, 0.10 mmol) were added. The reaction system was kept for reaction at normal temperature for 1 h. After the reaction was complete, the purification and separation were performed by preparative high performance liquid chromatography (Waters MS-triggered Prep-LC with QDA detector, chromatographic column: Xbridge 5 μm C18 150x19 mm; mobile phase 1: water (containing 0.1% FA); mobile phase 2: acetonitrile; 15-min gradient, gradient proportion: acetonitrile phase 45%-100%, flow rate: 25 mL/min) to give the title compound **38-1** (4 mg, yield: 11%) and the title compound **38-2** (4 mg, yield: 11%).

**38-1 (Monoconfigurational compound, compound with short retention time)**

MS m/z (ESI):562.3 $(M+1)^+$.
$^1$H NMR (400 MHz, DMSO-$d_6$) δ 8.63 (d, 1H), 7.74 (d, 1H), 7.28 (s, 1H), 6.52 (s, 1H), 5.53 (s, 1H), 5.42 (s, 4H), 4.69 (d, 1H), 3.22 (s, 2H), 2.61 (d, 1H), 2.42 (s, 3H), 2.31 (d, 1H), 2.08-1.69 (m, 6H), 0.87 (s, 3H), 0.81 (d, 1H), 0.37-0.20 (m, 3H), 0.16-0.08 (m, 1H).

**38-2 (Monoconfigurational compound, compound with long retention time)**

MS m/z (ESI):562.3 $(M+1)^+$.
$^1$H NMR (400 MHz, DMSO-$d_6$) δ 8.71 (d, 1H), 7.73 (d, 1H), 7.27 (s, 1H), 6.51 (s, 1H), 5.43 (s, 5H), 4.64 (d, 1H), 4.55-4.51(m, 1H), 3.22 (s, 2H), 2.65 (d, 1H), 2.41 (s, 3H), 2.31 (d, 1H), 2.13-1.57 (m, 6H), 0.88 (s, 3H), 0.82 (s, 1H), 0.45 (s, 1H), 0.32-0.13 (m, 3H).

**Example 22**

(2*S*,3*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxy-2-methylbutanamide 39

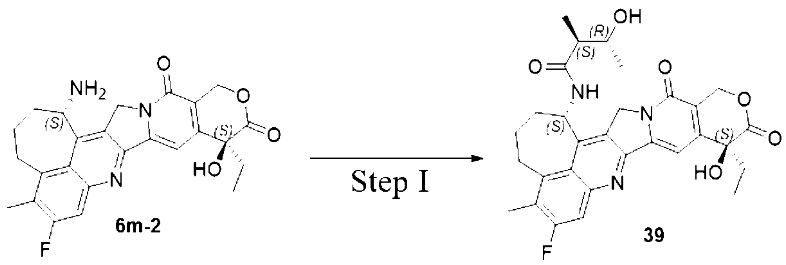


6m-2

39

Step I

(2*S*,3*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxy-2-methylbutanamide **39**

The compound **6m-2** (30 mg, 0.07 mmol) was dissolved in *N,N*-dimethylformamide (2 mL), and (2S,3R)-3-hydroxy-2-methylbutyric acid (12 mg, 0.10 mmol) and 2-(7-azabenzotriazole)-*N,N,N',N'*-tramethyluronium hexafluorophosphate (38 mg, 0.10 mmol) were added. The mixture was stirred for reaction at room temperature for 15 min. After the reaction was complete, the purification was performed by preparative high performance liquid chromatography (Waters MS-triggered Prep-LC with QDA detector, chromatographic column: Xbridge 5 μm C18 150x19 mm, 5 μm; mobile phase 1: water (containing 0.1% FA); mobile phase 2: acetonitrile; 10-min gradient, gradient proportion: acetonitrile phase 42%-52%, flow rate: 25 mL/min) to give the compound **39** (2 mg, yield: 5%).

MS m/z (ESI): 550.0 $(M+1)^+$.

$^1H$ NMR (400 MHz, $CDCl_3$) δ 7.73 (s, 1H), 7.42-7.28 (m, 2H), 5.51 (s, 1H), 5.41 (d, 1H), 5.35 (t, 1H), 5.26-5.16 (m, 2H), 5.04 (d, 1H), 4.18-4.00 (m, 2H), 3.33 (s, 2H), 2.65-2.50 (m, 3H), 2.48 (s, 3H), 2.27-2.18 (m, 1H), 2.11-2.04 (m, 2H), 2.03-1.97 (m, 1H), 1.33-1.28 (m, 6H), 0.95 (t, 3H).

## Biological Evaluation

The present invention is further described and interpreted below with reference to the test examples, but these test examples are not intended to limit the scope of the present invention.

**Test Example 1** Experiment of inhibition of compounds on proliferation of cells SK-BR-3

1.1 Experimental materials

| Materials and reagents | Suppliers | Article numbers |
|---|---|---|
| SK-BR-3 | ATCC | HTB-30 |
| 5A Medium | Gibco | 16600-082 |
| Penicillin-streptomycin | Gibco | 15140-122 |
| Fetal bovine serum | Gibco | 10099-141C |
| Phosphate buffer saline | Gibco | 10010-031 |
| TrypLE | Gibco | 12604-021 |
| DMSO | Sigma | D8418-1L |
| Staurosporine | MCE | HY-15141 |
| CelltiterGlo assay kit (CTG) | Promega | G7573 |

1.2 Experimental instruments:

| Consumables and instruments | Suppliers | Article numbers |
|---|---|---|
| 384-well white culture plate | PerkinElmer | 6007680 |
| Platform rocker | QILINBEIER | QB-9002 |
| Centrifuge | Eppendorf | 5810R |
| Carbon dioxide incubator | Thermo Scientific | 371 |
| Microscope | OLYMPUS | CKX41 |
| Echo Qualified 384-Well Polypropylene | LABCYTE | PP-0200 |
| Echo | LABCYTE | 655 |

(continued)

| Consumables and instruments | Suppliers | Article numbers |
|---|---|---|
| CountessII | Gibco | AMQAX1000 |
| Envision | PerkinElmer | 2105 |

1.3 Test method

(1) Cell plating: first, tumor cells SK-BR-3 were cultured using a corresponding culture medium, trypsinized, centrifuged, resuspended for counting, adjusted to an appropriate concentration, and then plated on a 384-well plate.
(2) Co-incubation of the compounds and tumor cells: after the cells adhered to the wall, 100 nL of a diluted bioactive substance (test compound) was added to the cell culture plate using ECHO, the final concentration of DMSO in wells of the cell plate was 0.33%, and the incubation was performed in an incubator at 37°C with 5% $CO_2$ for 72 h.
(3) After the incubation was complete, 30 μL of a CTG reagent (CelltiterGlo kit) was added to each well. The culture was oscillated on a fast oscillator for 2 min, centrifuged at 1,000 rpm for 1 min, and kept away from light at room temperature for 30 min. The chemiluminescence signal value was read using an Envision instrument.
(4) Cell viability detection: $IC_{50}$ was calculated using GraphPad Prism 8 software, and the $IC_{50}$ of the compounds was obtained using the following nonlinear fitting formula (see Table 1) :

$$Y=Bottom+(Top-Bottom)/(1+10^{\wedge}((LogIC_{50}-X)*HillSlope))$$

Y: inhibition rate; X: log value of compound concentration;

inhibition rate (%)=100-(compound well reading - low-reading control well reading)/(high-reading control well reading - low-reading control well reading)* 100;

high-reading control wells: cells with addition of 100 nL DMSO; and
low-reading control wells: cell-free wells.

Table 1 Antiproliferative activity of test compounds on SK-BR-3

| **Example and Compound No.** | **$IC_{50}$ (nM)** |
|---|---|
| Control compound (Dxd) | 11 |
| **3-1** | 13 |
| **3-2** | 15 |
| **4-1** | 61 |
| **4-2** | 14 |
| **5-1** | 20 |
| **5-2** | 20 |
| **6-1** | 11 |
| **6-2** | 7.3 |
| **7-1** | 8.6 |
| **7-2** | 7.6 |
| **8-1** | 44 |
| **8-2** | 87 |
| **10** | 10 |
| **11-1** | 2.2 |

(continued)

| Example and Compound No. | $IC_{50}$ (nM) |
|---|---|
| **11-2** | 2.4 |
| **12** | 5.7 |
| **17-1** | 4.6 |
| **17-2** | 6.6 |
| **18-1** | 13.8 |
| **18-2** | 9.6 |
| **19-1** | 8.3 |
| **19-2** | 7.6 |
| **20-1** | 21 |
| **20-2** | 25 |
| **33-1** | 7.8 |
| **33-2** | 8.4 |
| **34-1** | 5.1 |
| **34-2** | 4.8 |
| **36-1** | 9.2 |
| **36-2** | 4.4 |
| **37-1** | 19 |
| **37-2** | 6.6 |
| **38-1** | 12 |
| **38-2** | 5.4 |
| **39** | 11 |

The result shows that the compounds of the present invention have strong antiproliferative effects on the proliferation of cells SK-BR-3.

**Test Example 2** Experiment of inhibition of test compounds on proliferation of cells MDA-MB-468

2.1 Experimental materials

| Materials and reagents | Suppliers | Article numbers |
|---|---|---|
| MDA-MB-468 | ATCC | HTB-132 |
| L-15 Medium | Hyclone | SH30525.01 |
| Penicillin-streptomycin | Gibco | 15140-122 |
| Fetal bovine serum | Gibco | 10099-141C |
| Phosphate buffer saline | Gibco | 10010-031 |
| TrypLE | Gibco | 12604-021 |
| DMSO | Sigma | D8418-1L |
| Staurosporine | MCE | HY-15141 |
| CelltiterGlo assay kit (CTG) | Promega | G7573 |

2.2 Experimental instruments:

| Consumables and instruments | Suppliers | Article numbers |
|---|---|---|
| 384-well white culture plate | PerkinElmer | 6007680 |
| Platform rocker | QILINBEIER | QB-9002 |
| Centrifuge | Eppendorf | 5810R |
| Carbon dioxide incubator | Thermo Scientific | 371 |
| Microscope | OLYMPUS | CKX41 |
| Echo Qualified 384-Well Polypropylene | LABCYTE | PP-0200 |
| Echo | LABCYTE | 655 |
| CountessII | Gibco | AMQAX1000 |
| Envision | PerkinElmer | 2105 |

2.3 Test method

(1) Cell plating: first, tumor cells MDA-MB-468 were cultured using a corresponding culture medium, trypsinized, centrifuged, resuspended for counting, adjusted to an appropriate concentration, and then plated on a 384-well plate.
(2) Co-incubation of the compounds and tumor cells: after the cells adhered to the wall, 100 nL of a diluted bioactive substance (test compound) was added to the cell culture plate using ECHO, the final concentration of DMSO in wells of the cell plate was 0.33%, and the incubation was performed in an incubator at 37°C with 0% $CO_2$ for 72 h.
(3) After the incubation was complete, 30 μL of a CTG reagent (CelltiterGlo kit) was added to each well. The culture was oscillated on a fast oscillator for 2 min, centrifuged at 1,000 rpm for 1 min, and kept away from light at room temperature for 30 min. The chemiluminescence signal value was read using an Envision instrument.
(4) Cell viability detection: $IC_{50}$ was calculated using GraphPad Prism 8 software, and the $IC_{50}$ of the compounds was obtained using the following nonlinear fitting formula (see Table 2) :

$$Y=Bottom+(Top-Bottom)/(1+10^{\wedge}((LogIC_{50}-X)*HillSlope))$$

Y: inhibition rate; X: log value of compound concentration;

inhibition rate (%)=100-(compound well reading - low-reading control well reading)/(high-reading control well reading - low-reading control well reading)* 100;

high-reading control wells: cells with addition of 100 nL DMSO; and
low-reading control wells: cell-free wells.

Table 2 Antiproliferative activity of test compounds on MDA-MB-468

| **Example and Compound No.** | **$IC_{50}$ (nM)** |
|---|---|
| Control compound (Dxd) | 4.6 |
| **3-1** | 7.5 |
| **3-2** | 12 |
| **4-1** | 34 |
| **4-2** | 13 |
| **5-1** | 6.7 |

(continued)

| Example and Compound No. | $IC_{50}$ (nM) |
|---|---|
| **5-2** | 9.4 |
| **6-1** | 8.8 |
| **6-2** | 6.5 |
| **7-1** | 3.1 |
| **7-2** | 4.4 |
| **8-1** | 13 |
| **8-2** | 38 |
| **10** | 13 |
| **11-1** | 3.9 |
| **11-2** | 4.5 |
| **12** | 9.3 |
| **17-1** | 5.8 |
| **17-2** | 8.1 |
| **18-1** | 12 |
| **18-2** | 12 |
| **19-1** | 7.1 |
| **19-2** | 6.9 |
| **20-1** | 15.6 |
| **20-2** | 19.5 |
| **33-1** | 6 |
| **33-2** | 5 |
| **34-1** | 4 |
| **34-2** | 4 |
| **36-1** | 14 |
| **36-2** | 6.4 |
| **37-1** | 19 |
| **37-2** | 6.6 |
| **38-1** | 13 |
| **38-2** | 5.7 |
| **39** | 15 |

The result shows that the test compounds have strong antiproliferative effects on the proliferation of cells MDA-MB-468.

**Test Example 3** Experiment of inhibition of test compounds on proliferation of cells NCI-N87

3.1 Experimental materials

| Materials and reagents | Suppliers | Article numbers |
|---|---|---|
| NCI-N87 | ATCC | HTB-30 |

(continued)

| Materials and reagents | Suppliers | Article numbers |
|---|---|---|
| RPMI-1640 Medium | Gibco | A10491-01 |
| Penicillin-streptomycin | Gibco | 15140-122 |
| Fetal bovine serum | Gibco | 10099-141C |
| Phosphate buffer saline | Gibco | 10010-031 |
| TrypLE | Gibco | 12604-021 |
| DMSO | Sigma | D8418-1L |
| Staurosporine | MCE | HY-15141 |
| CelltiterGlo assay kit (CTG) | Promega | G7573 |

3.2 Experimental instruments:

| Consumables and instruments | Suppliers | Article numbers |
|---|---|---|
| 384-well white culture plate | PerkinElmer | 6007680 |
| Platform rocker | QILINBEIER | QB-9002 |
| Centrifuge | Eppendorf | 5810R |
| Carbon dioxide incubator | Thermo Scientific | 371 |
| Microscope | OLYMPUS | CKX41 |
| Echo Qualified 384-Well Polypropylene | LABCYTE | PP-0200 |
| Echo | LABCYTE | 655 |
| CountessII | Gibco | AMQAX1000 |
| Envision | PerkinElmer | 2105 |

3.3 Test method

(1) Cell plating: first, tumor cells NCI-N87 were cultured using a corresponding culture medium, trypsinized, centrifuged, resuspended for counting, adjusted to an appropriate concentration, and then plated on a 384-well plate.
(2) Co-incubation of the compounds and tumor cells: after the cells adhered to the wall, 100 nL of a diluted bioactive substance (test compound) was added to the cell culture plate using ECHO, the final concentration of DMSO in wells of the cell plate was 0.33%, and the incubation was performed in an incubator at 37°C with 0% $CO_2$ for 72 h.
(3) After the incubation was complete, 30 μL of a CTG reagent (CelltiterGlo kit) was added to each well. The culture was oscillated on a fast oscillator for 2 min, centrifuged at 1,000 rpm for 1 min, and kept away from light at room temperature for 30 min. The chemiluminescence signal value was read using an Envision instrument.
(4) Cell viability detection: $IC_{50}$ was calculated using GraphPad Prism 8 software, and the $IC_{50}$ of the compounds was obtained using the following nonlinear fitting formula (see Table 3) :

$$Y=Bottom+(Top-Bottom)/(1+10^{\wedge}((LogIC_{50}-X)*HillSlope))$$

Y: inhibition rate; X: log value of compound concentration;

inhibition rate (%)=100-(compound well reading - low-reading control well reading)/(high-reading control well reading - low-reading control well reading)* 100;

high-reading control wells: cells with addition of 100 nL DMSO; and
low-reading control wells: cell-free wells.

Table 3 Antiproliferative activity of test compounds on cells NCI-N87

| **Example and Compound No.** | **$IC_{50}$ (nM)** |
|---|---|
| Control compound (Dxd) | 11 |
| **3-1** | 16 |
| **3-2** | 16 |
| **4-2** | 22 |
| **5-1** | 14 |
| **5-2** | 18 |
| **6-1** | 7.5 |
| **6-2** | 6.8 |
| **7-2** | 4.9 |
| **8-1** | 4.2 |
| **9** | 3 |
| **10** | 23 |
| **11-1** | 2.5 |
| **11-2** | 2.3 |
| **12** | 20 |
| **17-1** | 3.6 |
| **17-2** | 5.3 |
| 18-1 | 11.2 |
| **18-2** | 16.5 |
| **19-1** | 7.4 |
| **19-2** | 7.4 |
| **20-1** | 26 |
| **20-2** | 28 |
| **33-1** | 8 |
| **33-2** | 9 |
| **34-1** | 6 |
| **34-2** | 6 |
| **36-1** | 8.5 |
| **36-2** | 3.9 |
| **37-1** | 8.6 |
| **37-2** | 3.2 |
| **38-1** | 7.5 |
| **38-2** | 2.9 |
| **39** | 9.3 |

The result shows that the test compounds have strong antiproliferative effects on the proliferation of cells NCI-N87.

**Test Example** 4 Kinetic solubility test in PBS 7.4

4.1. Experimental steps

1) Preparation of stock solutions

10 mM stock solutions of the test substances **6-2, 11-2, 17-2, Dxd,** and the control drugs progesterone and diclofenac were prepared using DMSO respectively.

2) Kinetic solubility test steps

15 μL of 10 mM stock solution of each of the test substances was added to a corresponding position of a corresponding 96-well plate in a specified sequence. 485 μL of the PBS 7.4 was added to a corresponding vial on a sample plate. The experiment was run in duplicate. Each vial was additionally provided with a stirring rod and was plugged. Then, the sample plate was transferred in a thermostatic mixer, and oscillated at a revolving speed of 1,100 rpm at 25 °C for 2 h. 2 h later, the plug was removed, the stirring rod was attracted away with a large magnet, and then the sample was transferred from the sample plate to the filter plate. Negative pressure was generated using a vacuum pump to filter the sample. 5 μL of filtrate and 5 μL of blank DMSO were transferred to another sample plate, and then 490 μL of internal standard water (acetonitrile:water=1:1) containing the internal standard was added. Depending on peak shapes, a certain proportion of internal standard water may be used to dilute the sample diluent to obtain better peak shapes.

3) Preparation of 3 μM of standard substance solution

6 μL was transferred from the 10 mM DMSO stock solution plate to a blank plate, and was prepared into a 300 μM standard substance solution with addition of 194 μL of DMSO. 5 μL was transferred from the 300 μM standard substance solution plate to still another blank plate, and then 5 μL of blank buffer and 490 μL of the internal standard water containing the internal standard (acetonitrile:water= 1:1) were added until a final concentration of the standard substance solution was 3 μM.

4) Sample analysis steps

An injection plate was transferred on an injection tray of an autosampler to evaluate the sample by LC/MS analysis.

4.0. Data analysis

All calculations were performed using Microsoft Excel.
The sample filtrate was analyzed and quantified by qualitative and quantitative LC/MS of standard substance peaks at known concentrations. The calculation formula for the solubility values of the reference drugs and the test substances are as follows:

$$[Sample] = \frac{AREA_{Sample} \times DF_{Sample} \times [STD]}{AREA_{Std}}$$

[Sample] is the compound concentration, $DF_{Sample}$ refers to the sample dilution factor, [STD] is the standard substance concentration of the compound, $AREA_{Std}$ is the standard substance peak area of the compound, and $AREA_{Sample}$ is the peak area of the compound sample.

4.2 Experimental result

The experimental result is as shown in Table 4.

Table 4 Solubility of representative compounds in PBS 7.4

| Compound | **6-2** | **11-2** | **17-2** | **Dxd** |
|---|---|---|---|---|
| Solubility in PBS 7.4 (μM) | 170 | 34 | 1.7 | 11.3 |

The result shows that the representative compounds **6-2** and **11-2** have significantly better solubility in the PBS 7.4 than Dxd.

**Test Example 5** Pharmacokinetic Test in SD Rats

After a single intravenous injection (dose: 2 mpk) of the compounds **6-2, 11-2, 17-2,** and **Dxd** into male SD rats (blood collection time points: 0.083 h, 0.25 h, 0.5 h, 1 h, 2 h, 4 h, 8 h, and 24 h after IV administration, totaling 8 blood collection time points), average pharmacokinetic parameters in the plasma are as shown in Table 5:

Table 5 Pharmacokinetic test result of representative compounds

| Compound | $C_0$ (ng/mL) | $AUC_{last}$ (h*ng/mL) | $AUC_{inf}$ (h*ng/mL) | $T_{1/2}$ (h) | CL (mL/min/kg) | $V_{ss}$ (L/kg) |
|---|---|---|---|---|---|---|
| **6-2** | 677 | 314 | 318 | 0.39 | 107.9 | 2.93 |
| **11-2** | 848 | 460 | 465 | 0.63 | 72.7 | 3.01 |
| **17-2** | 2693 | 2253 | 2734 | 1.64 | 12.32 | 1.4 |
| **Dxd** | 748 | 301 | 302 | 0.39 | 113 | 2.70 |

The result shows that the representative compounds of the present application are rapidly removed from the rats, have good safety, and have good pharmacokinetic properties.

**Test Example 6** Experiment of inhibition of compounds on human hepatomicrosomes CYP1A2, CYP2D6, and CYP3A4

6.1 Positive control inhibitors of each subtype

| CYP | Inhibitor | Final concentration (μM) |
|---|---|---|
| 1A2 | Furafylline | 0, 0.1, 0.3, 1, 3, 10, 30 |
| 2D6 | Quinidine | 0, 0.0015, 0.005, 0.015, 0.05, 0.15, 0.5 |
| 3A4 | Ketoconazole | 0, 0.0015, 0.005, 0.015, 0.05, 0.15, 0.5 |

6.2 Preparation of substrate stock solutions

| CYP | Labeled substrate | Working concentration (μM) | Final substrate concentration (μM) | Incubation time (min) |
|---|---|---|---|---|
| 1A2 | Phenacetin | 800 (100mM PBS) | 40 | 20 |
| 2D6 | Dextromethorphan | 40 (100mM PBS) | 2 | 20 |
| 3A4M | Midazolam | 20 (100mM PBS) | 1 | 5 |
| 3A4T | Testosterone | 80 (100mM PBS) | 40 | 10 |

6.3 Experimental process

The incubation was performed in a 96-well deep well plate. The following volumes were added to each well of the incubation plate: 169 μL of "master solution" and 1 μL of each of the test compounds or positive control compound (DMSO) at various concentrations. The incubation plate was transferred to a water bath for pre-incubation at 37°C for 5 min. Then, 10 μL of diluted substrate solution was added to the incubation plate, and mixed and incubated on a vortex mixer for 15 sec. Then, 20 μL of 10 mM NADPH solution was added, and the reaction started from the final concentration of 1 mM. At a predetermined time point, 300 μL of a quenching solution (cold acetonitrile with addition of 3% formic acid, 200 nM

alprazolam, 200 nM labetalol hydrochloride, and 200 nM tolbutamide) was added to quench the reaction. The mixture was centrifuged at 3,220 g for 40 min. 150 μL of the supernatant was transferred to another plate. The supernatant can be diluted with 150 μL of pure water. The mixture was fully mixed, and the content of substrate metabolites was determined by LC/MS/MS.

6.4 Data analysis

Automatic peak integration domains were checked for all samples. The peak areas and internal standard peak areas were analyzed, and then exported to an excel spreadsheet. Inhibition of each enzyme P450 in human hepatomicrosomes was measured as the percentage decrease of marker metabolite-forming activity, as compared to non-inhibited controls (=100% activity). The $IC_{50}$ values were calculated as the logarithm of residual activity (%) and inhibitor concentration.

The percentage of the residual activity was calculated as follows:

$$\text{Area ratio}=\text{analyte peak area}/\text{internal standard peak area}$$

$$\text{Residual activity (\%)}=\text{area ratio}_{\text{test drug}}/\text{area ratio}_{\text{blank control}}*100\%$$

The $IC_{50}$ value was calculated using Excel XLfit 5.5.1.3.

The experimental result is as shown in Table 6.

Table 6 Experimental result of inhibition of representative compounds on human hepatomicrosomes

| Compound | 1A2 ($IC_{50}$ μM) | 2D6 ($IC_{50}$ μM) | 3A4T ($IC_{50}$ μM) | 3A4M ($IC_{50}$ μM) |
|---|---|---|---|---|
| **6-2** | >30 | >30 | >30 | >30 |
| **11-2** | >30 | >30 | >30 | >30 |
| **17-2** | >30 | >30 | >30 | >30 |
| **Dxd** | >30 | >30 | >30 | >30 |

The result shows that the representative compounds of the present application have weak inhibitory effect on CYP enzymes and have good safety.

**Test Example 7** Test of stability of compounds in human plasma

7.1 Experimental process

(1) 199 μL of human plasma was added to the culture plate of each cell. The culture plate was preheated to 37°C, and kept at this temperature for 15 min.
(2) After preincubation, 1 μL of 1 mmol/L each test compound and 1 μL of 1 mmol/L control compound were added to 199 μL of plasma, so that a final concentration of each test compound was 5 μmol/L and that of the control compound was 5 μmol/L. The final concentration of the organic solvent was 0.5%. The experiment would be duplicated twice.
(3) Reaction samples were incubated at 37°C.
(4) The reaction was stopped by adding 600 μL of cold methanol containing the internal standard at 0, 1, 2, 6, and 24 h. Each sample was vortex mixed for 10 min, and then centrifuged at 3,220 g for 30 min to precipitate the protein. 100 μL of the supernatant was transferred to another plate. Based on the signal response and peak shapes of liquid chromatography-mass spectrometry (LC-MS), the supernatant would be diluted with ultrapure water.

7.2 Data analysis

All calculations were performed using Microsoft Excel. The peak area ratio was determined from the chromatogram of extracted ions. The percentage of compounds remaining at each time point was calculated as per the following formula:

$$\text{Residual percentage}_{tmin}(\%)=\text{peak area ratio}_{tmin}/\text{peak area}_{t0}\times 100$$

The experimental result is as shown in Table 7.

Table 7 Test result of stability of representative compounds in human plasma

| Compound | Residual percentage (%) | | | | | |
|---|---|---|---|---|---|---|
| | 0 h | 1 h | 2h | 4h | 6h | 24 h |
| 6-2 | 100.00 | 96.62 | 86.16 | 93.65 | 88.99 | 87.01 |
| 11-2 | 100.00 | 96.80 | 92.06 | 93.40 | 94.45 | 88.81 |
| 17-2 | 100.00 | 90.07 | 89.34 | 93.75 | 97.06 | 102.21 |
| Dxd | 100.00 | 89.43 | 86.80 | 83.58 | 86.42 | 82.33 |

The result shows that the representative compounds 6-2, 11-2, and 17-2 of the present application have good stability in human plasma.

The embodiments of the technical solutions of the present invention are illustratively described above. It is to be understood that the scope of protection of the present invention is not limited to the above-mentioned embodiments. Any modification, equivalent replacement, improvement, or the like made by those skilled in the art within the spirit and principle of the present invention should be encompassed within the scope of protection of the claims of the present application.

## Claims

**1.** A compound represented by formula I, a racemate, a stereoisomer, a tautomer, an isotopically labeled compound, a solvate, a polymorph, a pharmaceutically acceptable salt, or a prodrug compound thereof:

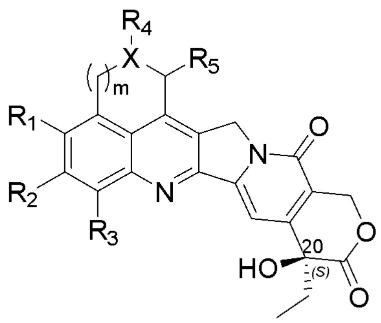

I

wherein $R_1$, $R_2$, and $R_3$ are identical or different, and are selected independently of each other from H, OH, CN, a halogen, a $C_{1-10}$ alkyl, a $C_{2-10}$ alkenyl, a $C_{2-10}$ alkynyl, a $C_{1-10}$ alkoxy, a $C_{1-10}$ haloalkyl, a $C_{1-10}$ haloalkoxy, a $C_{1-10}$ cyanoalkyl, a $C_{1-10}$ cyanoalkoxy, or a $C_{3-10}$ cycloalkyl;

$R_4$ is selected from H or

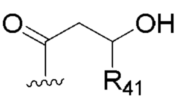

;

$R_{41}$ is selected from H, a $C_{1-6}$ alkyl, a $C_{1-6}$ haloalkyl, a $C_{1-6}$ alkyl-NH-, a $(C_{1-6}$ alkyl$)_2$N-, a $C_{1-6}$ alkyl-NH-$C_{1-6}$ alkyl, a $(C_{1-6}$ alkyl$)_2$N-$C_{1-6}$ alkyl, a $C_{1-6}$ alkoxyalkyl, a $C_{2-6}$ alkenyl, a $C_{2-6}$ alkynyl, a $C_{3-6}$ cycloalkyl, a 3-6-membered heterocyclyl, a $C_{6-14}$ aryl, and a 5-14-membered heteroaryl;

$R_5$ is selected from H,

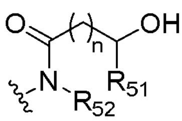, 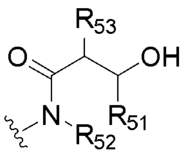, or 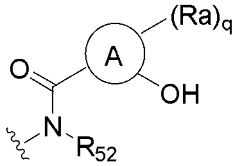;

$R_{51}$ and $R_{52}$ are identical or different, and are selected independently of each other from H, a $C_{1-6}$ alkyl, a $C_{1-6}$ haloalkyl, a $C_{1-6}$ alkyl-NH-, a $(C_{1-6}\text{ alkyl})_2N$-, a $C_{1-6}$ alkyl-NH-$C_{1-6}$ alkyl, a $(C_{1-6}\text{ alkyl})_2N$-$C_{1-6}$ alkyl, a $C_{1-6}$ alkoxyalkyl, a $C_{2-6}$ alkenyl, a $C_{2-6}$ alkynyl, a $C_{3-6}$ cycloalkyl, a 3-6-membered heterocyclyl, a $C_{6-14}$ aryl, and a 5-14-membered heteroaryl; $R_{53}$ is selected from a $C_{1-6}$ alkyl, a $C_{1-6}$ haloalkyl, a $C_{1-6}$ alkyl-NH-, a $(C_{1-6}\text{ alkyl})_2N$-, a $C_{1-6}$ alkyl-NH-$C_{1-6}$ alkyl, a $(C_{1-6}\text{ alkyl})_2N$-$C_{1-6}$ alkyl, a $C_{1-6}$ alkoxyalkyl, a $C_{2-6}$ alkenyl, a $C_{2-6}$ alkynyl, a $C_{3-6}$ cycloalkyl, a 3-6-membered heterocyclyl, a $C_{6-14}$ aryl, and a 5-14-membered heteroaryl; ring A is selected from a $C_{3-8}$ cycloalkyl or a $C_{3-8}$ heterocyclyl, Ra is selected from H, hydroxyl, CN, a halogen, a $C_{1-6}$ alkyl, or a $C_{1-6}$ haloalkyl; n is selected from 0, 1, or 2; and q is selected from 0, 1, or 2;

X is selected from CH or N; and

m is an integer selected from 0-6.

**2.** The compound according to claim 1, wherein the $R_1$ is selected from H, OH, CN, a halogen, a $C_{1-6}$ alkyl, a $C_{2-6}$ alkenyl, a $C_{2-6}$ alkynyl, a $C_{3-6}$ cycloalkyl, or a $C_{1-6}$ haloalkoxy;

preferably, the $R_1$ is selected from H, OH, CN, a halogen, a $C_{1-6}$ alkyl, a $C_{2-6}$ alkenyl, a $C_{2-6}$ alkynyl, or a $C_{1-6}$ haloalkoxy;
preferably, the $R_1$ is selected from H, OH, Br, methyl, difluoromethoxy, 2,2,2-trifluoroethoxy, ethenyl, cyclopropyl, or ethynyl;
preferably, the $R_1$ is selected from H, OH, Br, methyl, difluoromethoxy, 2,2,2-trifluoroethoxy, ethenyl, or ethynyl;
preferably, the $R_2$ is selected from H, a halogen, CN, or a $C_{1-6}$ alkyl;
preferably, the $R_2$ is selected from H or F;
preferably, the $R_3$ is selected from H or a $C_{1-6}$ alkyl; and
preferably, the $R_3$ is H.

**3.** The compound according to claim 1 or 2, wherein the $R_4$ is selected from H or

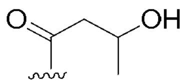

; for example,

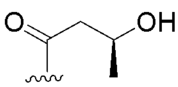 or 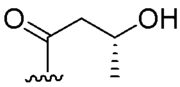 ;

preferably, the $R_4$ is selected from H or

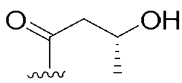 ;

preferably, the X-$R_4$ is

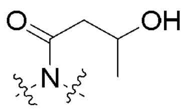 ,

and is preferably

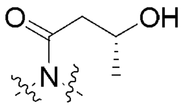 ;

and

preferably, the X-$R_4$ is $-CH_2-$.

**4.** The compound according to any one of claims 1 to 3, wherein the $R_5$ is selected from H,

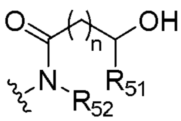 , 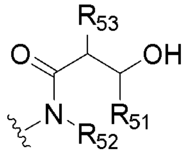 , or 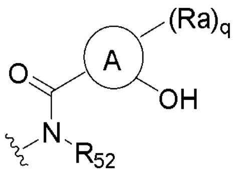 ;

wherein the $R_{51}$ is selected from H, methyl, ethyl, isopropyl, or cyclopropyl; the $R_{52}$ is selected from H or methyl; the $R_{53}$ is selected from methyl; the ring A is selected from a $C_{3-6}$ cycloalkyl; the Ra is selected from H, hydroxyl, CN, a halogen, a $C_{1-6}$ alkyl, or a $C_{1-6}$ haloalkyl; the n is selected from 0 or 1; and the q is selected from 0 or 1;

preferably, the ring A is selected from a cyclobutane ring;

preferably, the $R_5$ is selected from H,

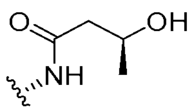 , 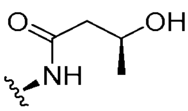 , 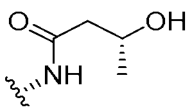 ,

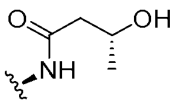 , 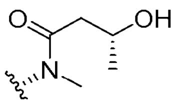 , 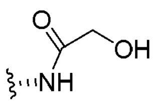 , 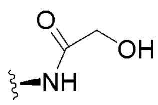 , 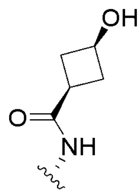 , 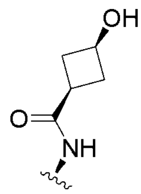 ,

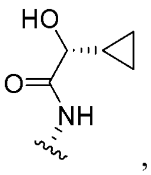 , 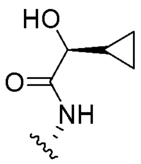 , 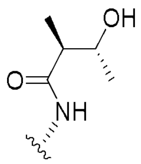 , 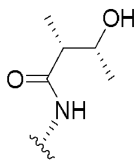 , 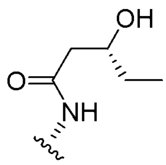 , 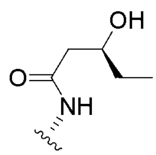 , 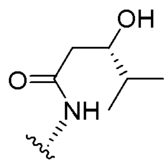 ,

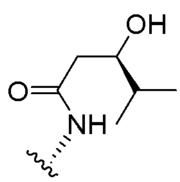 , 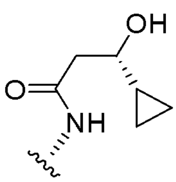 , or 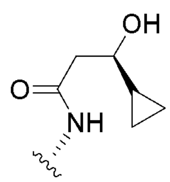 ,

preferably, the $R_5$ is selected from H,

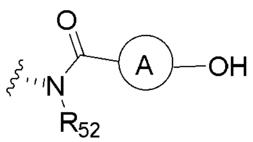 , 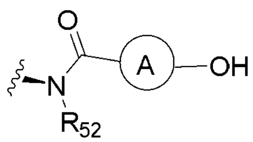 , 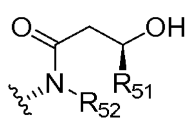

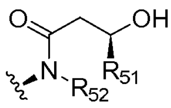, 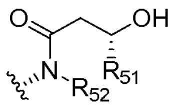, 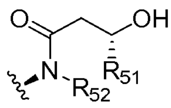, 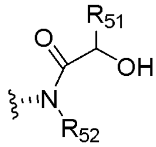, 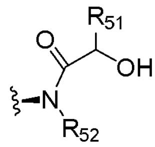, 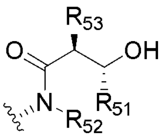, or

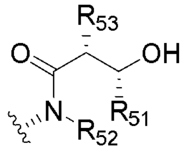;

preferably, the $R_{51}$ is selected from H, methyl, ethyl, isopropyl, or cyclopropyl; the $R_{52}$ is selected from H or methyl; the $R_{53}$ is selected from methyl; and the ring A is selected from a cyclobutane ring; and preferably, the $R_5$ is selected from H,

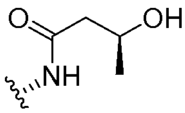, 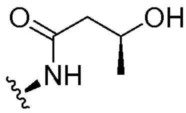, 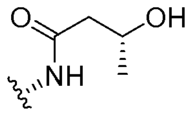,

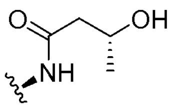, 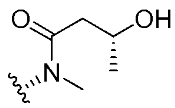, 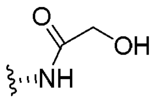, 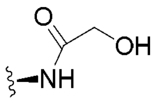, 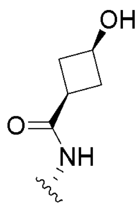, 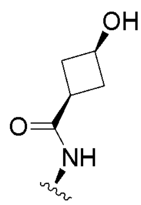,

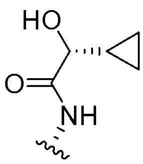, 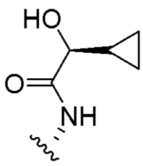, 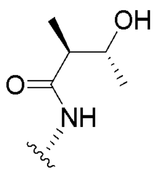, 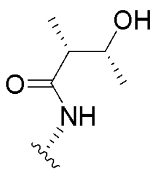, 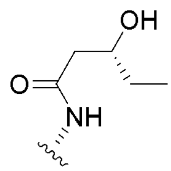, 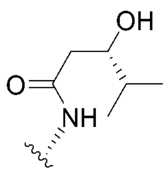,

or

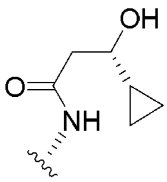.

**5.** The compound according to any one of claims 1 to 4, wherein the X is selected from CH or N; and when X is CH, the $R_4$ is H; or when X is N, the $R_5$ is H; and
preferably, the m is selected from 0, 1, or 2.

**6.** The compound according to any one of claims 1 to 5, wherein the compound represented by formula I has a structure as shown below:

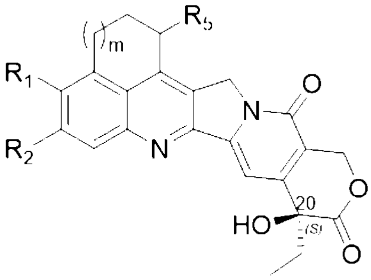

I-a

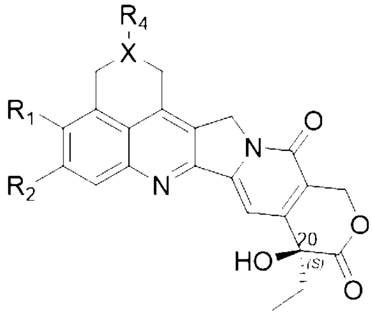

I-b

wherein the $R_1$, $R_2$, $R_4$, $R_5$, X, and m are as defined independently of each other in any one of claims 1 to 5; preferably, the compound represented by formula I has a structure as shown below:

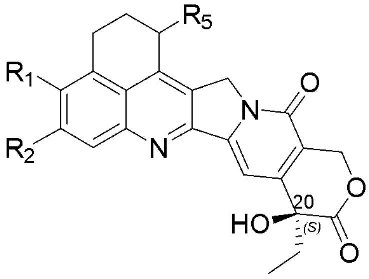

II

wherein the $R_1$, $R_2$, and $R_5$ are as defined independently of each other in any one of claims 1 to 5; and preferably, the compound represented by formula I has a structure as shown below:

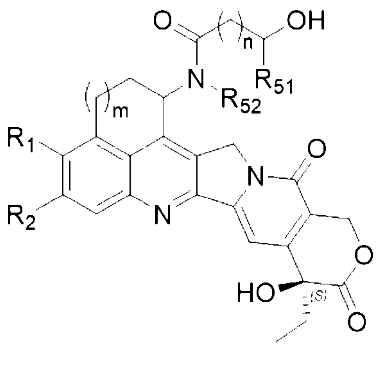

III-a

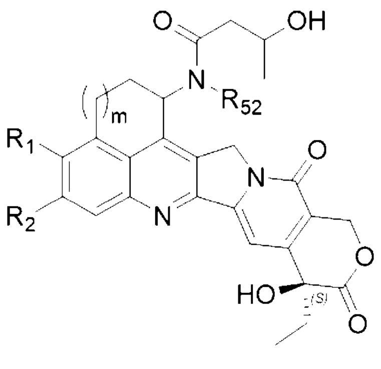

III-b

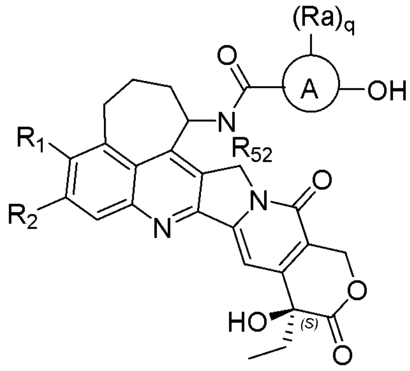

III-c

wherein the $R_1$, $R_2$, $R_{51}$, $R_{52}$, ring A, Ra, m, n, and q are as defined independently of each other in any one of claims 1 to 5.

7. The compound according to any one of claims 1 to 6, wherein the compound of formula I has a structure as shown below:

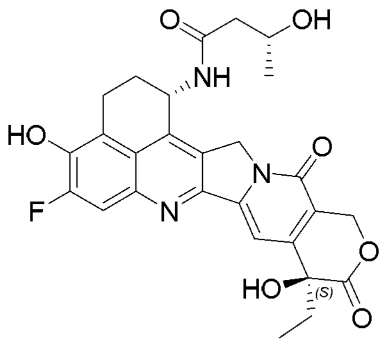 , 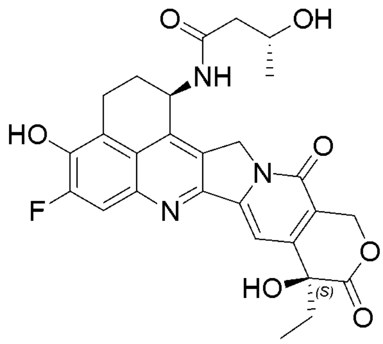 , 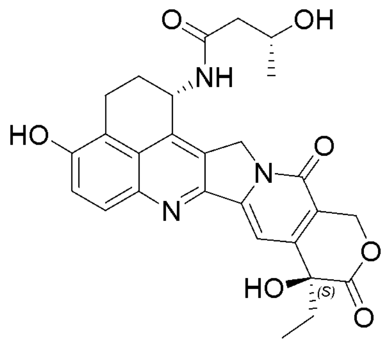 ,

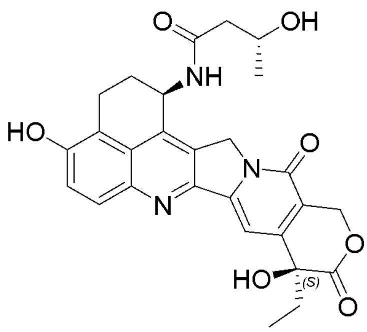 , 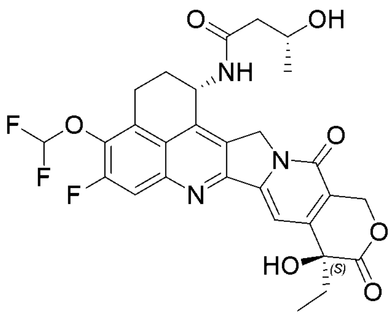 , 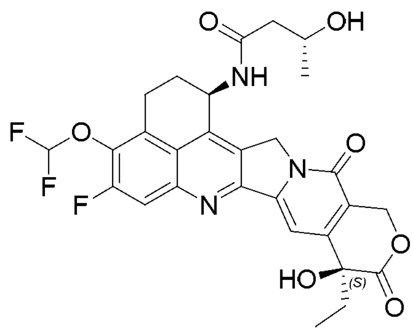

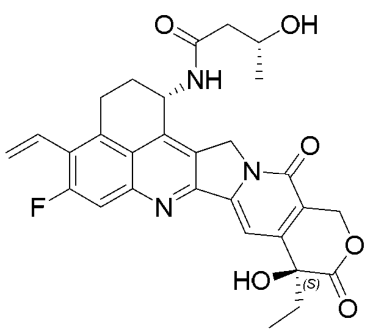 , 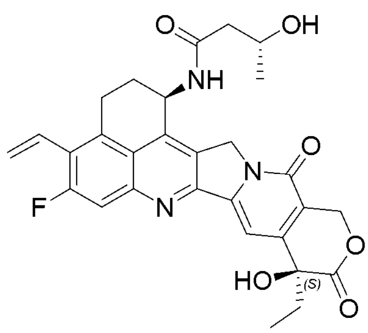 , 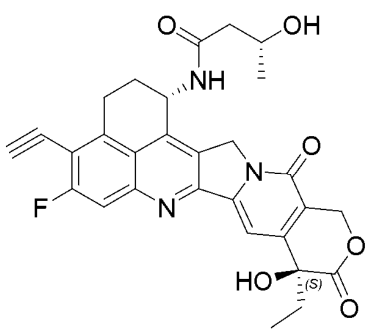 ,

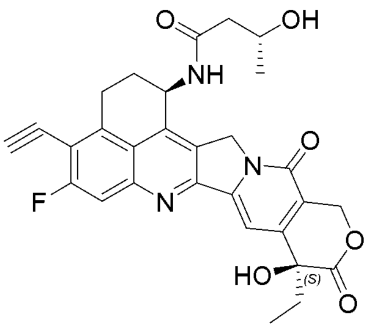,

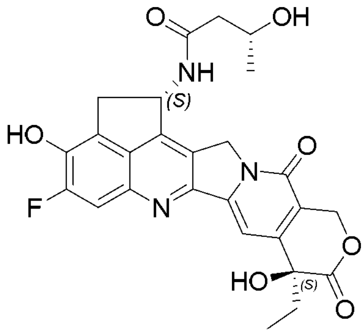,

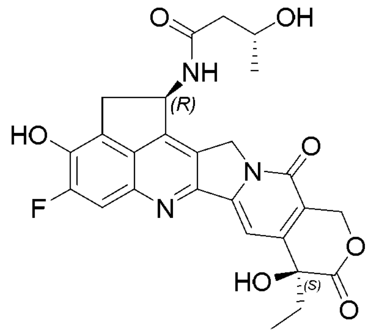,

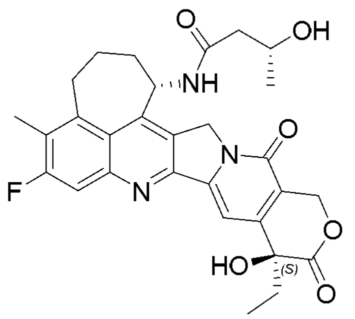,

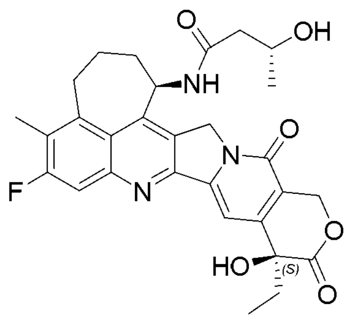,

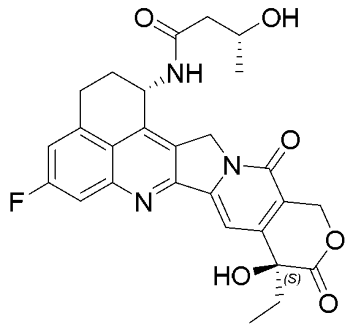,

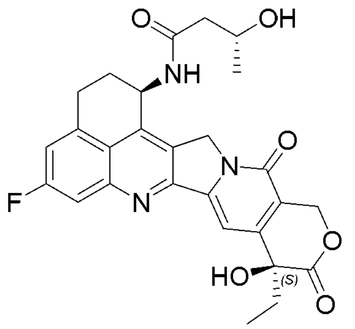,

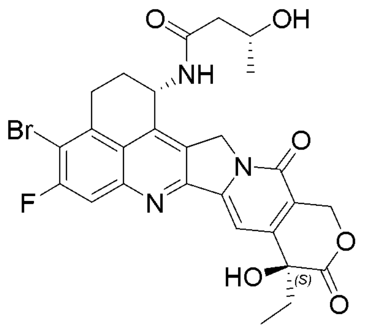,

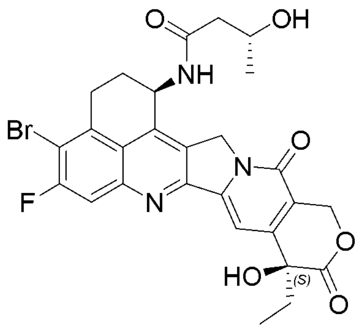,

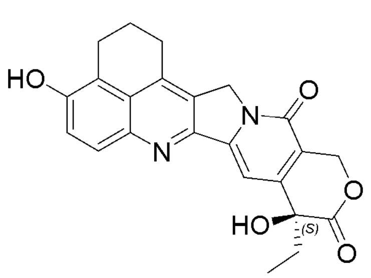,

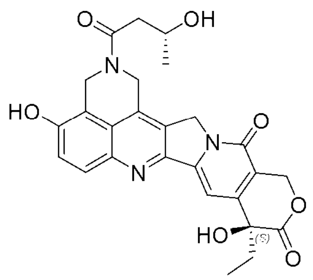,

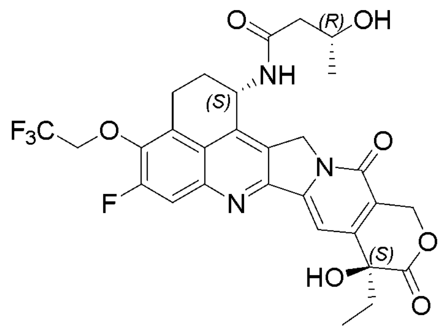,

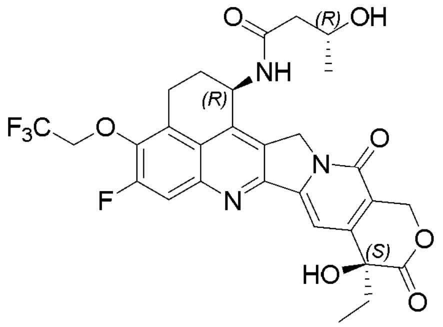
,
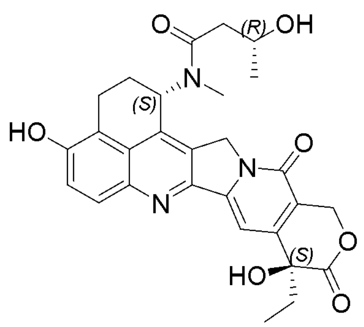
,
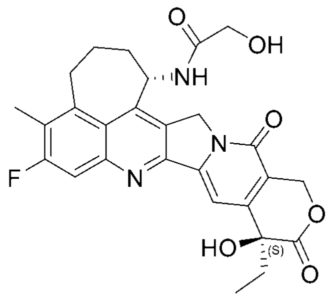
,
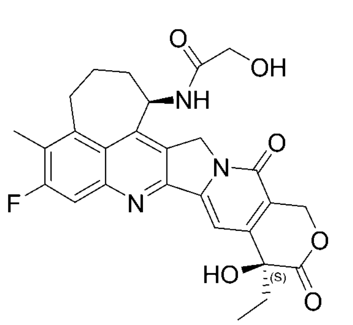
,
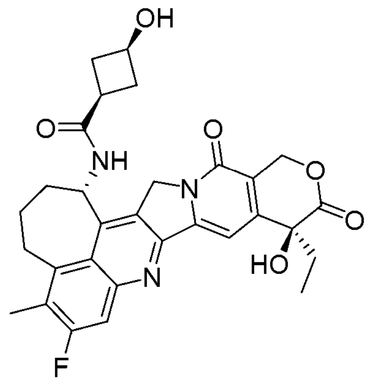
,
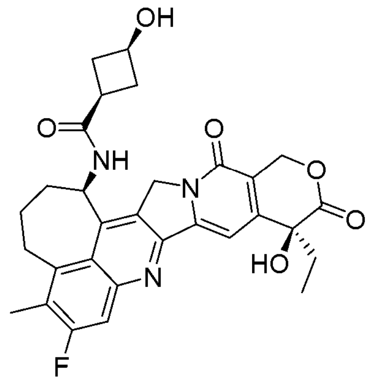
,
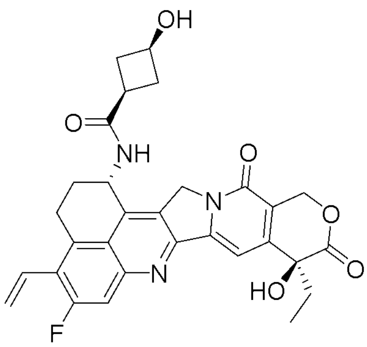
,
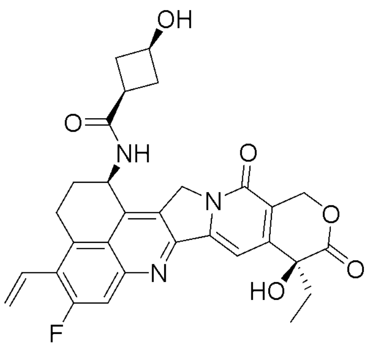
,
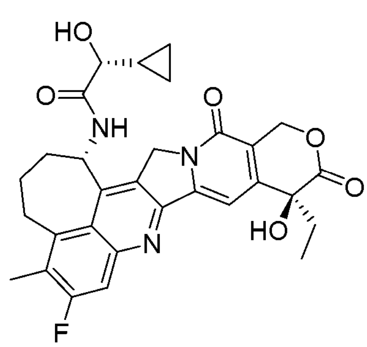
,
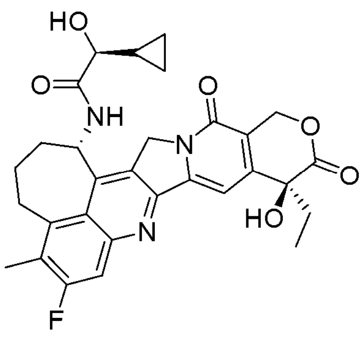
,
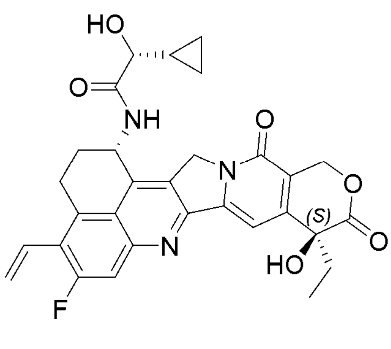
,
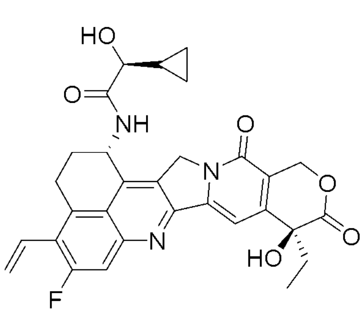
,

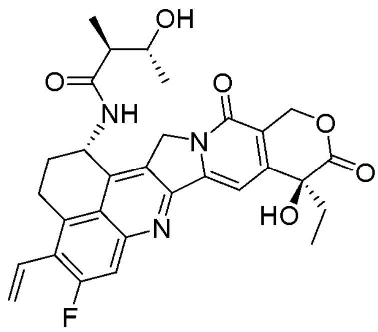 , 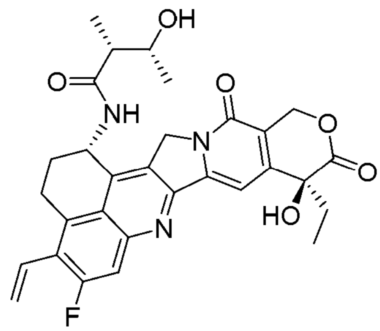 , 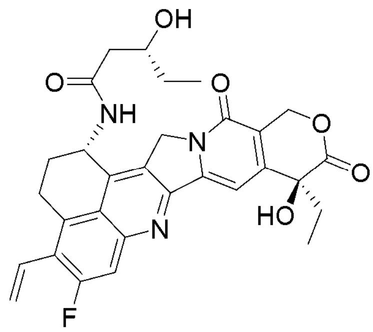 ,

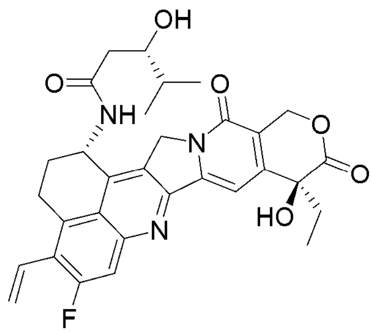 , 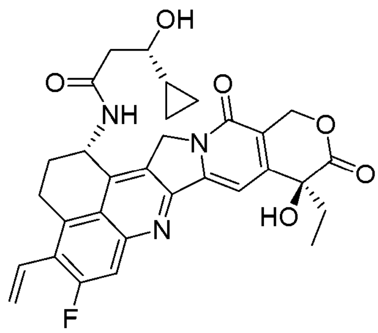 , 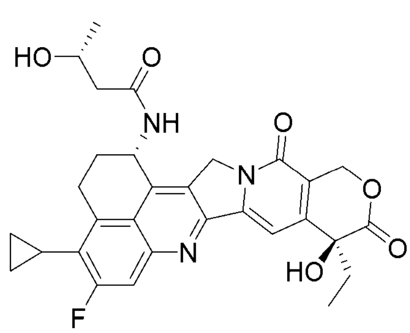 ,

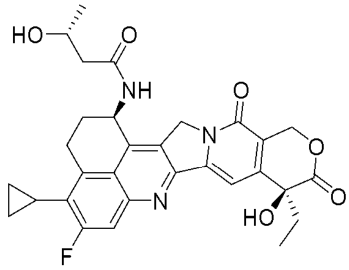 , 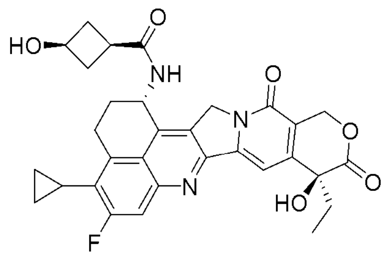 , 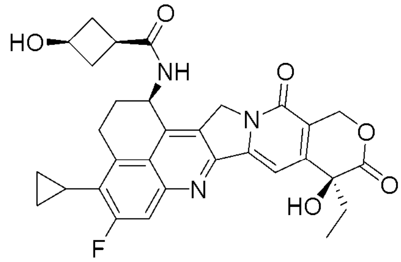 ,

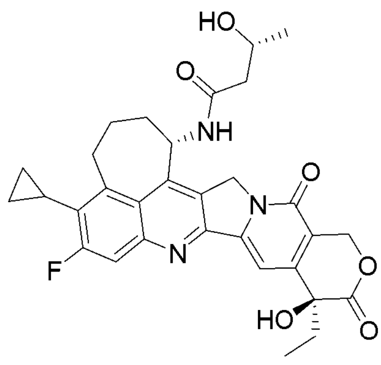 , 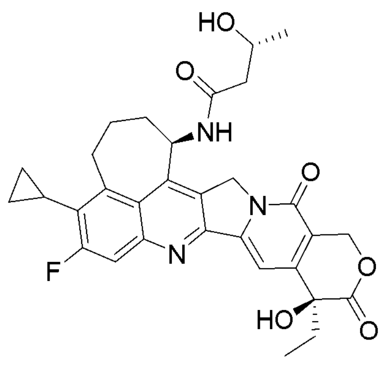 , 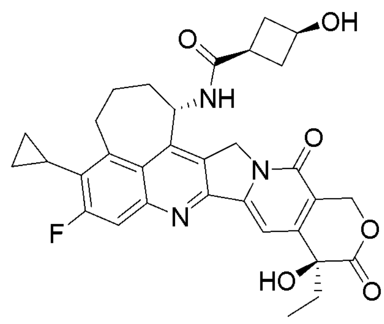 ,

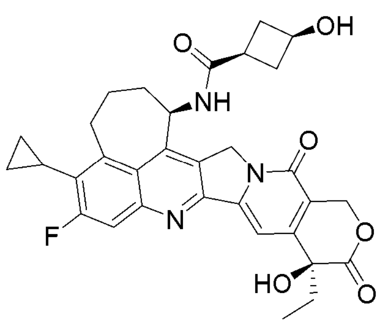,
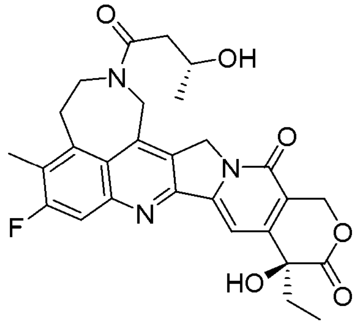,
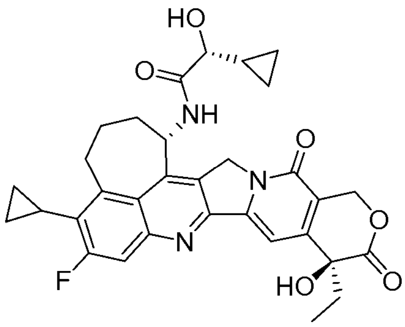,

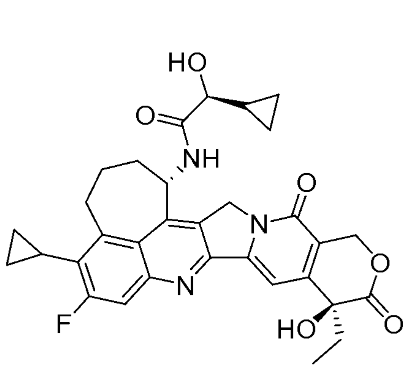,
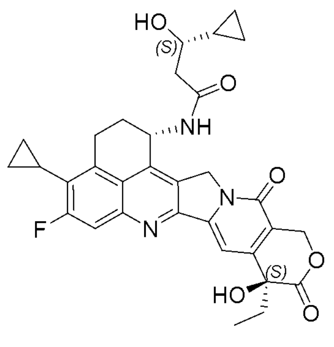,
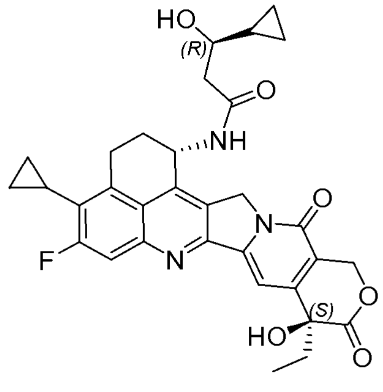,

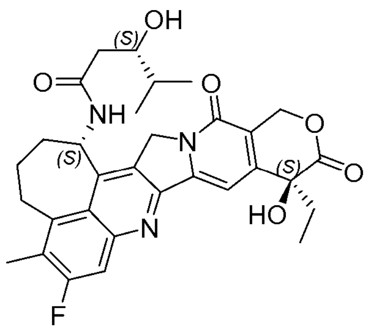,
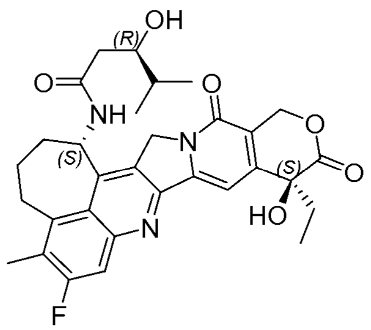,
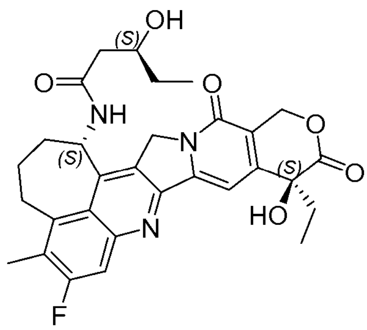,

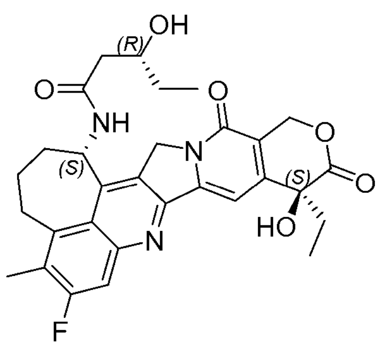,
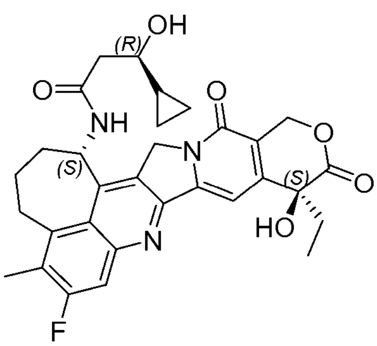,
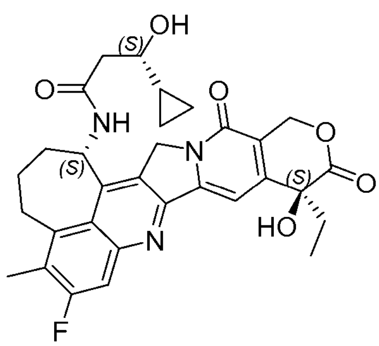,

or

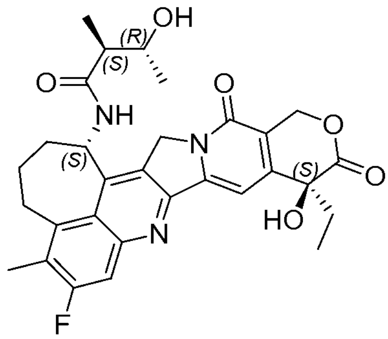

.

**8.** A structural fragment D, having the structure of the compound represented by formula I according to any one of claims 1 to 7 after dehydrogenation,
preferably, the D has a structure as shown below:

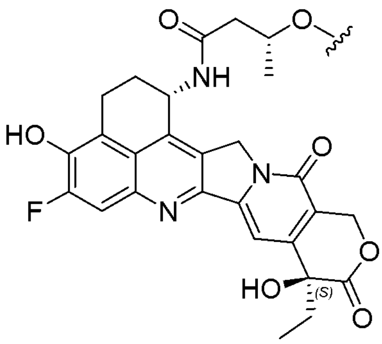

,

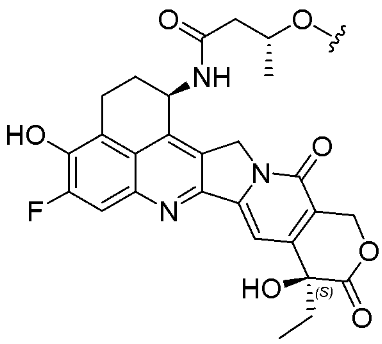

,

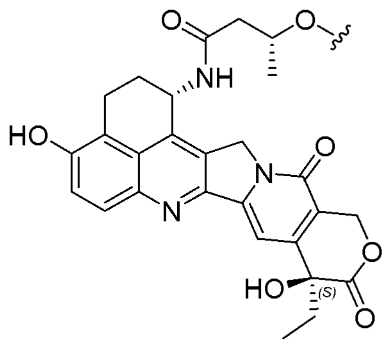

,

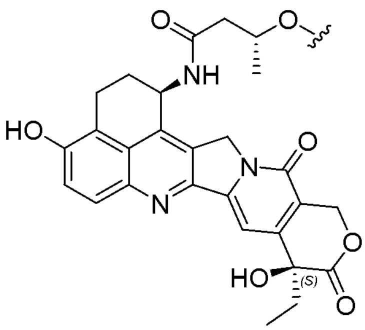

,

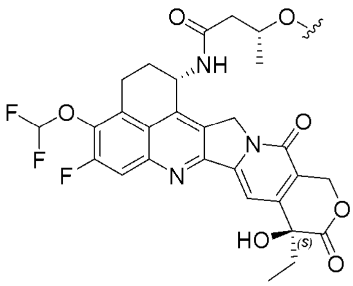

,

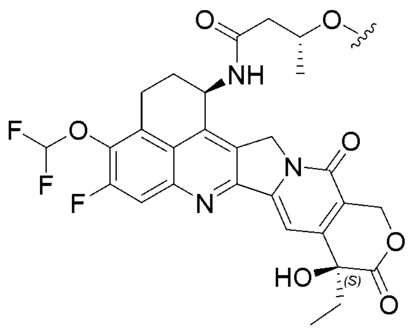

,

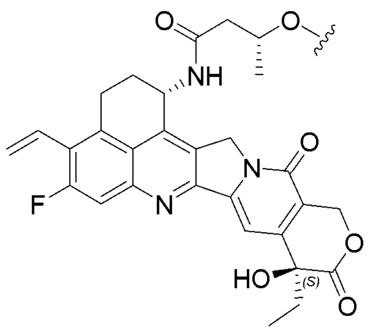

,

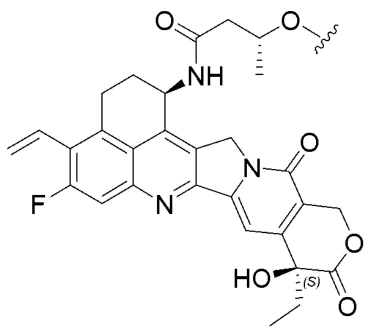

,

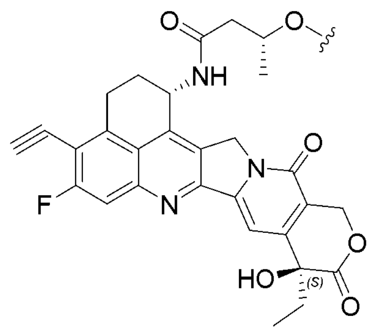

,

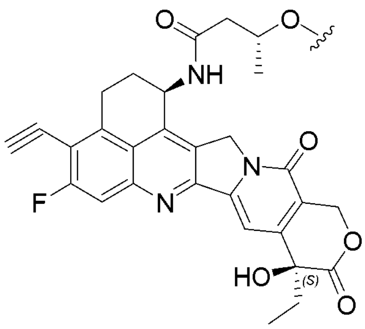,
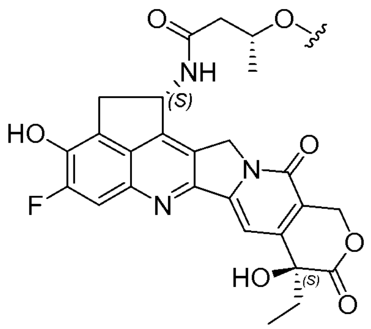,
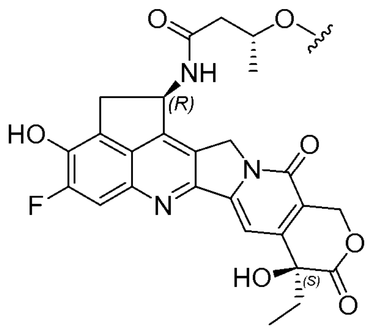,

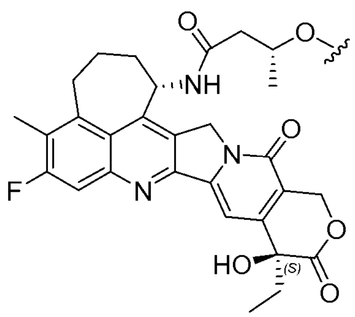,
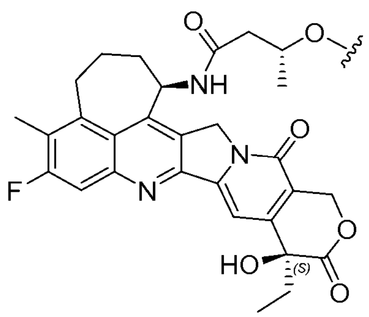,
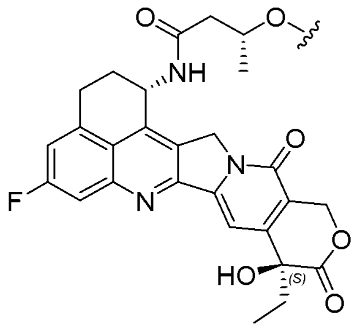,

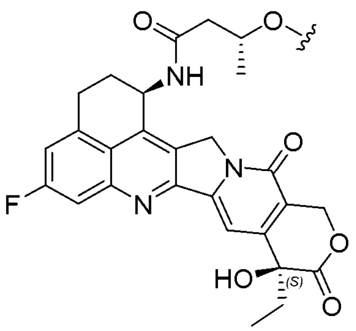,
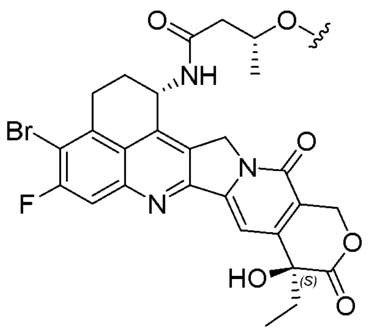,
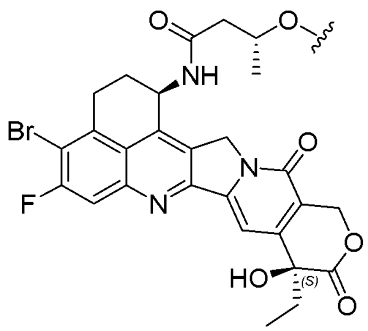,

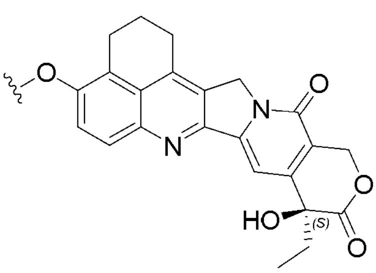,
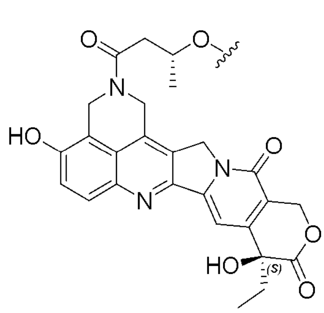,
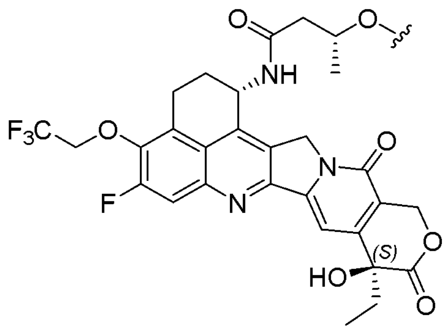,

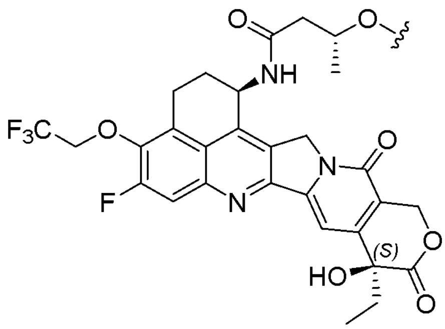,

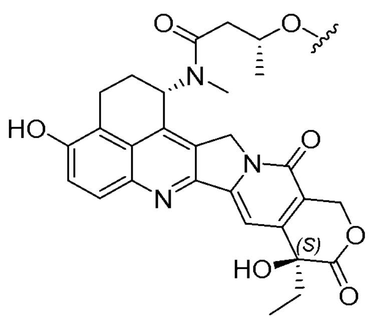,

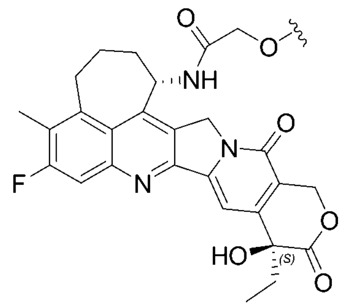,

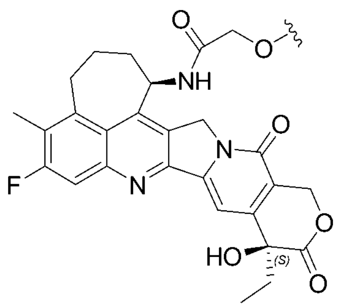,

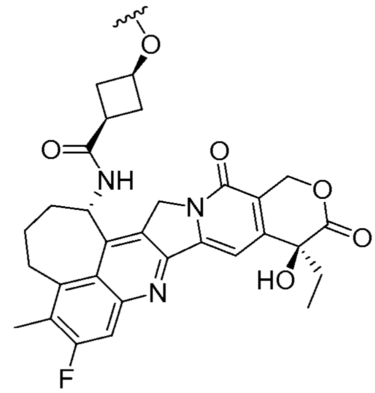,

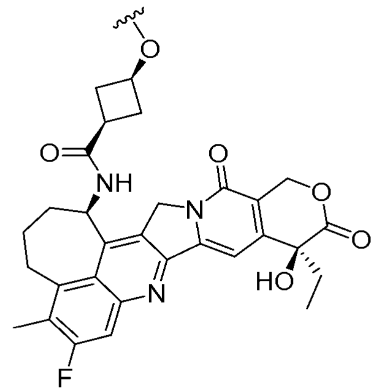,

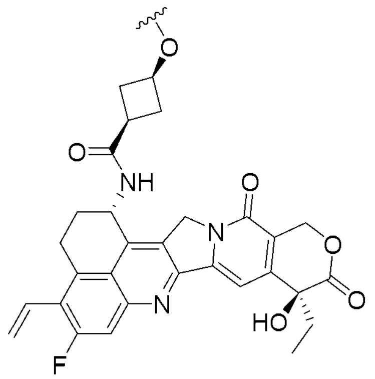,

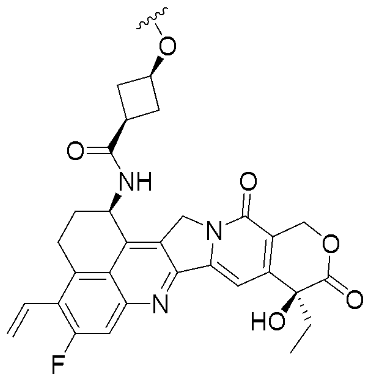,

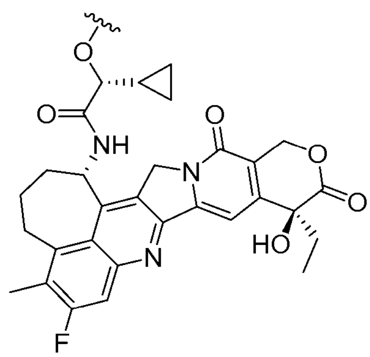,

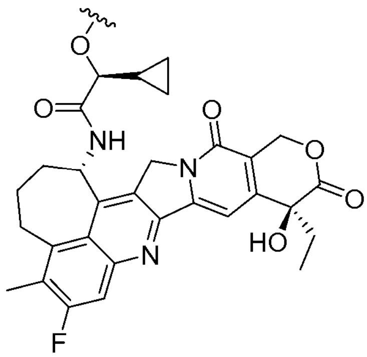,

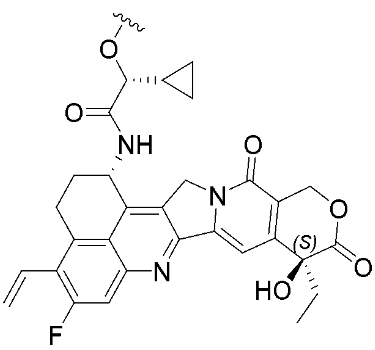,

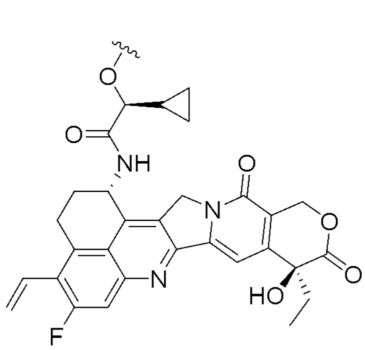,

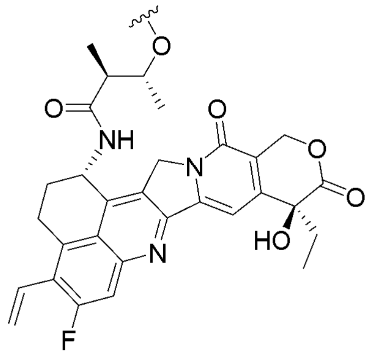
,
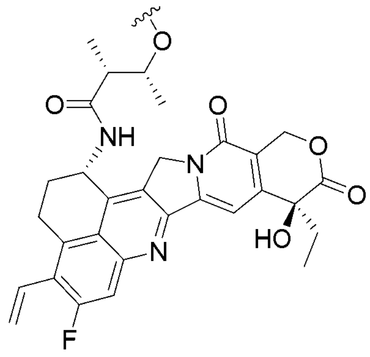
,
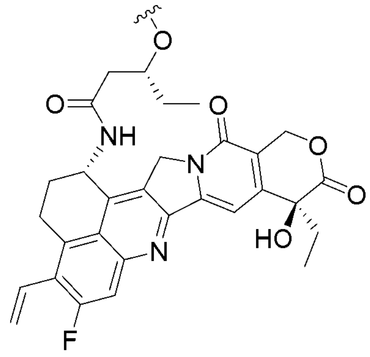
,

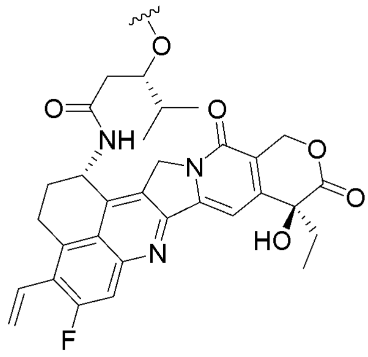
,
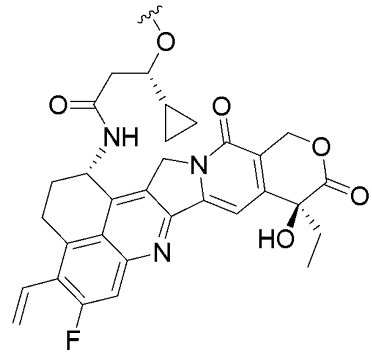
,
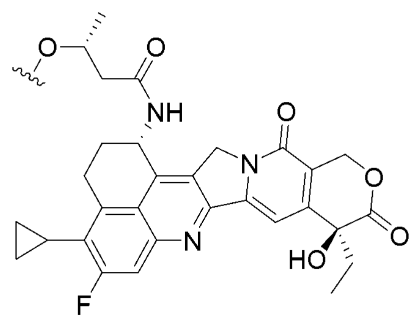
,

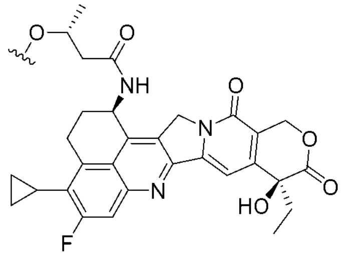
,
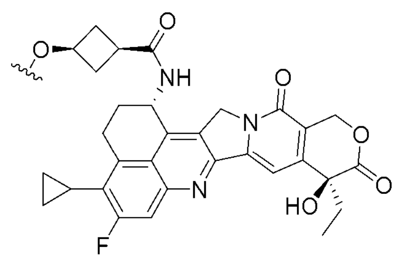
,
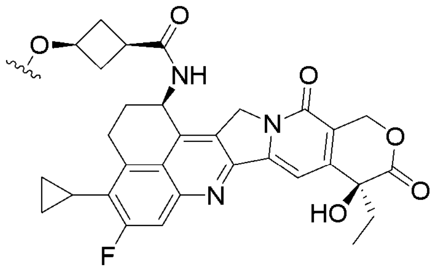

,
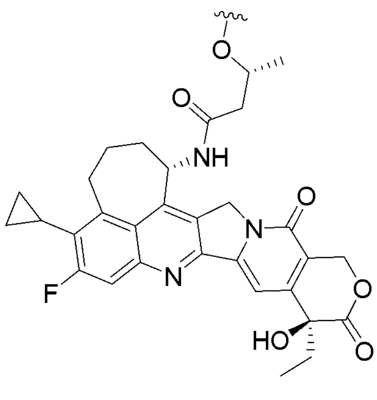
,
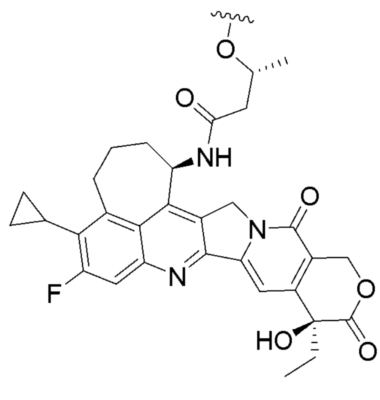
,
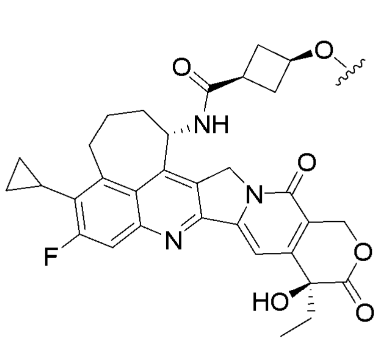
,

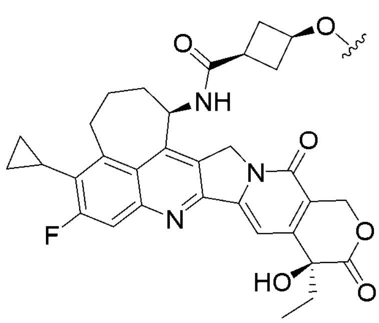,
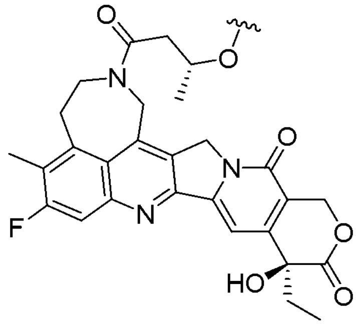,
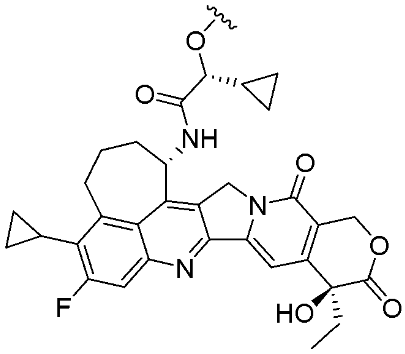,

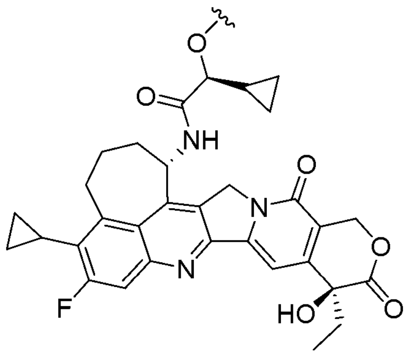,
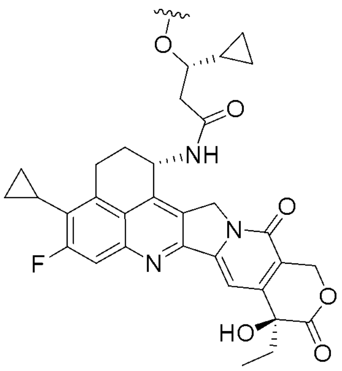,
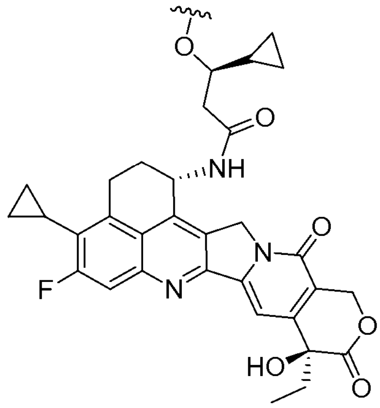,

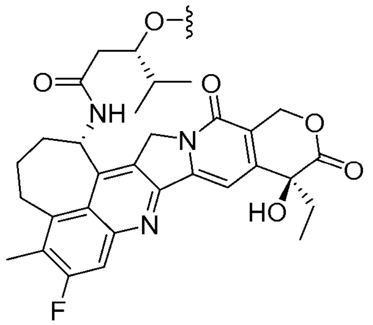,
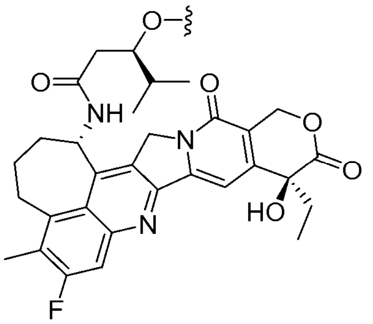,
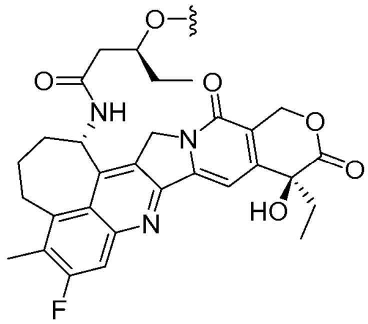,

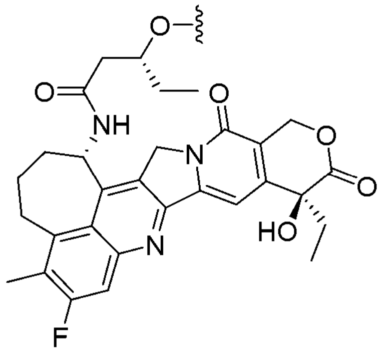,
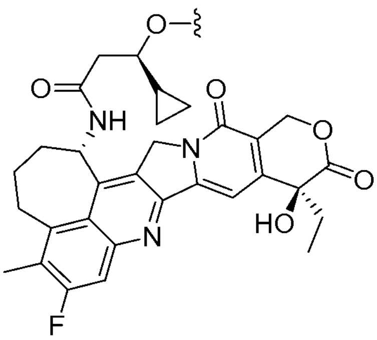,
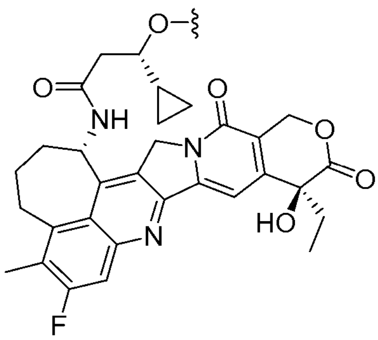,

or

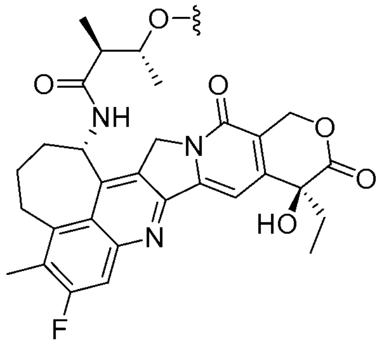

.

**9.** A compound represented by formula V, or a racemate, a stereoisomer, a tautomer, an isotopically labeled compound, a solvate, a polymorph, a pharmaceutically acceptable salt, or a prodrug compound thereof:

L'-D (formula V)

wherein L' is a linker containing a linker site M capable of reacting with an antibody or an antigen-binding fragment thereof, and after L'-D reacts with the antibody or the antigen-binding fragment thereof, the L' thereof forms a linker L;
preferably, the L' comprises a peptide residue $L_1$ and a fragment $L_2$ in which the peptide residue is connected to D; and
the D is as defined in claim 8.

**10.** An antibody-drug conjugate represented by formula VI,

Ab-[L-D]$_\beta$ (formula VI)

wherein Ab is an antibody or an antigen-binding fragment thereof, D is as defined in claim 8, L is a linker linking the Ab to the D, and $\beta$ is an integer or a decimal selected from 1-10.

**11.** A method for preparing the compound according to any one of claims 1 to 7, comprising solution I or solution II below:

solution I: comprising steps of:

(1) removing a protecting group $PG_4$ from compound I-41 to give compound I-42; and
(2) allowing the compound I-42 to react with compound I-43 to give the compound represented by formula I;

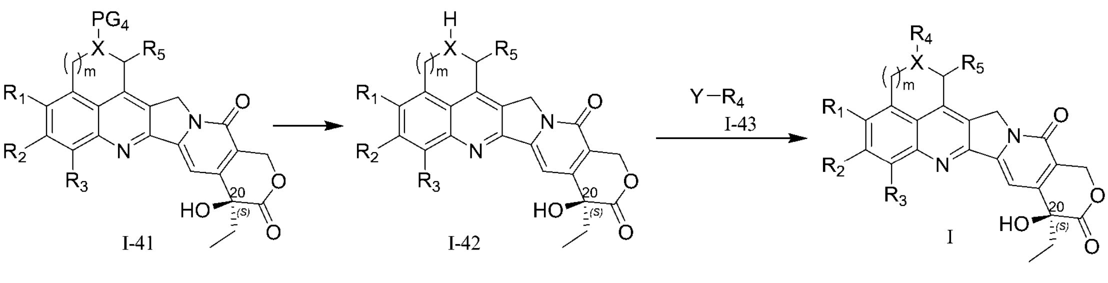


I-41 I-42 I

wherein the $R_1$, $R_2$, $R_3$, $R_4$, $R_5$, X, and m are as defined in any one of claims 1 to 7; Y is selected from a leaving group, for example, OH, Cl, Br, or I; and the $PG_4$ is selected from an amino protecting group, for example, Fmoc, Boc, Bn, or Cbz; or
solution II: comprising steps of:

(1) removing a protecting group $PG_5$ from compound I-51 to give compound I-52; and
(2) allowing the compound I-52 to react with compound I-53 to give the compound represented by formula I;

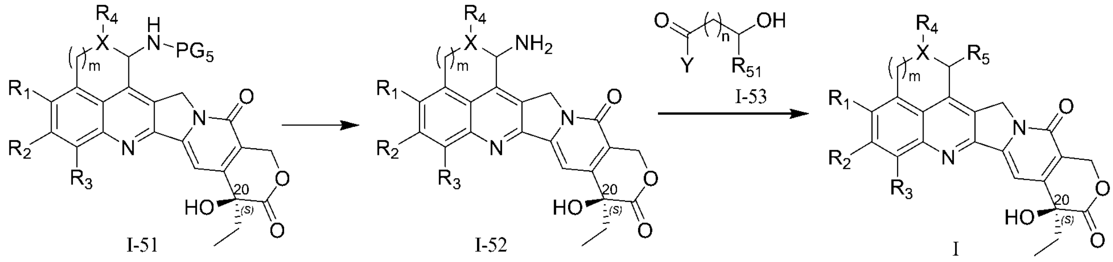

I-51 I-52 I

wherein the $R_1$, $R_2$, $R_3$, $R_4$, $R_5$, $R_{51}$, X, m, and n are as defined in any one of claims 1 to 7; Y is selected from a leaving group, for example, OH, Cl, Br, or I; and the $PG_5$ is selected from an amino protecting group, for example, Fmoc, Boc, Bn, or Cbz.

**12.** A pharmaceutical composition, comprising a therapeutically effective amount of at least one of the compound according to any one of claims 1 to 7, a racemate, a stereoisomer, a tautomer, an isotopically labeled compound, a solvate, a polymorph, a pharmaceutically acceptable salt, or a prodrug compound thereof; wherein
preferably, the pharmaceutical composition comprises a therapeutically effective amount of the antibody-drug conjugate according to claim 10.

**13.** Use of at least one of the compound according to any one of claims 1 to 7, or the compound represented by formula V according to claim 9, or the antibody-drug conjugate represented by formula VI according to claim 10, a racemate, a stereoisomer, a tautomer, an isotopically labeled compound, a solvate, a polymorph, a pharmaceutically acceptable salt, or a prodrug compound thereof, or the pharmaceutical composition according to claim 12 for the manufacture of a topoisomerase I inhibitor and/or for the manufacture of a medicament for preventing or treating a disease or condition associated with topoisomerase I; wherein
preferably, the disease or condition is a tumor, comprising breast cancer, gastric cancer, lung cancer, colorectal cancer, large intestine cancer, ovarian cancer, liver cancer, kidney cancer, esophageal cancer, cervical cancer, bladder cancer, pancreatic cancer, prostate cancer, nasopharyngeal carcinoma, melanoma, or leukemia.

**14.** A method for treating a tumor disease, comprising administering to a patient a prophylactically or therapeutically effective amount of at least one of a compound represented by formula I or formula V, or an antibody-drug conjugate represented by formula VI, a racemate, a stereoisomer, a tautomer, an isotopically labeled compound, a solvate, a polymorph, a pharmaceutically acceptable salt, or a prodrug compound thereof; wherein
preferably, the tumor comprises breast cancer, gastric cancer, lung cancer, colorectal cancer, large intestine cancer, ovarian cancer, liver cancer, kidney cancer, esophageal cancer, cervical cancer, bladder cancer, pancreatic cancer, prostate cancer, nasopharyngeal carcinoma, melanoma, or leukemia.

# INTERNATIONAL SEARCH REPORT

International application No.
**PCT/CN2023/142848**

**A. CLASSIFICATION OF SUBJECT MATTER**

C07D491/22(2006.01)i; A61K31/4375(2006.01)i; A61K47/68(2017.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D491/-, A61K31/-, A61K47/-

Documentation searched other than minimum documentation to the extent that such documents are included in the fields searched

Electronic data base consulted during the international search (name of data base and, where practicable, search terms used)

CNKI, CNTXT, VEN, STN: 喜树碱, 替康, 偶联, 耦联, camptothecin, ligand, conjugate, 基于权利要求7中的具体化合物进行了子结构检索, search for substructure on the basis of specific compounds in claim 7

**C. DOCUMENTS CONSIDERED TO BE RELEVANT**

| Category* | Citation of document, with indication, where appropriate, of the relevant passages | Relevant to claim No. |
|---|---|---|
| X | US 4939255 A (DAIICHI SEIYAKU CO.; YAKULT HONSHA KK.) 03 July 1990 (1990-07-03)<br>description, columns 2-6 | 1-7, 11-13 |
| X | WO 2022078260 A1 (SICHUAN BAILI PHARM CO., LTD. et al.) 21 April 2022 (2022-04-21)<br>embodiments 1-16, and claims 1-28 | 1-13 |
| X | WO 2020063673 A1 (JIANGSU HENGRUI MEDICINE CO., LTD. et al.) 02 April 2020 (2020-04-02)<br>embodiments, and claims | 1-13 |

☐ Further documents are listed in the continuation of Box C.   ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 April 2024** | **06 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/142848**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:
The subject matter of claim 14 relates to a method for treatment of the human/animal body (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/142848**

| Patent document cited in search report | Publication date (day/month/year) | Patent family member(s) | Publication date (day/month/year) |
|---|---|---|---|
| US 4939255 A | 03 July 1990 | KR 890000489 A | 15 March 1989 |
| | | KR 960002853 B1 | 27 February 1996 |
| | | NO 882785 D0 | 23 June 1988 |
| | | NO 882785 L | 27 December 1988 |
| | | NO 167738 B | 26 August 1991 |
| | | NO 167738 C | 04 December 1991 |
| | | AU 1834988 A | 05 January 1989 |
| | | AU 610189 B2 | 16 May 1991 |
| | | ES 2064329 T3 | 01 February 1995 |
| | | ATE 111913 T1 | 15 October 1994 |
| | | FI 883050 A0 | 23 June 1988 |
| | | FI 883050 A | 25 December 1988 |
| | | FI 91872 B | 13 May 1994 |
| | | FI 91872 C | 25 August 1994 |
| | | DK 344488 D0 | 23 June 1988 |
| | | DK 344488 A | 24 February 1989 |
| | | DE 3851577 D1 | 27 October 1994 |
| | | DE 3851577 T2 | 27 April 1995 |
| | | IE 881860 L | 24 December 1988 |
| | | IE 61281 B1 | 19 October 1994 |
| | | PH 27557 A | 18 August 1993 |
| | | US 5061795 A | 29 October 1991 |
| | | EP 0296597 A2 | 28 December 1988 |
| | | EP 0296597 A3 | 31 July 1991 |
| | | EP 0296597 B1 | 21 September 1994 |
| | | CA 1304370 C | 30 June 1992 |
| WO 2022078260 A1 | 21 April 2022 | JP 2023545581 A | 30 October 2023 |
| | | AU 2021359457 A1 | 11 May 2023 |
| | | IL 302054 A | 01 June 2023 |
| | | EP 4227310 A1 | 16 August 2023 |
| WO 2020063673 A1 | 02 April 2020 | MX 2021003446 A | 15 June 2021 |
| | | JP 2022512568 A | 07 February 2022 |
| | | JP 7408646 B2 | 05 January 2024 |
| | | EP 3854816 A1 | 28 July 2021 |
| | | EP 3854816 A4 | 07 September 2022 |
| | | TW 202028242 A | 01 August 2020 |
| | | ZA 202102696 B | 25 October 2023 |
| | | CA 3114474 A1 | 02 April 2020 |
| | | AU 2019351427 A1 | 15 April 2021 |
| | | BR 112021004829 A2 | 08 June 2021 |
| | | KR 20210068457 A | 09 June 2021 |
| | | US 2021347894 A1 | 11 November 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202211739606 A
- WO 202310367781 A
- WO 202310743291 A
- WO 202310941713 A
- WO 202311173712 A
- WO 202311802330 A


### Non-patent literature cited in the description


- *Cancer Res.*, 1989, vol. 49, 6365
- *Nature Review Cancer*, 2006, vol. 6, 789
- *Med Res. Rev.*, 2015, vol. 35, 753